(19) Europäisches Patentamt
European Patent Office
Office européen des brevets

(11) EP 4 660 181 A1

(12) **EUROPEAN PATENT APPLICATION**
published in accordance with Art. 153(4) EPC

(43) Date of publication:
**10.12.2025 Bulletin 2025/50**

(21) Application number: **24749437.0**

(22) Date of filing: **26.01.2024**

(51) International Patent Classification (IPC):
***C07C 233/64*** (2006.01) ***C07C 243/38*** (2006.01)
***C07C 243/14*** (2006.01) ***C07C 15/00*** (2006.01)
***C07D 213/00*** (2006.01) ***C07D 401/00*** (2006.01)
***A61K 31/166*** (2006.01) ***A61K 31/4965*** (2006.01)
***A61K 31/505*** (2006.01) ***A61K 31/44*** (2006.01)
***A61K 31/4353*** (2006.01) ***A61P 25/02*** (2006.01)
***A61P 25/04*** (2006.01) ***A61P 29/00*** (2006.01)

(52) Cooperative Patent Classification (CPC):
**A61K 31/166; A61K 31/4353; A61K 31/44; A61K 31/4965; A61K 31/505; A61P 25/02; A61P 25/04; A61P 29/00; C07C 15/00; C07C 233/64; C07C 243/14; C07C 243/38; C07D 213/00; C07D 401/00**

(86) International application number:
**PCT/BR2024/050024**

(87) International publication number:
**WO 2024/159284 (08.08.2024 Gazette 2024/32)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **30.01.2023 US 202363482235 P**

(71) Applicants:
- **Eurofarma Laboratórios S.A.**
**06696-000 São Paulo - SP (BR)**
- **Universidade Federal Do Rio De Janeiro - UFRJ**
**21941-853 Rio de Janeiro -RJ (BR)**

(72) Inventors:
- **BARREIRO, Gabriela**
**SP 04514-032 São Paulo (BR)**
- **SANT'ANA, Danilo Pereira De**
**SP 06709-320 São Paulo (BR)**
- **MONTEIRO, Júlia Lammoglia**
**SP 06704175 São Paulo (BR)**
- **GAMBA, Luis Eduardo Reina**
**SP 06404-326 São Paulo (BR)**
- **FRAGA, Carlos Alberto Manssour**
**(deceased) (BR)**
- **BARREIRO, Eliezer Jesus De Lacerda**
**(deceased) (BR)**
- **LIMA, Lídia Moreira**
**RJ 21931-220 Rio de Janeiro (BR)**

(74) Representative: **Engelhard, Markus**
**Boehmert & Boehmert**
**Anwaltspartnerschaft mbB**
**Pettenkoferstrasse 22**
**80336 München (DE)**

(54) **NAV1.7- AND/OR NAV1.8-INHIBITING HYDRAZIDES, PROCESSES FOR THE PREPARATION THEREOF, COMPOSITIONS, USES, METHODS FOR TREATMENT USING SAME, AND KITS**

(57) The present invention refers to Nav 1.7 and/or Nav 1.8 blocking hydrazides. More specifically, the present invention is related to hydrazides comprising Formula (I), in which the substituents $R_1$ to $R_8$ are selected independently of the groups defined in the specification, as well as their processes of obtaining, compositions comprising at least one of these compounds, uses, treatment methods to treat or prevent pain-related pathologies and kits. The present invention belongs to the fields of medicinal chemistry, organic synthesis, as well as in treatment of pain-related diseases.

EP 4 660 181 A1

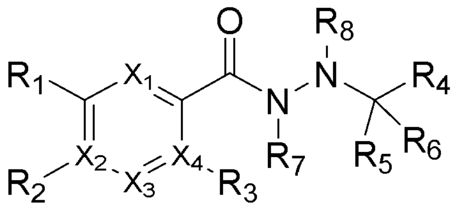

Formula (I)

## Description

## FIELD OF INVENTION

The present invention refers to Nav 1.7 and/or Nav 1.8 blocking hydrazides, their obtaining processes, compositions containing same, uses, kits and treatment methods to treat or prevent pain-related pathologies. The present invention belongs to the fields of medicinal chemistry, organic synthesis, as well as in treatment of pain-related diseases.

## BACKGROUND OF INVENTION

Physiological pain is an important protective mechanism designed to alert the body to real or potential injuries that can put its integrity at risk. Broadly speaking, physiological pain can be classified into nociceptive pain and inflammatory pain. Nociceptive pain is characterized by having a high activation threshold, which remains until the stimulus that generated it is eliminated. Inflammatory pain, which arises as a response to tissue damage, is characterized by having a low activation threshold and is a consequence of the activity of molecular mediators of the inflammatory process in sensitizing nociceptors (Schaible. Langenbecks Arch. Surg. 2004, 389, 237). When these nociceptive processes remain in the absence of noxious stimuli or in response to non noxious, stimuli, the protective and repair role of pain loses its functionality, configuring a maladaptive picture of neural plasticity and, as a consequence, a pathological state of chronic pain. Among the syndromes included in this classification, neuropathic pain has a high prevalence and impact today (Smith. Pain. 2020, 161, 1:S127; Cavalli. Int. J. Immunopathol. Pharmacol. 2019, 33:2058738419838383; Bouhassira. Rev Neurol (Paris). 2019, 175(1-2) :16; Scholz. Nature Neurosci., 2002, 5,1062; Costigan. Annu. Rev. Neurosci., 2009, 32, 1).

Neuropathic pain is defined by the International Association for the Study of Pain (IASP) as pain initiated or caused by a primary dysfunction or injury in the central and/or peripheral nervous system (Dworkin. Clin. J. Pain, 2002, 18(6), 343). Central neuropathic pain comes from spinal cord injuries or central nervous system diseases such as multiple sclerosis or Parkinson's disease (Ducreux. Brain, 2006, 129, 963). Peripheral neuropathic pain, on the other hand, can be caused by trauma, metabolic disorders, chemical neurotoxicity, infection, or tumor invasion, among others. Among the most common syndromes of neuropathic pain are chemotherapy-induced neuropathic pain, complex regional pain, neuropathy related to viral infection, neuropathy secondary to tumor infiltration, diabetic neuropathy, phantom limb pain, postherpetic neuralgia, trigeminal neuralgia, and postsurgical neuralgia (Pak. Curr. Pain Headache Rep., 2018, 22(2), 9).

Currently, there is no specific treatment for the control of pathologies related to neuropathic pain, however, the first-line alternative consists of the use of opioid analgesics, and - as adjuvants - local anesthetics, anticonvulsants and antidepressants. However, the adverse effects and low efficacy drastically limit the use of these agents in the control of various pain-related pathologies (Kushnarev. Expert Opin. Investig. Drugs, 2020, 29(3), 259; Emery. Expert Opin. Ther. Targets, 2016, 20(8), 975).

Voltage-gated sodium channels (Nav) play a key role in the transmission of pain-related stimuli. These channels are activated in response to membrane depolarization, allowing the generation and propagation of action potentials in neurons (and other electrically excitable cells), by controlling the flow of sodium ions through the membranes. Structurally, voltage-gated sodium channels are heteromeric transmembrane proteins consisting of one $\alpha$ subunit and two $\beta$ auxiliary subunits. The $\alpha$ subunit is organized into four homologous domains (I-IV), each with six transmembrane segments (S1-S6). The S4 segment of each domain is characterized by presenting a conserved region of arginine residues, which act as sensors of the intra- and extracellular electrical environment of the neuron. This mechanism makes it possible to transform changes in the cellular electric field into specific conformational changes that, in turn, regulate the activation, deactivation and inactivation of voltage-gated sodium channels (Catterall. Nat. Chem. Biol., 2020, 16, 1314; Wisedchaisri. Cell., 2019, 178(4), 993; Clairfeuille. Science, 2019, 363, 1302).

In mammals, nine subunits $\alpha$ (Nav 1.1 - Nav 1.9) and four auxiliary subunits $\beta$ ($\beta$1-$\beta$4) have been identified. The $\alpha$ subunits can further be classified according to their susceptibility to blocking by tetrodotoxin (TTX), being classified as sensitive to tetrodotoxin (Nav 1.1, Nav 1.2, Nav 1.3, Nav 1.4, Nav 1.6 and Nav 1.7) or resistant to tetrodotoxin (Nav 1.5, Nav 1.8 and Nav 1.9) (Lera-Ruiz. J. Med. Chem., 2015, 58(18), 7093; Bagal. J. Med. Chem., 2013, 56(3), 593). Each of these subunits $\alpha$ has a different profile of expression and function, so that some of them are essential for the proper functioning of organs such as the heart and/or brain. Thus, the non-selective blockade of these channels is related to several types of adverse effects, such as migraine, epilepsy, paralysis and muscle and cardiac syndromes, among others (Bagal. J. Med. Chem., 2013, 56(3), 593; Bagal. Channels, 2015, 9(6), 360).

Broadly speaking, sodium channels are distributed mainly in the central and peripheral nervous system, in neurons and glia. Nav channels 1.1, 1.2, and 1.3 are primarily expressed in the brain. The Nav 1.4 and Nav 1.5 channels are found primarily in skeletal and cardiac muscles, respectively. Nav 1.6 channels are expressed in the central and peripheral nervous systems, while Nav 1.9 channels are selectively expressed in C-type nociceptive fibers in the dorsal

root ganglion. On the other hand, the Nav 1.7 and Nav 1.8 channels are mainly found in the peripheral nervous system and are directly related to the processes of pain transmission (Law. Drug Discovery Today, 2019, 24(7), 1389; Bagal. Channels (Austin), 2015, 9(6), 360; Lera-Ruiz. J. Med. Chem., 2015, 58(18), 7093).

Nav 1.7 sodium channels are expressed broadly in the olfactory epithelium, sympathetic ganglion, and dorsal root ganglion, predominantly in nociceptive fibers C and A$\delta$. A large amount of evidence supports the important role of Nav 1.7 sodium channels in pain transmission processes. For example, gain-of-function related mutations in the gene (SCN9A), which encodes sodium channel Nav 1.7, are associated with extreme pain disorders such as congenital pain insensitivity, paroxysmal extreme pain disorder, and primary erythromelalgia. On the other hand, mutations related to loss of gene function (SCN9A) are related to congenital insensitivity to pain in individuals who, in general terms, are free of motor or cognitive impairment (Vetter. Pharmacology & Therapeutics, 2017, 172, 73; Ahuja. Science, 2015, 350(6267), 1491; Kingwell. Nat. Rev. Drug Discov., 2019, 18, 321; Safina. J. Med. Chem., 2021, 64, 2953; Luo. J. Med. Chem., 2019, 62, 831; Bankar. Cell Reports, 2018, 24, 3133).

Nav 1.8 sodium channels are most expressed in the peripheral nervous system, widely (but not exclusively) in C-type nociceptive fibers in the dorsal root ganglion. Recent evidence including elevated expression levels of Nav 1.8 in chronic pain states, data with Nav 1.8 *knockout* animals, and analgesic activity of Nav 1.8-specific desensitizing oligodeoxynucleotides, among others (Brown. Bioorg. Med. Chem., 2019, 27(1), 230; Payne. Br. J. Pharmacol., 2015, 172(10), 2654; Bagal. Med. Chem. Lett. 2015, 6(6) 650; Kort. J. Med. Chem. 2008, 51, 407; Zhang. Neuropharmacology, 2010, 59, 201 and 207), support the role of the sodium channel Nav 1.8 in the development and process of pain-related pathologies (Kingwell. Nat. Rev. Drug Discov., 2019, 18, 321; Law. Drug Discovery Today, 2019, 24(7), 1389; Bagal. Channels (Austin), 2015, 9(6), 360; Lera-Ruiz. J. Med. Chem., 2015, 58(18), 7093).

Thus, the voltage-gated sodium channels Nav 1.7 and Nav 1.8 are considered promising therapeutic targets for the treatment of neuropathic pain-related dysfunctions (Kornecook. J. Pharmacol. Exp. Ther., 2017, 362, 146; Kingwell. Nat. Rev. Drug Discov., 2019, 18, 321; Bagal. Channels (Austin), 2015, 9(6), 360; Lera-Ruiz. J. Med. Chem., 2015, 58(18), 7093; Deuis. Neuropharmacology, 2017, 127, 87 and 108; Kushnarev. Expert Opin. Investig. Drugs, 2020, 29(3), 259; McKerrall. Bioorganic & Medicinal Chemistry Lett., 2018, 28, 3141; Emery. Expert Opin. Ther. Targets, 2016, 20(8), 975; Bagal. Bioorganic & Medicinal Chemistry Lett., 2014, 24, 3690).

A large number of compounds have been described in the literature for their ability to act as blockers of Nav 1.7 and 1.8 sodium channels, however, they present a great structural diversity, a fact that does not allow the establishment of a common pharmacophoric group.

The patent literature contains several examples of compounds that act as sodium channel blockers. In particular, Nav 1.7 selective sodium channel blockers are described in US10550080, US9765029, and US10000475. Additionally, some documents describe selective blockers of Nav 1.8 sodium channels as WO2020261114, WO2020092667, US9163042, WO2014120808, WO2014120815, WO2018213426, WO2019014352, WO2015006280, and US7928107. These documents reveal compounds with different structures from the present invention.

In addition, there are patent documents that describe dual Nav blockers 1.7 and 1.8, including WO2018235851, US8629149, JP2017001991 that reveal, respectively, pyridyl amines, oxopiperazine derivatives and benzoxazolons. However, all these documents reveal compounds with structures and physicochemical characteristics different from the present invention.

In this context, it is advantageous to develop new alternatives of compounds that can act as Nav 1.7 and/or Nav 1.8 blockers that have adequate pharmacological action and, preferably, provide mitigated adverse effects. Therefore, the present invention refers to hydrazides as an alternative and/or complement to the treatment of pain-related diseases.

## SUMMARY OF THE INVENTION

The present invention discloses hydrazides with blocking activity of Nav 1.7 and/or 1.8 channels, against pain-related pathologies, as well as compositions, uses, kits, treatment methods and related preparation processes.

The present invention refers to compound(s) of Formula (I):

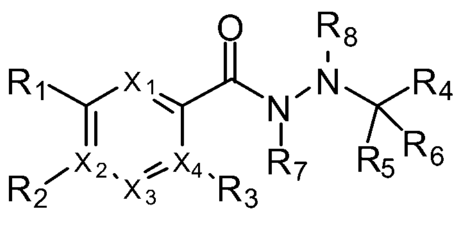

Formula (I)

or a pharmaceutically acceptable salt, hydrate, solvate, ester and isomer thereof, wherein:

- $X_1$, $X_2$, $X_3$, and $X_4$ are independently selected from the group consisting of carbon, CH, or nitrogen, where:
- when at least one of the substituents $X_2$, $X_4$ is N or CH, the corresponding R ($R_2$, $R_3$) is null.
- $R_1$ is:

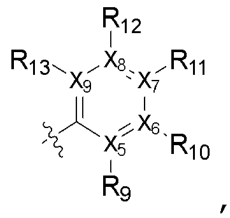

where:

- $X_5$, $X_6$, $X_7$, $X_8$, and $X_9$ are independently selected from the group consisting of carbon, CH, or nitrogen, where:
    - when at least one of the substituents $X_5$-$X_9$ is N or CH, the corresponding R ($R_9$-$R_{13}$) is null;
    - $R_9$, $R_{10}$, $R_{11}$, $R_{12}$, and $R_{13}$ are independently selected from the group consisting of hydrogen, halogen, linear or branched $C_1$-$C_6$ alkyl, linear or branched $C_1$-$C_6$ alkoxy, cycloalkoxy $C_3$-$C_7$, difluoromethyl, trifluoromethyl, linear or branched $C_1$-$C_6$ haloalkoxy;
    - $R_2$ and $R_3$ are independently selected from the group consisting of hydrogen, halogen, amine, hydroxy, and alkyl $C_1$-$C_6$ linear or branched, alkoxy $C_1$-$C_6$ linear or branched;
    - $R_4$ is :

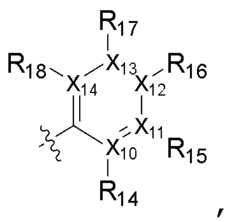

wherein:

- $X_{10}$, $X_{11}$, $X_{12}$, $X_{13}$, and $X_{14}$ are independently selected from the group consisting of carbon, CH, or nitrogen; where:
    - when at least one of the substituents $X_{10}$-$X_{14}$ is N or CH, the corresponding R ($R_{14}$-$R_{18}$) is null,
    - $R_{14}$, $R_{15}$, $R_{16}$, $R_{17}$, and $R_{18}$ are independently selected from the group consisting of hydrogen, halogen, hydroxy (alkyl $C_2$-$C_4$ linear or branched), alkyl $C_1$-$C_6$ linear or branched, alkoxy $C_1$-$C_6$ linear or branched;
    - $R_4$ and $R_8$ are optionally substituted to form a piperidine, homopiperidine, homomorpholine, or pyrrolidine;
    - $R_5$ and $R_6$ are independently selected from the group consisting of hydrogen, linear or branched $C_1$-$C_6$ alkyl; and
    - $R_7$ and $R_8$ are independently selected from the group consisting of hydrogen, linear or branched $C_1$-$C_6$ alkyl.

Additionally, the present invention further refers to compositions comprising one or more compound(s) of Formula (I) or a salt, hydrate, solvate and pharmaceutically acceptable isomer thereof; and one or more pharmaceutically acceptable excipients.

In addition, kits in accordance with this invention may comprise such compositions and application devices, which may include ampoules, syringes and others. Alternatively, the kits according to this invention comprise more than one compound of Formula (I) arranged in one or more dosage forms, including without limitation, tablets, accompanied by administration instructions.

The present invention further refers to methods of treatment, prevention, relief, suppression and/or control of diseases related to neuropathic pain. Uses of Formula (I) compound(s) to prepare a drug for the treatment of pathologies related to neuropathic pain are also taught. Finally, the present invention teaches processes for obtaining compound(s) of

Formula (I).

## DETAILED DESCRIPTION OF THE INVENTION

The present invention presents, in a first embodiment, the compound of Formula (I):

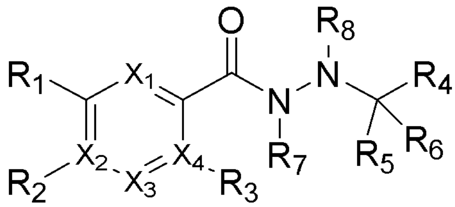

Formula (I)

or a pharmaceutically acceptable salt, hydrate, solvate, ester and isomer thereof, wherein:

- $X_1$, $X_2$, $X_3$, and $X_4$ are independently selected from the group consisting of carbon, CH, or nitrogen, wherein:
- when at least one of the substituents $X_2$, $X_4$ is N or CH, the corresponding R ($R_2$, $R_3$) is null;
- $R_1$ is:

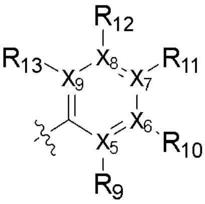

,

where:

- $X_5$, $X_6$, $X_7$, $X_8$, and $X_9$ are independently selected from the group consisting of carbon, CH, or nitrogen, where:
  - when at least one of the substituents $X_5$-$X_9$ is N or CH, the corresponding R ($R_{9}$-$_{13}$) is null;
  - $R_9$, $R_{10}$, $R_{11}$, $R_{12}$, and $R_{13}$ are independently selected from the group consisting of hydrogen, halogen, linear or branched $C_1$-$C_6$ alkyl, linear or branched $C_1$-$C_6$ alkoxy, $C_3$-$C_7$ cycloalkoxy, difluoromethyl, trifluoromethyl, linear or branched $C_1$-$C_6$ haloalkoxy;
  - $R_2$ and $R_3$ are independently selected from the group consisting of hydrogen, halogen, amine, hydroxy, and alkyl $C_1$-$C_6$ linear or branched, alkoxy $C_1$-$C_6$ linear or branched;
  - $R_4$ is :

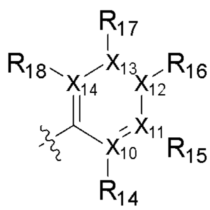

,

wherein:

- $X_{10}$, $X_{11}$, $X_{12}$, $X_{13}$, and $X_{14}$ are independently selected from the group consisting of carbon, CH, or nitrogen, wherein:
  - when at least one of the substituents $X_{10}$-$X_{14}$ is N or CH, the corresponding R ($R_{14}$-$R_{18}$) is null,
  - $R_{14}$, $R_{15}$, $R_{16}$, $R_{17}$, and $R_{18}$ are independently selected from the group consisting of hydrogen, halogen, hydroxy (alkyl $C_2$-$C_4$ linear or branched), alkyl $C_1$-$C_6$ linear or branched, alkoxy $C_1$-$C_6$ linear or branched;
  - $R_4$ and $R_8$ are optionally substituted to form together a piperidine, homopiperidine, homomorpho-

line, or pyrrolidine, whereby $R_{18}$ is null;
- $R_5$ and $R_6$ are independently selected from the group consisting of hydrogen, linear or branched $C_1$-$C_6$ alkyl; and
- $R_7$ and $R_8$ are independently selected from the group consisting of hydrogen, linear or branched $C_1$-$C_6$ alkyl.

As described herein, the compounds of the present invention comprise multiple variable groups (R, $X_1$, etc.). As a person skilled in the art will recognize, the group combinations contemplated by this invention are those combinations that result in the formation of stable or chemically viable compounds. The term "stable" in this context refers to compounds that are not substantially altered when subjected to conditions that allow their production, detection and preferably their recovery, purification and use for one or more of the purposes disclosed herein. In some embodiments, a stable compound or chemically viable compound is one that is not substantially altered when kept at a temperature of 40 °C or less, in the absence of moisture or other chemically reactive conditions, for at least one week.

Additionally, the chemical structures drawn herein are intended to be understood as they would be understood by a person skilled in the art. For example, with respect to the formulas III, IV, X, a person skilled in the art would understand that $X_2$ and $X_3$ are connected by a simple bond, even though the bonds between these groups can be hidden by the labels of the atoms in the chemical structures.

In an embodiment, $X_1$, $X_2$, $X_3$, and $X_4$ are independently selected from the group consisting of carbon, CH, or nitrogen, where when at least one of the substituents $X_2$, $X_4$ is N or CH, the corresponding R ($R_2$, $R_3$) is null; $R_1$ is:

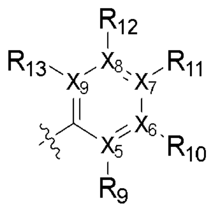

,

where:

$X_5$, $X_6$, $X_7$, $X_8$, and $X_9$ are independently selected from the group consisting of carbon, CH, or nitrogen, where when at least one of the substituents $X_3$-$X_9$ is N or CH, the corresponding R ($R_9$-. $R_{13}$) is null; $R_9$, $R_{10}$, $R_{11}$, $R_{12}$, and $R_{13}$ are independently selected from the group consisting of hydrogen, halogen, $C_1$-$C_6$ linear or branched alkyl, $C_1$-$C_6$ linear or branched alkoxy, $C_3$-$C_7$ cycloalkoxy, difluoromethyl, trifluoromethyl, haloalkoxy $C_1$-$C_6$ linear or branched; $R_2$ and $R_3$ are independently selected from the group consisting of hydrogen, halogen, amine, hydroxy, and alkyl $C_1$-$C_6$ linear or branched, alkoxy $C_1$-$C_6$ linear or branched; $R_4$ is:

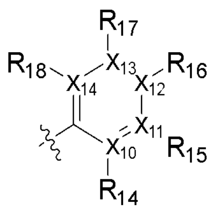

,

where:

$X_{10}$, $X_{11}$, $X_{12}$, $X_{13}$ and $X_{14}$ are independently selected from the group consisting of carbon, CH, or nitrogen, where when at least one of the substituents $X_{10}$-$X_{14}$ is N or CH, the corresponding R ($R_{14}$-$R_{18}$) is null; $R_{14}$, $R_{15}$, $R_{16}$, $R_{17}$, and $R_{18}$ are independently selected from the group consisting of hydrogen, halogen, hydroxy (alkyl $C_{2-4}$ linear or branched), $C_1$-$C_6$ linear or branched alkyl, $C_1$-$C_6$ linear or branched alkoxy; $R_4$ and $R_8$ are optionally substituted to form a piperidine, homopiperidine, homomorpholine, or a pyrrolidine; $R_5$ and $R_6$ are independently selected from the group consisting of hydrogen, linear or branched $C_1$-$C_6$ alkyl, and $R_7$ and $R_8$ are independently selected from the group consisting of hydrogen, linear or branched $C_1$-$C_6$ alkyl.

In an embodiment, $X_1$, $X_2$, $X_3$, and $X_4$ are independently selected from the group consisting of carbon and nitrogen.

In an embodiment, $R_1$ is:

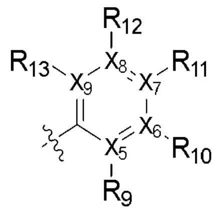

wherein $X_5$, $X_6$, $X_7$, $X_8$, and $X_9$ are independently selected from the group consisting of carbon and nitrogen. Preferably, $X_5$, $X_6$, $X_7$, and $X_9$ are carbon and $X_8$ is selected from carbon and nitrogen.

In an embodiment, $R_9$, $R_{10}$, $R_{11}$, $R_{12}$, and $R_{13}$ are independently selected from the group consisting of hydrogen, fluorine, chlorine, bromine, methyl, ethyl, propyl, isopropyl, butyl, *sec* -butyl, tert-butyl, methoxyl, ethoxyl, propoxyl, isopropoxyl, butoxyl, sec-butoxyl, tert-butoxyl, cyclopropoxyl, cyclobutoxyl, cyclopentoxyl, difluoromethyl, trifluoromethyl, difluoromethoxyl and trifluoromethoxyl. Preferably, $R_9$ is selected from the group consisting of hydrogen, fluorine, and methyl; $R_{10}$ is hydrogen; $R_{11}$ is selected from the group consisting of chlorine, methoxyl, ethoxyl, isopropoxyl, cyclopropoxyl, and difluoromethoxyl; $R_{12}$ is hydrogen or absent when $X_8$ is nitrogen; and $R_{13}$ is selected from the group consisting of hydrogen and methyl.

In an embodiment, $R_2$ and $R_3$ are independently selected from the group consisting of hydrogen, fluorine, chlorine, bromine, amine, hydroxy, methyl, ethyl, propyl, isopropyl, butyl, sec-butyl, tert-butyl, methoxyl, ethoxyl, propoxyl, isopropoxyl, butoxyl, sec-butoxyl and tert-butoxyl; or absent when the corresponding substituent $X_2$ and $X_4$ is nitrogen. Preferably, $R_2$ is hydrogen or absent when $X_2$ is nitrogen; and $R_3$ is hydrogen or absent when $X_4$ is nitrogen.

In an embodiment, $R_4$ is:

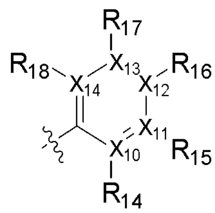

wherein $X_{10}$, $X_{11}$, $X_{12}$, $X_{13}$, and $X_{14}$ are independently selected from the group consisting of carbon and nitrogen. Preferably, $X_{10}$, $X_{11}$, $X_{12}$ and $X_{14}$ are carbon and $X_{13}$ is selected from carbon and nitrogen.

In an embodiment, $R_{14}$, $R_{13}$, $R_{16}$, $R_{17}$, and $R_{18}$ are independently selected from the group consisting of hydrogen, fluorine, chlorine, bromine, 1-hydroxyethyl, 2-hydroxyethyl, methyl, ethyl, propyl, isopropyl, butyl, *sec*-butyl, tert-butyl, methoxyl, ethoxyl, propoxyl, isopropoxyl, butoxyl, sec-butoxyl and tert-butoxyl; or null when $R_4$ and $R_8$ together form a piperidine homopiperidine, homomorpholine, or pyrrolidine. Preferably, $R_{14}$ is selected from the group consisting of hydrogen and fluorine; $R_{15}$ is selected from the group consisting of hydrogen, methoxyl, ethoxyl, and 2-hydroxyethyl; $R_{16}$ is selected from the group consisting of hydrogen, chlorine, methoxyl; $R_{17}$ is selected from the group consisting of hydrogen, methoxyl, ethoxyl, or absent when $X_{13}$ is nitrogen; and $R_{18}$ is selected from the group consisting of hydrogen, fluorine, methyl, or null when $R_4$ and $R_8$ together form a piperidine homopiperidine, homomorpholine, or pyrrolidine.

In one embodiment, $R_4$ and $R_8$ are optionally substituted to form together a piperidine, homopiperidine, homomorpholine, or pyrrolidine, where $R_{18}$ is null.

In one embodiment, $R_5$ and $R_6$ are independently selected from the group consisting of hydrogen, methyl, ethyl, propyl, isopropyl, butyl, *sec*-butyl, and *tert*-butyl. Preferably, $R_5$ and $R_6$ are independently selected from the group consisting of hydrogen and methyl.

In one embodiment, $R_7$ and $R_8$ are independently selected from the group consisting of hydrogen, methyl, ethyl, propyl, isopropyl, butyl, *sec*-butyl, and *tert*-butyl. Preferably, $R_7$ and $R_8$ are independently selected from the group consisting of hydrogen and methyl.

In an implementation of the first embodiment, the Formula (I) compound(s) is(are) selected from the group(s) consisting of:

- *N*'-(3,5-dimethoxybenzyl)-6-(4-methoxyphenyl)pyrazine-2-carbohydrazide (Compound 1);
- *N*'-(4-chlorobenzyl)-6-(4-methoxyphenyl)pyrazine-2-carbohydrazide (Compound 2);
- *N*'-(3,5-dimethoxybenzyl)-6-(4-isopropoxyphenyl)pyrazine-2-carbohydrazide (Compound 3);
- *N*'-(4-chlorobenzyl)-6-(4-cyclopropoxyphenyl)pyrazine-2-carbohydrazide (Compound 4);
- *N*'-(4-chlorobenzyl)-6-(4-chlorophenyl)pyrazine-2-carbohydrazide (Compound 5);
- *N*-(6-chloro-3,4-dihydroisoquinoline-2(1H)-yl)-6-(4-chlorophenyl)pyrazine-2-carboxamide (Compound 6);

- 6-(4-(difluoromethoxy)phenyl)-*N*'-(3,5-dimethoxybenzyl)pyrazine-2-carbohydrazide (Compound 7);
- 6-(6-(difluoromethoxy)pyridin-3-yl)-*N'*-(3,5-dimethoxybenzyl)pyrazine-2-carbohydrazide (Compound 8);
- *N'*-(4-chlorobenzyl)-6-(4-ethoxyphenyl)pyrazine-2-carbohydrazide (Compound 9);
- *N'*-(3,5-dimethoxybenzyl)-6-(4-ethoxyphenyl)pyrazine-2-carbohydrazide (Compound 10);
- 6-(4-ethoxyphenyl)-*N*-(8-methoxy-1,3,4,5-tetrahydro-2H-benzo[c]azepin-2-yl)pyrazine-2-carboxamide (Compound 11);
- 6-(4-ethoxyphenyl)-*N*-(7-methoxy-2,3-dihydrobenzo[f] [1,4]oxazepine-4(5H)-yl)pyrazine-2-carboxamide (Compound 12);
- 6-(4-ethoxyphenyl)-*N*-(5-methoxyisoindolin-2-yl)pyrazine-2-carboxamide (Compound 13);
- 6-(4-ethoxyphenyl)-*N*'-(5-methoxy-2-methylbenzyl)pyrazine-2-carbohydrazide (Compound 14);
- 6-(4-ethoxyphenyl)-*N*'-(2-fluoro-5-methoxybenzyl)pyrazine-2-carbohydrazide (Compound 15);
- 6-(4-ethoxyphenyl)-*N*'-(3-methoxybenzyl)pyrazine-2-carbohydrazide (Compound 16);
- *N*'-(3,5-dimethoxybenzyl)-6-(4-ethoxyphenyl)-*N*'-methylpyrazine-2-carbohydrazide (Compound 17);
- 6-(4-ethoxyphenyl)-*N*'-((5-methoxy-2-methylpyridin-3-yl)methyl)pyrazine-2-carbohydrazide (Compound 18);
- 6-(4-ethoxyphenyl)-*N*'-(3-(1-hydroxyethyl)-5-methoxybenzyl)pyrazine-2-carbohydrazide (Compound 19);
- 6-(4-ethoxyphenyl)-*N*'-((2-fluoro-5-(1-hydroxyethyl)pyridin-3-yl)methyl)pyrazine-2-carbohydrazide (Compound 20);
- 6-(4-ethoxyphenyl)-*N*'-(2-fluoro-5-(1-hydroxyethyl)benzyl)pyrazine-2-carbohydrazide (Compound 21);
- 6-(4-ethoxyphenyl)-*N*'-(2-fluorobenzyl)pyrazine-2-carbohydrazide (Compound 22);
- 6-(4-ethoxyphenyl)-*N*'-(3-(1-hydroxyethyl)benzyl)pyrazine-2-carbohydrazide (Compound 23);
- (*S*)-*N'*-(1-(3,5-dimethoxyphenyl)ethyl)-6-(4-ethoxyphenyl)pyrazine-2-carbohydrazide (Compound 24);
- (*R*)-*N'*-(1-(3,5-dimethoxyphenyl)ethyl)-6-(4-ethoxyphenyl)pyrazine-2-carbohydrazide (Compound 25);
- 6-(4-ethoxyphenyl)-*N*'-((2-fluoro-5-methoxypyridin-3-yl)methyl)pyrazine-2-carbohydrazide (Compound 26);
- *N*'-(3,5-dimethoxybenzyl)-6-(4-ethoxy-2-fluorophenyl)pyrazine-2-carbohydrazide (Compound 27);
- *N'*-(3,5-dimethoxybenzyl)-6-(4-ethoxy-2-methylphenyl)pyrazine-2-carbohydrazide (Compound 28);
- *N'*-(3,5-dimethoxybenzyl)-6-(6-ethoxypyridin-3-yl)pyrazine-2-carbohydrazide (Compound 29);
- 6-(6-ethoxypyridin-3-yl)-*N'*-(3-methoxybenzyl)pyrazine-2-carbohydrazide (Compound 30);
- 6-(6-ethoxypyridin-3-yl)-*N'*-((2-fluoro-5-methoxypyridin-3-yl)methyl)pyrazine-2-carbohydrazide (Compound 31);
- 6-(6-ethoxypyridin-3-yl)-*N'*-(2-fluoro-5-methoxybenzyl)pyrazine-2-carbohydrazide (Compound 32);
- 6-(6-ethoxypyridin-3-yl)-*N'*-(5-methoxy-2-methylbenzyl)pyrazine-2-carbohydrazide (Compound 33);
- 6-(6-ethoxypyridin-3-yl)-*N'*-((5-methoxy-2-methylpyridin-3-yl)methyl)pyrazine-2-carbohydrazide (Compound 34);
- 6-(6-ethoxypyridin-3-yl)-*N'*-(3-(1-hydroxyethyl)benzyl)pyrazine-2-carbohydrazide (Compound 35);
- 6-(6-ethoxypyridin-3-yl)-*N'*-(3-(1-hydroxyethyl)-5-methoxybenzyl)pyrazine-2-carbohydrazide (Compound 36);
- 6-(6-ethoxypyridin-3-yl)-*N'*-((2-fluoro-5-(1-hydroxyethyl)pyridin-3-yl)methyl)pyrazine-2-carbohydrazide (Compound 37);
- 6-(6-ethoxypyridin-3-yl)-*N'*-(2-fluoro-5-(1-hydroxyethyl)benzyl)pyrazine-2-carbohydrazide (Compound 38);
- 6-(6-ethoxypyridin-3-yl)-*N'*-(2-fluorobenzyl)pyrazine-2-carbohydrazide (Compound 39);
- *N'*-(3,5-dimethoxybenzyl)-6-(6-ethoxy-4-methylpyridin-3-yl)pyrazine-2-carbohydrazide (Compound 40);
- *N'*-(3,5-dimethoxybenzyl)-6-(6-ethoxy-2-methylpyridin-3-yl)pyrazine-2-carbohydrazide (Compound 41);
- 6-(6-cyclopropoxypyridin-3-yl)-*N'*-(3,5-dimethoxybenzyl)pyrazine-2-carbohydrazide (Compound 42);
- *N'*-(3,5-dimethoxybenzyl)-6-(6-isopropoxypyridin-3-yl)pyrazine-2-carbohydrazide (Compound 43);
- 4-(4-chlorophenyl)-*N'*-(3,5-dimethoxybenzyl)pyrimidine-2-carbohydrazide (Compound 44);
- 6-(4-ethoxyphenyl)-*N*-(7-methoxy-3,4-dihydroisoquinoline-2(1*H*)-yl)pyrazine-2-carboxamide (Compound 45);
- 6-(4-ethoxyphenyl)-*N'*-(2-fluorobenzyl)pyridazine-4-carbohydrazide (Compound 46);
- 5-(4-ethoxyphenyl)-*N'*-(2-fluorobenzyl)pyridazine-3-carbohydrazide (Compound 47);
- 4-(4-ethoxyphenyl)-*N'*-(2-fluorobenzyl)picolinohydrazide (Compound 48);
- 5-(4-ethoxyphenyl)-*N'*-(2-fluorobenzyl)nicotinohydrazide (Compound 49);
- 6-(4-ethoxyphenyl)-*N'*-(2-fluorobenzyl)picolinohydrazide (Compound 50);
- 2-(4-ethoxyphenyl)-*N'*-(2-fluorobenzyl)isonicotinohydrazide (Compound 51);
- 2-(4-ethoxyphenyl)-*N'*-(2-fluorobenzyl)pyrimidine-4-carbohydrazide (Compound 52) and
- 4-(4-ethoxyphenyl)-*N'*-(2-fluorobenzyl)pyrimidine-2-carbohydrazide (Compound 53).

In an implementation of the first embodiment, the Formula (I) compound(s) are voltage-gated sodium channel blockers Nav 1.7 and/or Nav 1.8. In a preferred embodiment, the Formula (I) compounds are dual voltage-gated sodium channel blockers Nav 1.7 and Nav 1.8.

In an implementation of the first embodiment, the compound(s) of Formula (I) may have a basic nature and, consequently, pharmaceutically acceptable salts may be obtained by the addition of organic or inorganic acids. Nonlimiting examples of organic acids that can be used are fumaric, maleic, benzoic, lactic acids, among others. Among the inorganic acids, we can mention hydrochloric acid, phosphoric acid, hydrobromic acid, sulfuric acid, nitric acid, among

others.

In an implementation of the first embodiment, the compound(s) of Formula (I) can be obtained in the form of crystals, which can be optionally presented as pharmaceutically acceptable solvates, in which the solvent is incorporated in stoichiometric proportions or not into the crystal lattice. In further embodiments, the crystallization solvent is water, resulting in pharmaceutically acceptable hydrates.

Finally, in an implementation of the first embodiment, the Formula (I) compound(s) may present more than one isomer, including without limitation, spatial isomerism, such as geometric and optical isomerism.

## DEFINITIONS

In order to clarify or elucidate the terms used in this invention, the following definitions are presented, whereby the scope is not limited thereto.

The term "halogen" refers to the elements of the 7A family of the periodic table, which are: fluorine (F), chlorine (Cl), bromine (Br), iodine (I), astatine (At) and tennessine (Ts).

The term "linear or branched $C_1$-$C_6$ alkyl refers to the saturated hydrocarbon groups of linear or branched chain, such as methyl, ethyl, propyl, isopropyl, isobutyl, butyl, sec-butyl, and tert-butyl but not being limited to the same.

The term "linear or branched $C_1$-$C_6$ alkoxy" refers to alkyl groups attached to a radical oxygen, such as methoxyl, ethoxyl, propoxyl, isopropoxyl, butoxyl, sec-butoxyl, and tert-butoxyl but not being limited to the same.

The term "cycloalkoxy $C_3$-$C_7$" refers to alkyl cycle groups attached to a radical oxygen such as cyclopropoxyl, cyclobutoxyl, cyclopentoxyl and cyclohexyl, but not being limited to the same.

The term "linear or branched haloalkoxy $C_1$-$C_6$" refers to alkyl groups attached to a radical oxygen and at least one halogen, such as, but not limited to, chloromethoxyl, dichloromethoxyl, trichloromethoxyl, fluoromethoxyl, difluoromethoxyl and trifluoromethoxyl.

The term "hydroxy(alkyl $C_2$-$C_4$ linear or branched)" refers to saturated, linear or branched alkyl groups replaced with at least one hydroxyl, such as 1-hydroxyethyl, 2-hydroxyethyl, 1-hydroxypropyl, 2-hydroxypropyl, 3-hydroxypropyl, hydroxyisopropyl, hydroxyisobutyl, 1-hydroxybutyl, 2-hydroxybutyl, 3-hydroxybutyl, 4-hydroxybutyl, hydroxy-sec-butyl and hydroxy-tert-butyl, but not being limited to the same.

In a second embodiment, the present invention presents a composition comprising a therapeutically effective amount of compound(s) of Formula (I) of the present invention or a pharmaceutically acceptable salt, hydrate, solvate, and isomer thereof; and one or more pharmaceutically acceptable excipients.

Pharmaceutically acceptable excipients are any substance, other than the active pharmaceutical ingredient, that has been evaluated for its safety and that is intentionally added to the dosage form. Such excipients are selected according to the pharmaceutical dosage form of interest, its route of administration, physicochemical compatibility with the active ingredient, and the effect on efficacy.

In addition, these excipients are widely known in the state of the art and are classified according to their function, including without limitation diluents, binders, disintegrants or disaggregators, lubricants, suspending agents, thickeners, solvents, surfactants, sliders, anti-caking agents or flow agents, glazing agents, plasticizers, sweeteners, isotonicity agents, dyes and pigments, preservatives, antioxidants, modifying agents or pH control, complexing agents, chelating agents, flavorings, viscosity modifying agents, opacifiers, permeation promoters, among others.

In an implementation of the second embodiment, pharmaceutical compositions can be administered by several routes including oral, sublingual, nasal, parenteral, injectable, submuscular, topical, transdermal, ocular, rectal, but not limited to the same.

In a third embodiment, the present invention presents the use of compound(s) of Formula (I) or a salt, hydrate, solvate and pharmaceutically acceptable isomer thereof to prepare a drug to treat pathologies related to neuropathic pain.

In an implementation of the third embodiment, said pathologies are selected from the group consisting of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia and postsurgical neuralgia.

In a fourth embodiment, the present invention presents a method of treatment, prevention, relief, suppression and/or control of pathologies related to neuropathic pain comprising the administration of an effective amount of Formula (I) compound(s) or a salt, hydrate, solvate and pharmaceutically acceptable isomer thereof.

In an implementation of the fourth embodiment, the method is for the treatment, prevention, relief, suppression and/or control of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia and postsurgical neuralgia.

In another implementation of the fourth embodiment, the administration of at least one Formula (I) compound is selected from the group comprising oral, sublingual, nasal, parenteral, injectable, submuscular, topical, transdermal, ocular and rectal routes.

In a fifth embodiment of the invention, processes for obtaining Formula (I) compounds are presented, comprising the following steps:

(a) formation of the Formula IV or Formula VI intermediate:

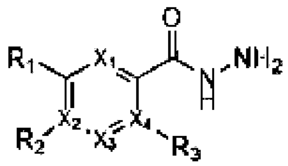

Formula IV

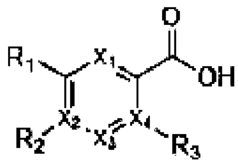

Formula VI

from the hydrazinolysis reaction or hydrolysis of a Formula V intermediate:

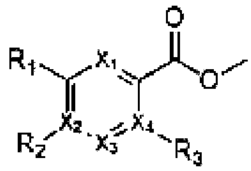

Formula V

(b) formation of the Formula II intermediate:

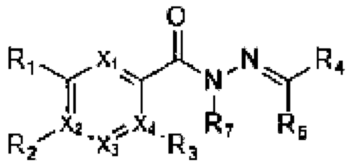

Formula II

from condensation between Formula III intermediates

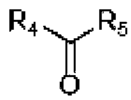

Formula III

and Formula IV intermediates with or without the presence of a catalyst and a suitable solvent;
(c) Formula I compound is obtained:

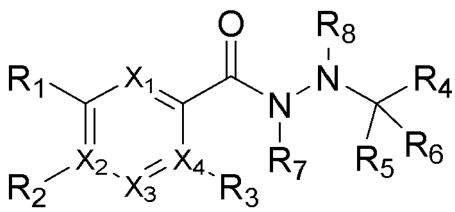

Formula (I)

from the reduction of Formula II intermediates or from reactions and amide coupling between intermediates VI and VII with or without the presence of a catalyst and a suitable solvent:

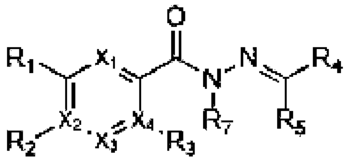

Formula II

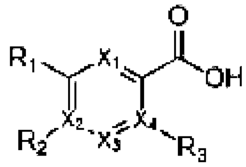

Formula VI

$RNH_2$ Formula VII

wherein:

- $X_1$, $X_2$, $X_3$, and $X_4$ are independently selected from the group consisting of carbon, CH, or nitrogen, wherein:
- when at least one of the substituents $X_2$, $X_4$ is N or CH, the corresponding R ($R_2$, $R_3$) is null;
- $R_1$ is:

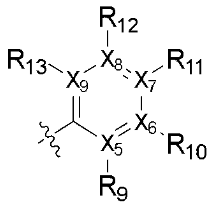

,

wherein:

- $X_5$, $X_6$, $X_7$, $X_8$, and $X_9$ are independently selected from the group consisting of carbon, CH, or nitrogen, wherein:

  - when at least one of the substituents $X_5$-$X_9$ is N or CH, the corresponding R ($R_9$-$R_{13}$) is null;
  - $R_9$, $R_{10}$, $R_{11}$, $R_{12}$, and $R_{13}$ are independently selected from the group consisting of hydrogen, halogen, linear or branched $C_1$-$C_6$ alkyl, linear or branched $C_1$-$C_6$ alkoxy, cycloalkoxy $C_3$-$C_7$, difluoromethyl, trifluoromethyl, linear or branched $C_1$-$C_6$ haloalkoxy;
  - $R_2$ and $R_3$ are independently selected from the group consisting of hydrogen, halogen, amine, hydroxy, and alkyl $C_1$-$C_6$ linear or branched, alkoxy $C_1$-$C_6$ linear or branched;
  - $R_4$ is:

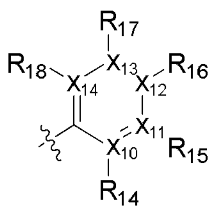

,

wherein:

- $X_{10}$, $X_{11}$, $X_{12}$, $X_{13}$, and $X_{14}$ are independently selected from the group consisting of carbon, CH, or nitrogen, wherein:

  - when at least one of the substituents $X_{10}$-$X_{14}$ is N or CH, the corresponding R ($R_{14}$-$R_{18}$) is null,
  - $R_{14}$, $R_{15}$, $R_{16}$, $R_{17}$, and $R_{18}$ are independently selected from the group consisting of hydrogen, halogen, hydroxy (alkyl $C_2$-$C_4$ linear or branched), alkyl $C_1$-$C_6$ linear or branched, alkoxy $C_1$-$C_6$ linear or branched;
  - $R_4$ and $R_8$ are optionally substituted to form a piperidine, homopiperidine, homomorpholine, or pyrrolidine;

- $R_5$ and $R_6$ are independently selected from the group consisting of hydrogen, linear or branched $C_1$-$C_6$ alkyl; and
- $R_7$ and $R_8$ are independently selected from the group consisting of hydrogen, linear or branched $C_1$-$C_6$ alkyl.

In an implementaton of the fifth embodiment, the process additionally comprises a step (d) of formation of compound(s) of Formula (I) in which $R_7$ is methyl from the reaction of N-alkylation of a compound obtained in step (c) with formaldehyde.

In an implementaton of the fifth embodiment, step (b) the compound of Formula III is a derivative of acetophenone. In another embodiment, the compound of Formula III is a derivative of benzaldehyde.

In an implementaton of the fifth embodiment, the reducing agent of step (c) is selected between triethylsilane or sodium borohydride. In another embodiment, in step (c) the acid is selected as TFA.

The compound(s) of Formula (I) of this invention have been prepared from the synthetic route described in General Scheme 1. However, those skilled in the art will readily notice that additional detailing and/or modifications to the arrangement of one or more steps can be accomplished without departing from the processes taught here. Such variations can be, without limitation, combinations of solvents and catalysts, including stereoselective ones, protective groups, among others.

In the following General Scheme 1, the formation of the Formula II, III, IV and V intermediates is described in addition to the formation of the Formula (I) compound(s), $R_7$ can be hydrogen or an alkyl group $C_{1-6}$.

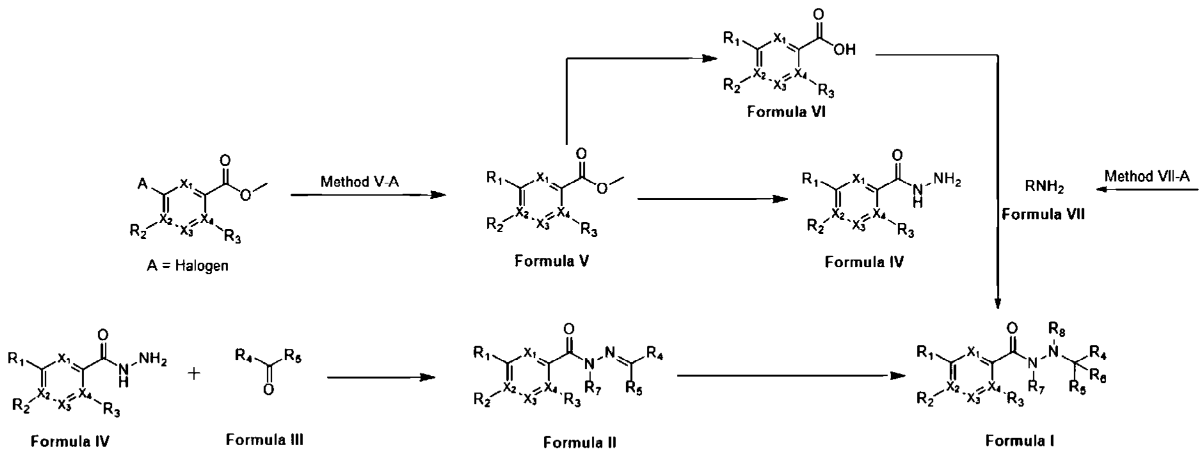

Formula VI
Formula V
Formula IV
Formula VII
Formula IV
Formula III
Formula II
Formula I

**GENERAL SCHEME 1**

As illustrated in General Scheme 1, Formula I compounds can be prepared from reduction reactions of Formula II intermediates without or by means of acid catalysis using concentrated hydrochloric acid, acetic acid, trifluoroacetic acid, or formic acid in suitable solvents such as THF. Formula II intermediates can be prepared by condensation reaction between Formula IV hydrazides and Formula III aldehydes or ketones. Formula IV intermediates can be prepared through hydrazinolysis reactions from the corresponding esters (Formula V) obtained following the methods described below. Formula III intermediates can be prepared from the corresponding reagents following the methods described below, some of which can be obtained commercially.

**FORMULA V INTERMEDIATES:**

Following General Scheme 1, the intermediates of Formula V are presented, however, such methods are not limiting.

**METHOD V-A**

*Intermediates V-1 to IV-18:*

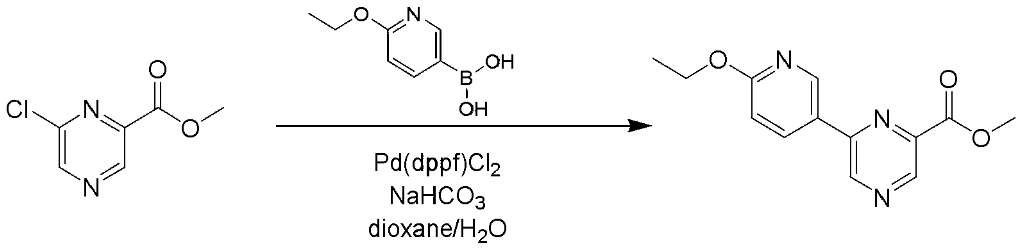


In a round-bottomed flask containing methyl 6-chloropyrazine-2-carboxylate (26.0 mmol) in dioxane (50.0 mL) and $H_2O$ (10.0 mL), boronic acid 6-(ethoxy-pyridin-3-yl) (28.6 mmol), $NaHCO_3$ (39.1 mmol) and Pd(dppf)$Cl_2$ (1.30 mmol) were added under an atmosphere of $N_2$. The reaction mixture was kept under agitation at 70 °C for 12 hours. After full consumption of the starting reagent (monitored by CCD), the mixture was concentrated under reduced pressure to remove the solvent. The residue obtained was diluted with $H_2O$ (100 mL) and extracted with AcOEt (100 mL x 3). The organic phase was concentrated under reduced pressure and purified by chromatography column (petroleum ether/AcOEt: 50/1 → 0/1). 4.50 g (61.3% yield) of the corresponding ester were obtained in the form of a white solid.

**Table 1.** Intermediates obtained from the corresponding reagents following Method V-A.

| Intermediat e | Structure | Intermediate | Structure |
|---|---|---|---|
| V-1 | 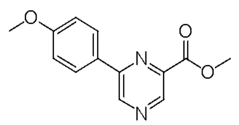 | V-10 | 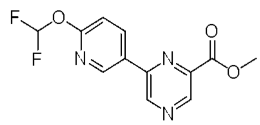 |
| V-2 | 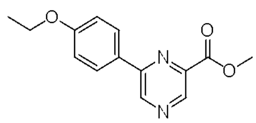 | V-11 | 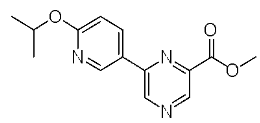 |
| V-3 | 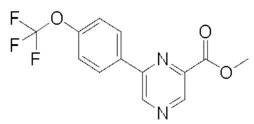 | V-12 | 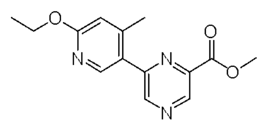 |
| V-4 | 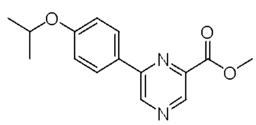 | V-13 | 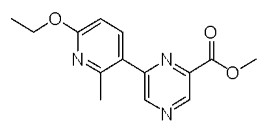 |
| V-5 | 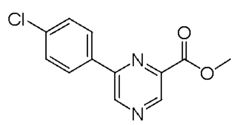 | V-14 | 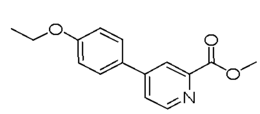 |
| V-6 | 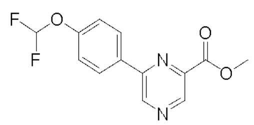 | V-15 | 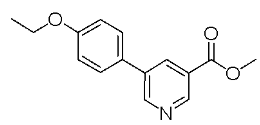 |
| V-7 | 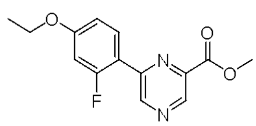 | V-16 | 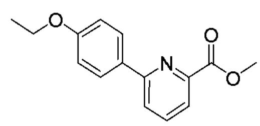 |
| V-8 | 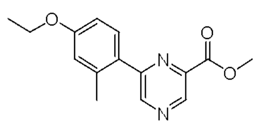 | V-17 | 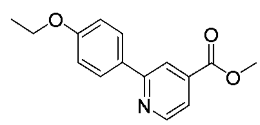 |

(continued)

| Intermediat e | Structure | Intermediate | Structure |
|---|---|---|---|
| V-9 | 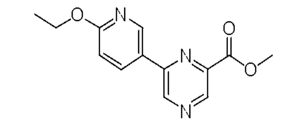 | V-18 | 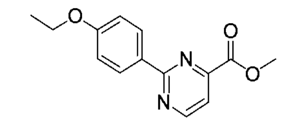 |

**METHOD V-B**

INTERMEDIATE V-19

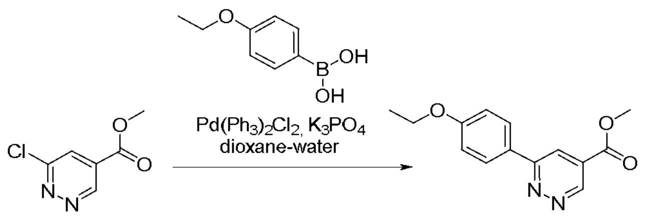


In a round-bottomed flask containing methyl 6-chloropyridazine-4-carboxylate (17.4 mmol) and (4-ethoxyphenyl)boronic acid (20.8 mmol) in dioxane (10.0 mL) and water (10 mL), $K_3PO_4$ (43.5 mmol) was added. The reaction mixture was degassed with $N_2$ and then bis(triphenylphosphine)palladium chloride (8.7 mmol) was added at room temperature. The reaction was maintained under agitation at 60 °C for 16 hours. After total consumption of the starting reagent (monitored by CCD), the mixture was filtered in celite and concentrated. The residue was diluted in water and extracted with AcOEt. The organic phase was concentrated and purified by chromatography column (petroleum ether/AcOEt 85/15) providing the product of interest in the form of a greenish solid at 40% yield (1.8 g).

**V-C METHOD**

**STEP V-C-I:**

**PRECURSOR V-20I:**

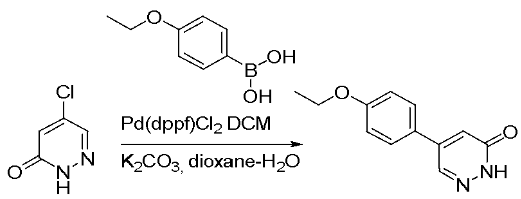


In a round-bottomed flask containing 5-chlorpyridazine-3(2*H*)-one (45.9 mmol) in dioxane (120.0 mL) and water (12 mL), (4-ethoxyphenyl)boronic acid (45.9 mmol), $K_2CO_3$ (68.9 mmol) and Pd(dppf)$Cl_2$.DCM (4.59 mmol) was added. The reaction was maintained under agitation at 90 °C for 16 hours. After total consumption of the starting reagent (monitored by CCD), the mixture was cooled to room temperature, diluted in ice water (50 mL) and extracted with AcOEt (150 mL x 2). The organic phase was concentrated resulting in a yellow solid. The compound obtained was used in the next step without further purification.

STEP V-C-II:

PRECURSOR V-20II:

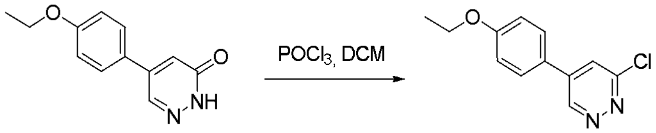


In a round-bottomed flask containing 5-(4-ethoxyphenyl) pyridazin-3(2*H*)-one (23.1 mmol) in DCM (100.0 mL), phosphoryl chloride (34.7 mmol) was added at 0 °C. The mixture was heated to 80 °C and kept under agitation for 16 hours. After total consumption of the starting reagent (monitored by CCD), the mixture was concentrated under reduced pressure, diluted in ice water (50 mL) and extracted with AcOEt (200 mL x 2). The organic phase was concentrated resulting in a yellow solid. The compound obtained was used in the next step without further purification.

STEP V-C-III:

INTERMEDIATE V-20:

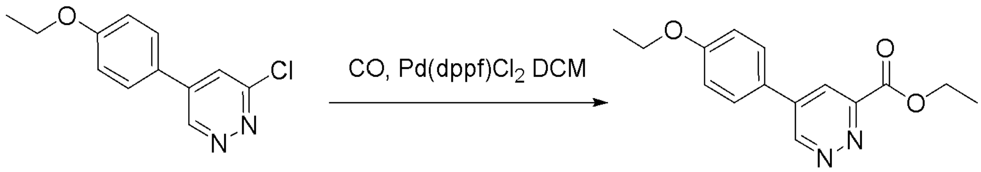


In a round-bottomed flask containing 3-chloro-5-(4-ethoxyphenyl) pyridazine (8.5 mmol) in ethanol (50.0 mL), triethylamine (3.59 mmol) and $Pd(dppf)Cl_2$.DCM (0.85 mmol) were added at room temperature and CO (100 Psi) was applied in an autoclave. The mixture was heated to 90 °C and kept under agitation for 16 hours. After total consumption of the starting reagent (monitored by CCD), the mixture was concentrated under reduced pressure, diluted in ice water (50 mL) and extracted with AcOEt (100 mL x 2). The organic phase was concentrated resulting in a brown solid. 1.6 g of the product of interest were obtained with a yield of 68.9%.

**METHOD V-D**

**STEP V-D-I:**

**PRECURSORS V-21I AND V-22I:**

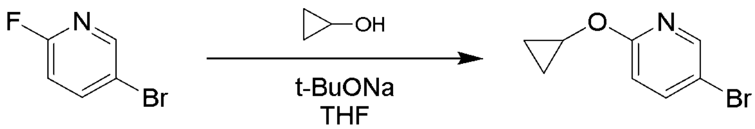


In a round-bottomed flask containing 5-bromo-2-fluoropyridine (28.4 mmol) in THF (50.0 mL), cyclopropanol (42.62 mmol) and *t*-BuONa (42.6 mmol) were added. The mixture was heated to 90 °C and kept under agitation for 16 hours. After total consumption of the starting reagent (monitored by LC-MS), the mixture was concentrated under reduced pressure, diluted in water (50 mL) and extracted with AcOEt (30 mL x 3). The organic phase was concentrated, and the residue was purified by chromatography column (petroleum ether/AcOEt: 50/1 → 5/1). The product of interest was obtained in 82.2% yield (5.0 g) in the form of a yellow oil.

**STEP V-D-II:**

**PRECURSORS V-21II AND V-22II:**

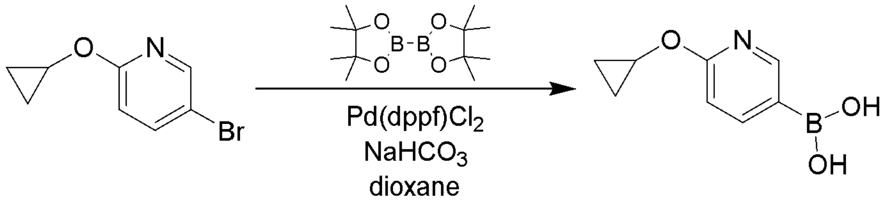


In a round-bottomed flask containing 5-bromo-2-cyclopropoxypyridine (23.4 mmol) in dioxane (50.0 mL), 4,4,5,5-tetramethyl-2-(4,4,5,5-tetramethyl-1,3,2-dioxaborolan-2-yl)-1,3,2-dioxaborolan (28.0 mmol), KOAc (70.1 mmol), and Pd(dppf)$Cl_2$ (2.34 mmol) were added. The mixture was heated to 90 °C and kept under agitation for 16 hours in an inert atmosphere. After total consumption of the starting reagent (monitored by LC-MS), the mixture was concentrated under reduced pressure, diluted in water (50 mL) and extracted with AcOEt (30 mL x 3). The organic phase was concentrated, and the residue was purified by chromatography column (petroleum ether/AcOEt: 50/1 → 5/1). The product of interest was obtained at 80.4% yield (5.6 g) in the form of a white solid.

**STEP V-D-III:**

**V-21 AND V-22 INTERMEDIATES:**

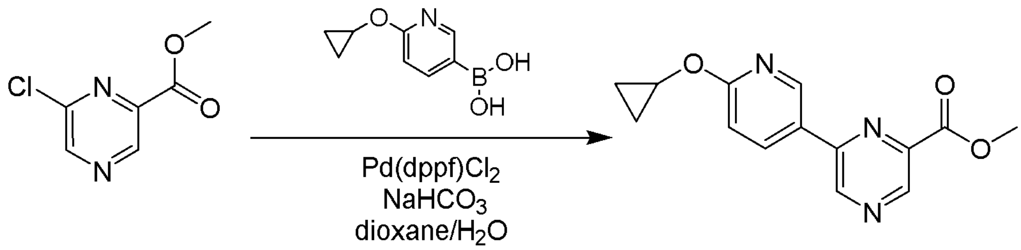


In a round-bottomed flask containing methyl 6-chloropyrazine-2-carboxylate (31.3 mmol) in dioxane (50.0 mL) and water (10 mL), (6-cyclopropoxypyridin-3-yl)boronic acid (37.5 mmol), $NaHCO_3$ (62.6 mmol) and Pd(dppf)$Cl_2$ (1.6 mmol) were added. The mixture was heated to 60 °C and kept under agitation for 12 hours in an atmosphere of $N_2$. After total consumption of the starting reagent (monitored by LC-MS), the mixture was concentrated under reduced pressure, diluted in water (50 mL) and extracted with AcOEt (30 mL x 3). The organic phase was concentrated, and the residue was purified by chromatography column (petroleum ether/AcOEt: 30/1 → 2/1). 5.0 g of the product of interest (49.5% yield) were obtained in the form of a yellow oil.

**TABLE 2. INTERMEDIATES OBTAINED FROM THE CORRESPONDING REAGENTS FOLLOWING *STEP V-D-III.***

| Intermediate | Structure |
|---|---|
| V-21 | 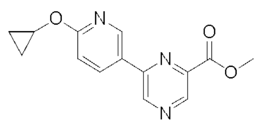 |
| V-22 | 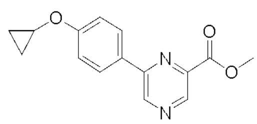 |

**V-E METHOD**

**STEP V-E-I:**

**PRECURSOR V-22I:**

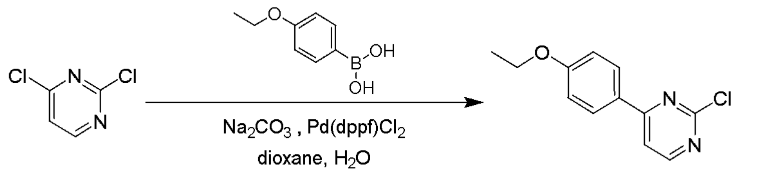


In a round-bottomed flask containing 2,4-dichloropyrimidinemethyl (67.1 mmol) in dioxane (100 mL) and water (30 mL), (4-ethoxyphenyl)boronic acid (67.1 mmol), $NaHCO_3$ (201 mmol) and $Pd(dppf)Cl_2$ (671 μmol) were added. The mixture was heated to 60 °C and kept under agitation for 16 hours in an atmosphere of $N_2$. After total consumption of the starting reagent (monitored by LC-MS), the mixture was concentrated under reduced pressure, diluted in water (150 mL) and extracted with AcOEt (50 mL x 3). The organic phase was concentrated, and the residue was purified by chromatography column (petroleum ether/AcOEt: 20/1 → 1/1). 8.9 g of the product of interest (56.5% yield) were obtained in the form of a white solid.

**STEP V-E-II:**

**INTERMEDIATE V-22:**

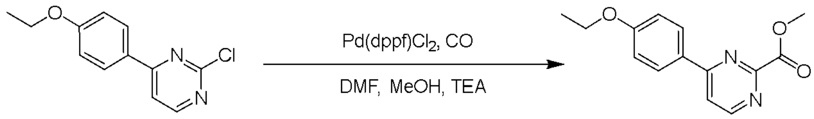


In a round-bottomed flask containing 2-chloro-4-(4-ethoxyphenyl)pyrimidine (21.3 mmol) in methanol (25 mL) and DMF (25 mL), triethylamine (25 mL) and $Pd(dppf)Cl_2$ (1.07 mmol) were added. The mixture was heated to 80 °C, CO (50 Psi) was applied and kept under agitation for 16 hours. After total consumption of the starting reagent (monitored by LC-MS), the mixture was concentrated under reduced pressure, diluted in water (80 mL) and extracted with AcOEt (20 mL x 3). The organic phase was concentrated, and the residue was purified by chromatography column (petroleum ether/AcOEt: 20/1 → 1/1). 4.45 g of the product of interest (80.9% yield) were obtained in the form of a brown solid.

**FORMULA IV INTERMEDIATES:**

Following General Scheme 1, the intermediates of Formula IV are presented, however, such methods are not limiting.

**METHOD IV-A**

**INTERMEDIATES IV-1 TO IV-21:**

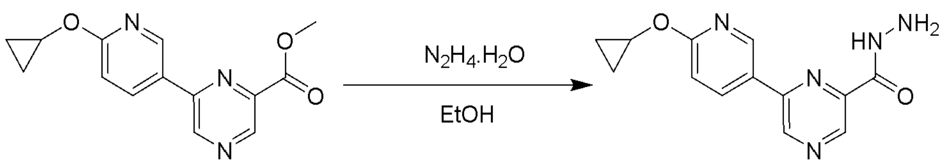


In a round-bottomed flask containing methyl 6-(6-cyclopropoxypyridin-3-yl)pyrazine-2-carboxylate (18.4 mmol) in EtOH (35.0 mL) $N_2H_4 \cdot H_2O$ (56.7 mmol) was added. The reaction mixture was kept under agitation at 90 °C for 12 hours. After total consumption of the starting reagent (monitored by CCD), the mixture was filtered and concentrated. 3.80 g (76% yield) of the hydrazide of interest were obtained in the form of a white solid, which was sent to the next step without further

purification.

**TABLE 3. INTERMEDIATES OBTAINED FROM THE CORRESPONDING REAGENTS FOLLOWING METHOD IV-A.**

| Intermediat e | Structure | Intermediate | Structure |
|---|---|---|---|
| IV-1 | 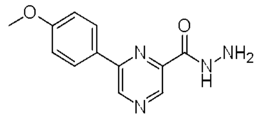 | IV-12 | 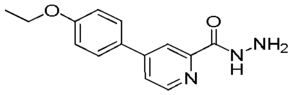 |
| IV-2 | 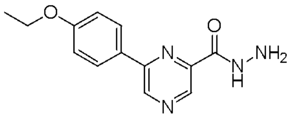 | IV-13 | 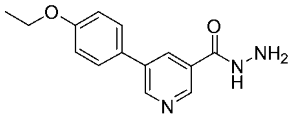 |
| IV-3 | 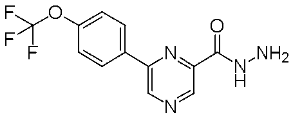 | IV-14 | 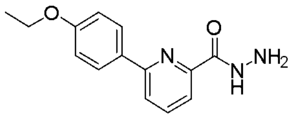 |
| IV-4 | 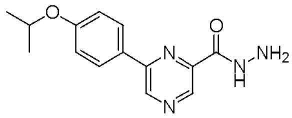 | IV-15 | 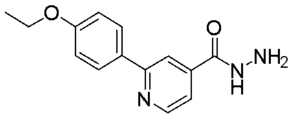 |
| IV-5 | 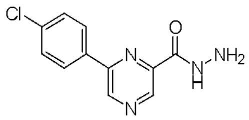 | IV-16 | 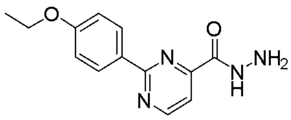 |
| IV-6 | 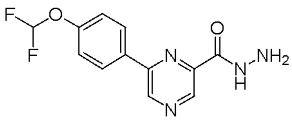 | IV-17 | 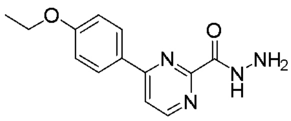 |
| IV-7 | 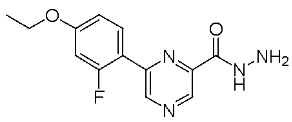 | IV-18 | 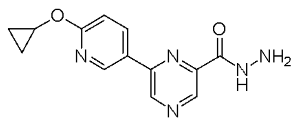 |
| IV-8 | 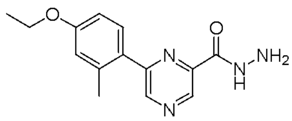 | IV-19 | 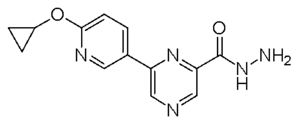 |
| IV-9 | 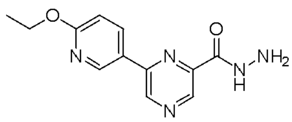 | IV-20 | 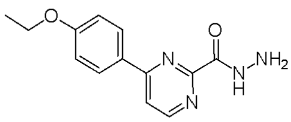 |
| IV-10 | 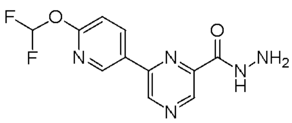 | IV-21 | 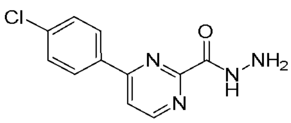 |
| IV-11 | 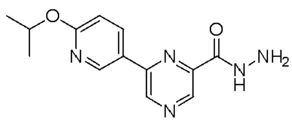 | | |

**METHOD IV-B**

**STEP IV-B-I:**

**PRECURSORS IV-22I AND IV-23I:**

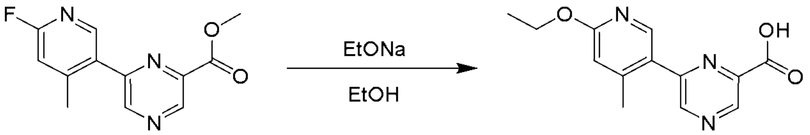


In a round-bottomed flask containing methyl 6-(6-fluoro-4-methylpyridin-3-yl)pyrazine-2-carboxylate (19.4 mmol) in EtOH (50.0 mL), EtONa (135.9 mmol) was added. The reaction mixture was kept under agitation at 90 °C for 16 hours. After total consumption of the starting reagent (monitored by LC-MS), the mixture was concentrated under reduced pressure, diluted in water (50 mL) and extracted with AcOEt (30 mL x 3). The organic phase was concentrated and the residue obtained was used directly in the next step without further purification. 2.5g of the product of interest (24.8% yield) were obtained in the form of a yellow solid.

**TABLE 4. PRECURSORS OBTAINED FROM THE CORRESPONDING REAGENTS FOLLOWING STEP IV-B-I.**

| Precursor | Structure |
|---|---|
| *IV-22i* | 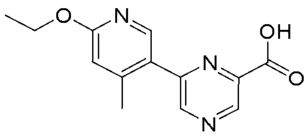 |
| *IV-22i* | 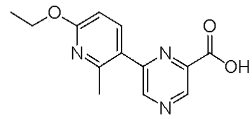 |

**STEP IV-B-II:**

**PRECURSORS IV-22II AND IV-23II:**

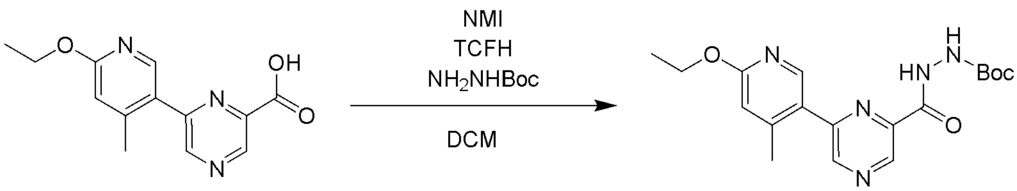


In a round-bottomed flask containing 6-(6-ethoxy-4-methylpyridin-3-yl)pyrazine-2-carboxylic acid (4.82 mmol) in DCM (20.0 mL) *tert*-butyl*N*-aminocarbamate (5.06 mmol), TCFH (7.23 mmol), and NMI (9.64 mmol) were added. The reaction mixture was kept under agitation at room temperature for 3 hours. After total consumption of the starting reagent (monitored by LC-MS), the mixture was concentrated under reduced pressure, diluted in water (20 mL) and extracted with AcOEt (10 mL x 3). The organic phase was concentrated, and the residue was purified by chromatography column (petroleum ether/AcOEt: 30/1 → 2/1) and by HPLC prep [$H_2O(NH_4CO_3)$-ACN] (gradient 30%-60% B). 3.74 g of the product of interest (77.6% yield) were obtained in the form of a yellow oil.

**TABLE 5. PRECURSORS OBTAINED FROM THE CORRESPONDING REAGENTS FOLLOWING STEP IV-B-II.**

| Precursor | Structure |
|---|---|
| *IV-22ii* | 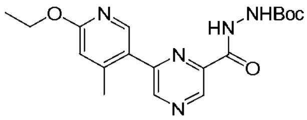 |
| *IV-22ii* | 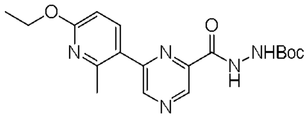 |

**STEP IV-B-III:**

**INTERMEDIATES IV-22 AND IV-23:**

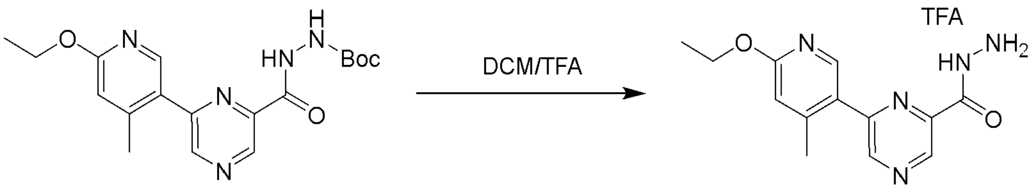


In a round-bottomed flask containing *tert*-butyl 2-(6-(6-ethoxy-4-methylpyridin-3-yl)pyrazine-2-carbonyl)hydrazine-1-carboxylate (3.75 mmol) in DCM (15.0 mL) TFA (5.0 mL) was added. The reaction mixture was kept under agitation at room temperature for 1 hour. After total consumption of the starting reagent (monitored by CCD), the mixture was concentrated under reduced pressure. 2.2 g of the product of interest (90.9% yield) were obtained in the form of a black solid that was used in the next step without further purification.

**TABLE 6. INTERMEDIATES OBTAINED FROM THE CORRESPONDING REAGENTS FOLLOWING STEP IV-B-III.**

| Intermediate | Structure |
|---|---|
| IV-22 | 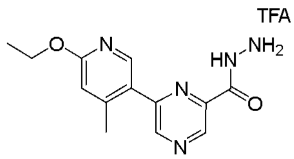 |
| IV-23 | 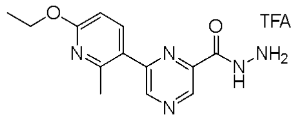 |

**FORMULA VI INTERMEDIATES:**

Following General Scheme 1, Formula VI intermediates are presented, but such methods are not limiting.

**METHOD VI-A**

**INTERMEDIATES VI-1 TO VI-3:**

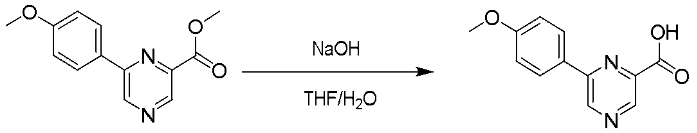


In a round-bottomed flask containing methyl 6-(4-methoxyphenyl)pyrazine-2-carboxylate (15.2 mmol) in THF (50.0 mL), NaOH (22.9 mmol) was added to water (10.0 mL). The reaction mixture was kept under agitation at room temperature for 12 hours. After total consumption of the starting reagent (monitored by LC-MS), the pH was adjusted to 5 with 2N HCL, and the precipitate formed was filtered. The filtrate was concentrated and used directly in the next step without further purification. 3.5 g of the product of interest (97.6% yield) were obtained in the form of a white solid.

**TABLE 7. INTERMEDIATES OBTAINED FROM THE CORRESPONDING REAGENTS FOLLOWING METHOD VI-A.**

| Intermediate | Structure |
|---|---|
| IV-1 | 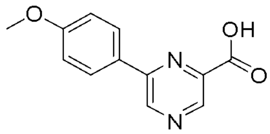 |
| IV-2 | 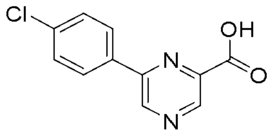 |
| IV-3 | 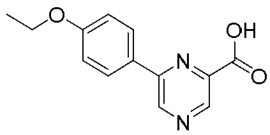 |

**FORMULA VII INTERMEDIATES:**

Following General Scheme 1, the intermediates of Formula VII are presented, however, such methods are not limiting.

**METHOD VII-A**

INTERMEDIATE VII-1:

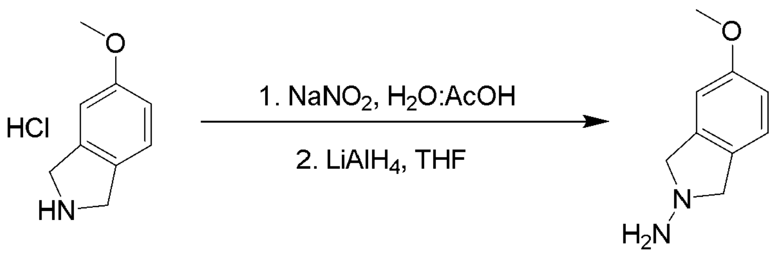


In a round-bottomed flask containing a solution of $NaNO_2$ (2.00 eq.) in $H_2O$ (30 mL), 5-methoxyisoindoline hydrochloride (1.00 eq., HCl) was added. Next, AcOH (3 mL) was added at 20 °C and the reaction mixture was kept under agitation for 2 hours. After total consumption of the starting reagent (monitored by CCD), the mixture was filtered and the residue obtained was lyophilized. Then, the crude reaction was dissolved in THF (45.0 mL) and $LiAlH_4$ (2.0 eq.) was added in portions to the reaction balloon. The reaction mixture was kept under agitation at 20 °C for 3 hours. After total consumption of the starting reagent (monitored by CCD and LC-MS), $NaNO_2$ (2.0 eq.) was added in $H_2O$, the reactive crude was filtered under celite and the residue obtained was purified by HPLC ([$H_2O$( $NH_4HCO_3$) - ACN]; B%: 10%-40%).

**METHOD VII-B**

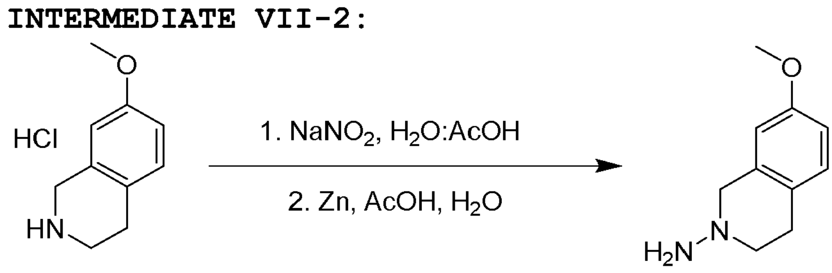


Step 1 was carried out by means of an experimental procedure equivalent to Step 1 of Intermediate VII-1 - Method VII-A, through the use of the corresponding starting materials. Then, the crude reaction was dissolved in AcOH (10.0 mL) and $H_2O$ (10.0 mL) and Zn (5.00 eq.) was added in portions to the reaction balloon. The reaction mixture remained at 20 °C for 16 hours. After total consumption of the starting reagent (monitored by CCD and LC-MS), the mixture was filtered under celite and the residue obtained was purified by HPLC ([$H_2O$(HCl)-ACN] 1%→ 21% B). Next, MeCN was evaporated, and the obtained product was lyophilized.

**METHOD VII-C**

**STEP VII-C-I:**

**PRECURSOR VII-3I:**

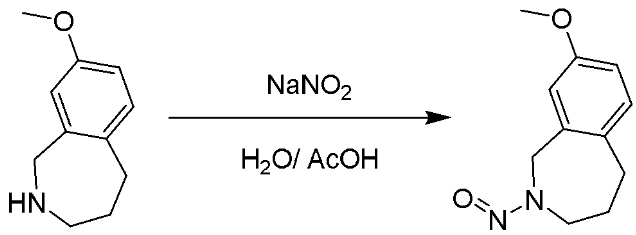


In a round-bottomed flask containing 8-methoxy-2,3,4,5-tetrahydro-1*H*-benzo[c]azepine (5.64 mmol) in $H_2O$ (10.0 mL) and AcOH (10.0 mL), $NaNO_2$ (16.9 mmol) was added. The reaction mixture remained at 0 °C for 3 hours. After total consumption of the starting reagent (monitored by LC-MS), the pH of the mixture was adjusted to 8 by adding an aqueous solution of $Na_2CO_3$. The residue obtained was diluted with $H_2O$ (20.0 mL) and extracted with AcOEt (20.0 mL x 3). The organic phase was concentrated under reduced pressure. 1.00 g (85.9% yield) of the product of interest were obtained in the form of a yellow oil, which was used for the next step without further purification.

**STEP VII-C-II:**

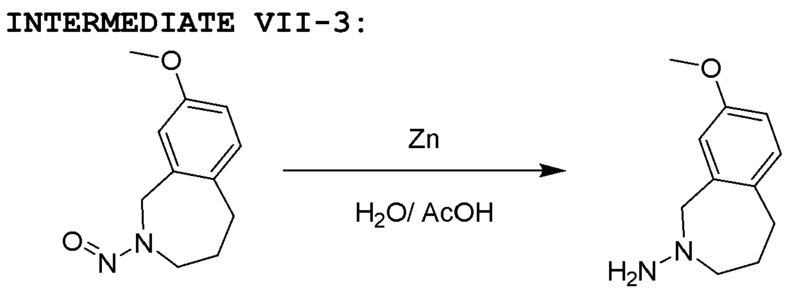

In a round-bottomed flask containing 8-methoxy-2-nitrous-2,3,4,5-tetrahydro-1*H*-benzo[c]azepine (4.85 mmol) in AcOH (10.0 mL) and $H_2O$ (10.0 mL), Zn (24.2 mmol) was added in portions at 0 °C. The reaction mixture remained at 20 °C for 16 hours. After total consumption of the starting reagent (monitored by CCD and LC-MS), the mixture was filtered, and the pH of the organic phase was adjusted to 8 by adding an aqueous solution of $Na_2CO_3$. The residue obtained was diluted with $H_2O$ (20.0 mL) and extracted with AcOEt (10.0 mL x 3). The organic phase was concentrated under reduced pressure. 700 mg (75.0% yield) of the product of interest were obtained in the form of a yellow oil, which was used for the next step without further purification.

**METHOD VII-D**

**STEP VII-D-I:**

**PRECURSOR VII-4I:**

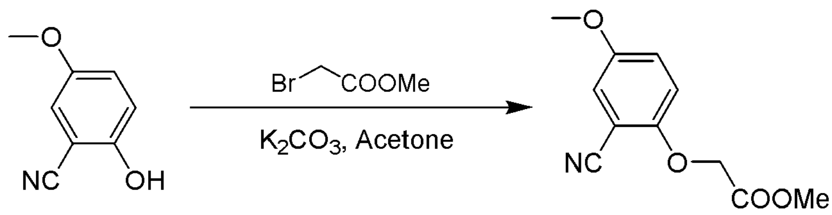


In a round-bottomed flask containing 2-hydroxy-5-methoxybenzonitrile (33.5 mmol) and $K_2CO_3$ (50.2 mmol) in acetone (50.0 mL), methyl 2-bromoacetate (36.8 mmol) was added dropwise at 20 °C. The reaction mixture remained at 20 °C for 16 hours. After total consumption of the starting reagent (monitored by CCD and LC-MS), the mixture was diluted with $H_2O$ (100 mL) and extracted with AcOEt (50.0 mL x 3). The organic phase was concentrated under reduced pressure. 7.40 g (99.7% yield) of the product of interest were obtained in the form of a white solid, which was used for the next step without further purification.

**STEP VII-D-II:**

**PRECURSOR VII-4II:**

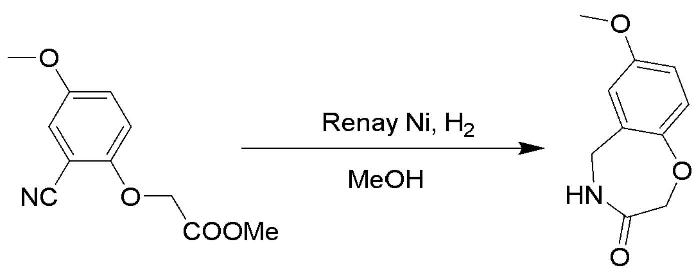


In a round-bottomed flask containing 2-(2-cyano-4-methoxyphenoxy)methyl acetate (33.4 mmol) in MeOH (70.0 mL), Raney-Ni (172 mmol) and $NH_3 \cdot H_2O$ (33.4 mmol) was added under the atmosphere of $N_2$. The suspension was degassed and purged with $H_2$. The mixture was kept in agitation under an atmosphere of $H_2$ (50 psi) at 50 °C for 6 hours. After total consumption of the starting reagent (monitored by LC-MS), the mixture was filtered, washed with MeOH (50.0 mL x 3) and concentrated. 6.00 g (92.8% yield) of the product of interest were obtained in the form of a white solid, which was used for the next step without further purification.

**STEP VII-D-III:**

**PRECURSOR VII-4III:**

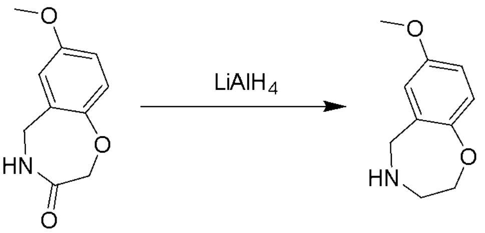


In a round-bottomed flask containing 7-methoxy-4,5-dihydrobenzo [1,4]oxazepine-3(2*H*)-one (10.3 mmol) in THF (20 mL), $LiAlH_4$ (41.4 mmol) was added at 0 °C. Subsequently, the reaction mixture was kept in agitation at 70 °C for 12 hours. After total consumption of the starting reagent (monitored by LC-MS), $H_2O$ (2.0 mL) and NaOH aqueous solution (2.0 mL) were added to the reaction mixture. Next, the mixture was diluted with $H_2O$ (10.0 mL) and extracted with AcOEt (10.0 mL x 3). The organic phase was concentrated under reduced pressure. 1.80 g (97.02% yield) of the product of interest were obtained in the form of a yellow solid, which was used for the next step without further purification.

**STEP VII-D-IV:**

**PRECURSOR VII-4IV:**

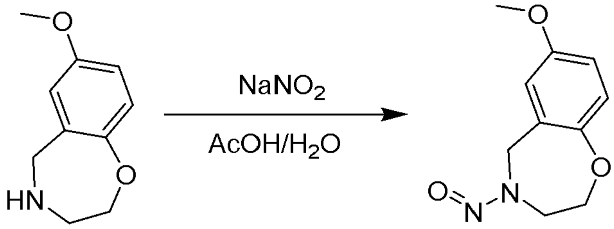


In a round-bottomed flask containing 7-methoxy-2,3,4,5-tetrahydrobenzo[1,4]oxazepine (10.0 mmol) in AcOH (10.0 mL), $NaNO_2$ (30.1 mmol) was added. The reaction mixture remained at 0 °C for 3 hours. After total consumption of the starting reagent (monitored by LC-MS), the pH of the mixture was adjusted to 8 by adding an aqueous solution of $Na_2CO_3$. The residue obtained was diluted with $H_2O$ (50.0 mL) and extracted with AcOEt (30.0 mL x 3). The organic phase was concentrated under reduced pressure. 1.80 g (86.0% yield) of the product of interest were obtained in the form of a yellow solid, which was used for the next step without further purification.

**STEP VII-D-V:**

**INTERMEDIATE VII-4:**

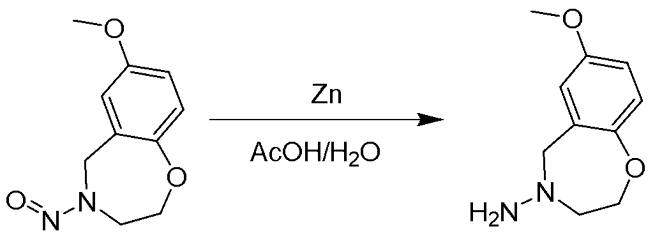


In a round-bottomed flask containing 7-methoxy-4-nitrous-2,3,4,5-tetrahydrobenzo[1,4]oxazepine (8.64 mmol) in AcOH (10.0 mL) and $H_2O$ (10.0 mL), Zn (48.1 mmol) was added in portions at 0 °C. The reaction mixture remained at 20 °C for 16 hours. After total consumption of the starting reagent (monitored by LC-MS), the mixture was filtered, and the pH of the organic phase was adjusted to 8 by adding an aqueous solution of $Na_2CO_3$. The residue obtained was diluted with $H_2O$ (20.0 mL) and extracted with AcOEt (20.0 mL x 3). The organic phase was concentrated under reduced pressure. 700 mg (41.6% yield) of the product of interest were obtained in the form of a yellow oil, which was used for the next step without further purification.

**FORMULA II INTERMEDIATES:**

Following General Scheme 1, the Formula II intermediates are presented, however such methods are not exhaustive.

**METHOD II-A**

**INTERMEDIATES II-1 TO II-23:**

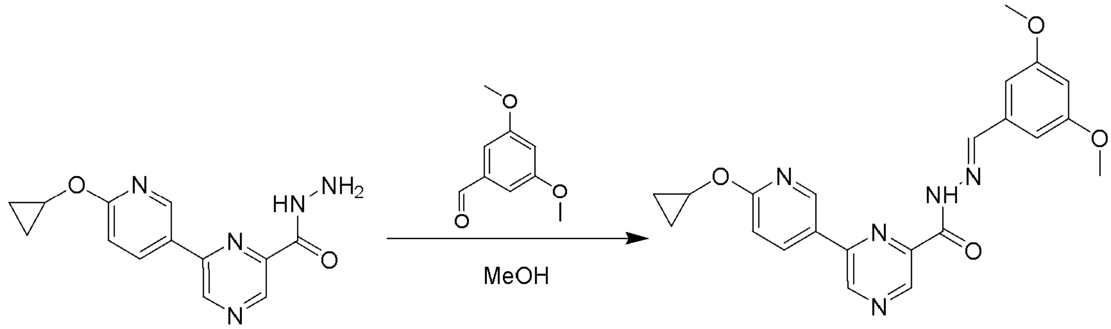


In a round-bottomed flask containing 6-(6-cyclopropoxypyridin-3-yl)pyrazine-2-carbohydrazide (1.11 mmol) in methanol (3 mL) 3,5-dimethoxybenzaldehyde (1.16 mmol) was added. The reaction mixture was kept under agitation at 60 °C for 12 hours. Upon conclusion of the reaction, the mixture was filtered, the residue washed with MeOH (5 mL) and the filtrate was concentrated under reduced pressure. 400 mg of the product of interest (86.1% yield) were obtained in the form of a white solid that was used for the next step without further purification.

**TABLE 8. INTERMEDIATES OBTAINED FROM THE CORRESPONDING REAGENTS FOLLOWING METHOD IIA.**

| Intermedia te | Structure | Intermediate | Structure |
|---|---|---|---|
| II-1 | 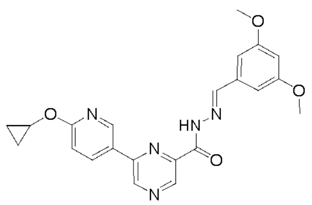 | II-12 | 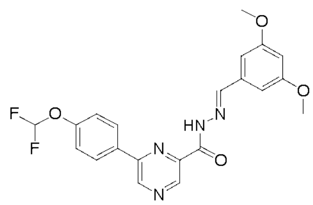 |
| II-2 | 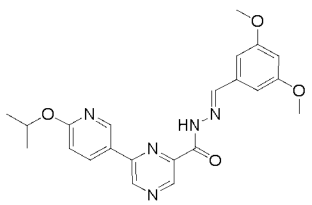 | II-13 | 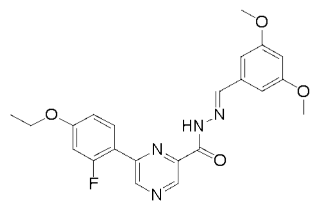 |
| II-3 | 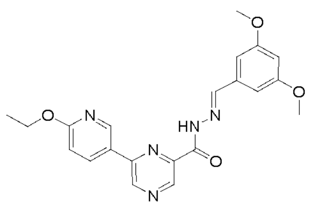 | II-14 | 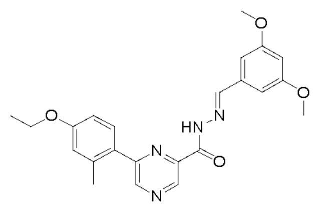 |

(continued)

| Intermedia te | Structure | Intermediate | Structure |
|---|---|---|---|
| II-4 | 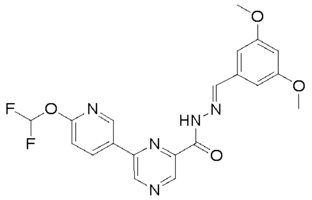 | II-15 | 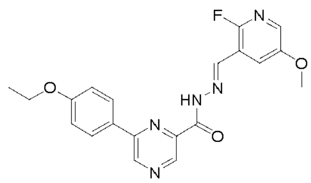 |
| II-5 | 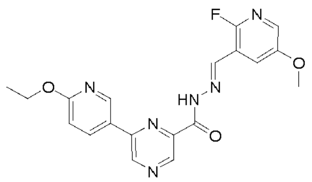 | II-16 | 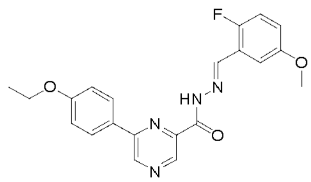 |
| II-6 | 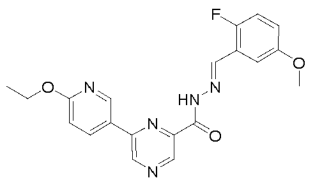 | II-17 | 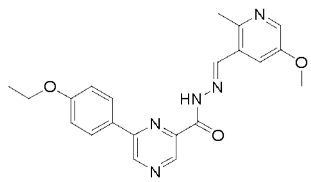 |
| II-7 | 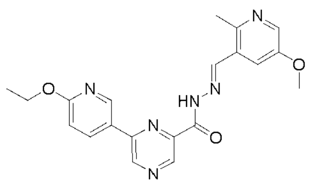 | II-18 | 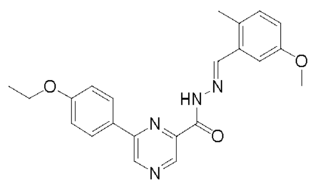 |
| II-8 | 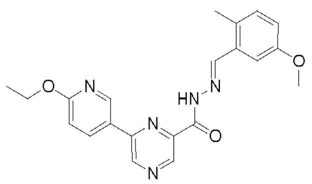 | II-19 | 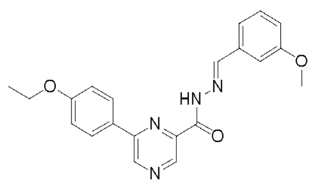 |
| II-9 | 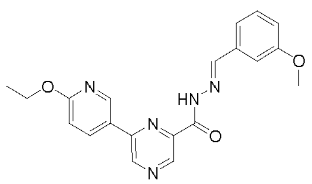 | II-20 | 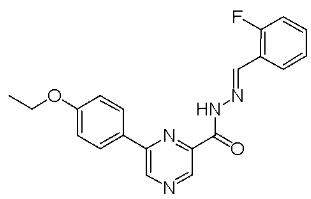 |
| II-10 | 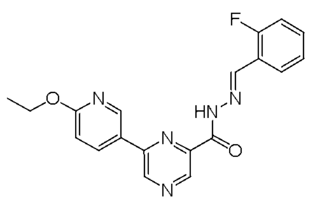 | II-21 | 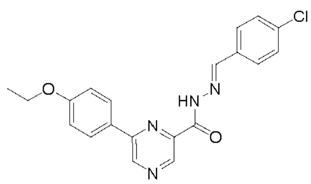 |
| II-11 | 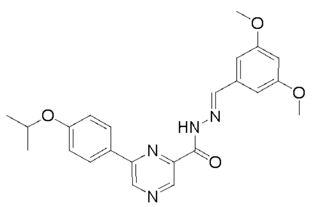 | II-22 | 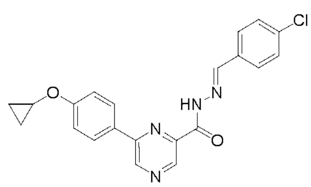 |

(continued)

| Intermedia te | Structure | Intermediate | Structure |
|---|---|---|---|
| II-12 | 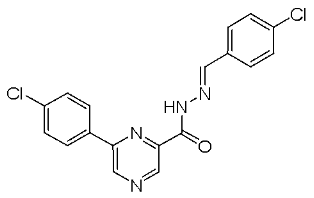 | II-23 | 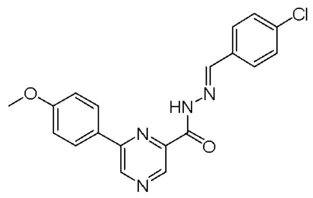 |

**METHOD II-B**

**INTERMEDIATES II-24 AND II-25:**

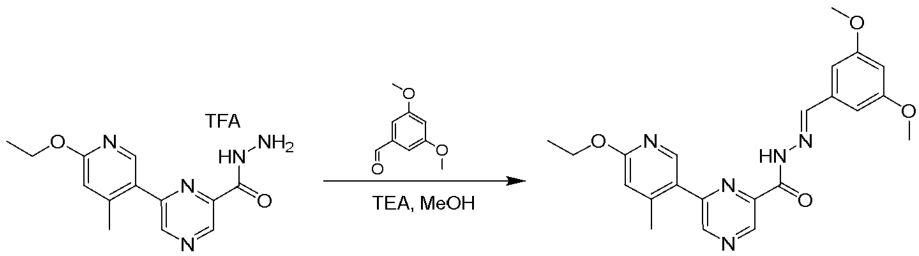


In a round-bottomed flask containing 6-(6-ethoxy-4-methylpyridin-3-yl)pyrazine-2-carbohydrazide (3.41 mmol) in methanol (20 mL), TEA (3.41 mmol) and 3,5-dimethoxybenzaldehyde (3.75 mmol) were added. The reaction mixture was kept under agitation at 50 °C for 12 hours. Upon conclusion of the reaction, the mixture was filtered, the residue washed with MeOH (5 mL) and the filtrate was concentrated under reduced pressure. 1.2 g of the product of interest (80.2% yield) were obtained in the form of a white solid that was used for the next step without further purification.

**TABLE 9. INTERMEDIATES OBTAINED FROM THE CORRESPONDING REAGENTS FOLLOWING METHOD II-B.**

| Intermediate | Structure |
|---|---|
| II-24 | 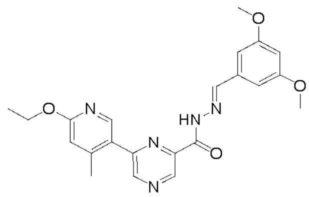 |
| II-25 | 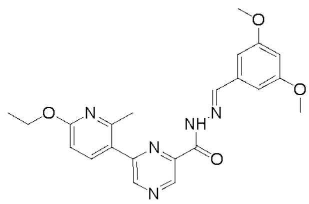 |

**METHOD II-C**

**INTERMEDIATE II-26:**

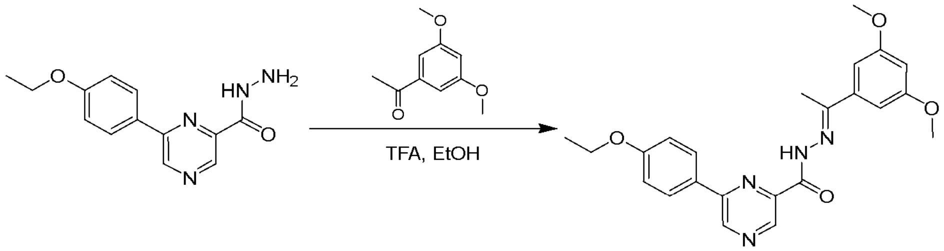


In a round-bottomed flask containing 6-(4-ethoxyphenyl)pyrazine-2-carbohydrazide (3.87 mmol) in ethanol (15 mL), TFA (7.74 mmol), 1-(3,5-dimethoxyphenyl)ethane (4.07 mmol) and 4A molecular sieve (3.87 mmol) were added. The reaction mixture was kept under agitation at 90 °C for 16 hours. Upon conclusion of the reaction, the mixture was diluted in DCM (20 mL), the precipitate formed was filtered and washed with DCM and the resulting filtrate was concentrated under reduced pressure. 1.55 g of the product of interest (95.2% yield) were obtained in the form of a white solid that was used for the next step without further purification.

**METHOD II-D**

**INTERMEDIATES II-27 TO II-32:**

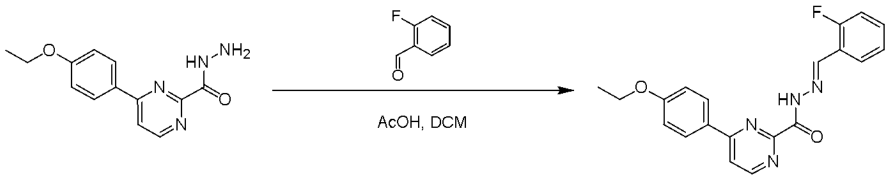


In a round-bottomed flask containing 4-(4-ethoxyphenyl)pyrimidine-2-carbohydrazide (3.10 mmol) and 2-fluorobenzaldehyde (3.25 mmol) in DCM (8.0 mL) AcOH (3.10 mmol) was added. The reaction mixture was kept under agitation at room temperature for 1 hour. Upon conclusion of the reaction, the mixture was concentrated and the residue obtained was crushed with MTBE (5.0 mL). 1.1 g of the product of interest (97.5% yield) were obtained in the form of a white solid.

**TABLE 10. INTERMEDIATES OBTAINED FROM THE CORRESPONDING REAGENTS FOLLOWING METHOD II-D.**

| Intermedia te | Structure | Intermediate | Structure |
|---|---|---|---|
| II-27 | 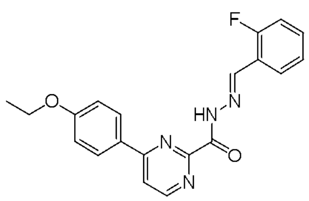 | II-30 | 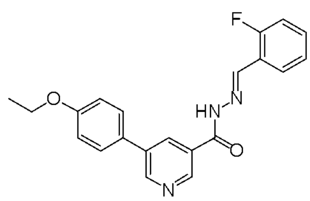 |
| II-28 | 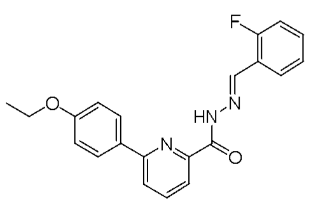 | II-31 | 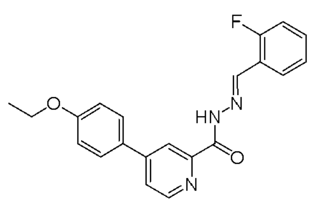 |

(continued)

| Intermedia te | Structure | Intermediate | Structure |
|---|---|---|---|
| II-29 | 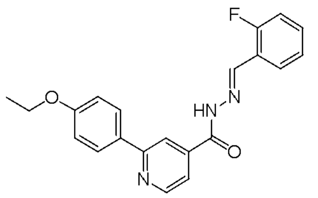 | II-32 | 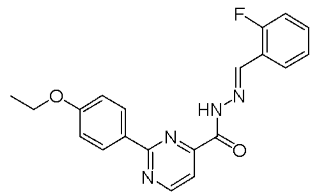 |

## EXAMPLES

The following examples, described in detail, serve to illustrate the embodiments of this invention without, however, having any limitation character to the scope of protection thereof.

The compound(s) of General Formula I of this invention were obtained from the reduction of Formula II intermediates using or not acid catalysis and reducing agents, as described above. The preparation methodologies for intermediates II-V are outlined in General Scheme 1, however they are not limited thereto.

### EXAMPLE 1. 6-(6-CYCLOPROPOXYPYRIDIN-3-IL)-*N*'-(3,5-DIMETHOXYBENZYL) PYRAZINE-2-CARBOHYDRAZIDE.

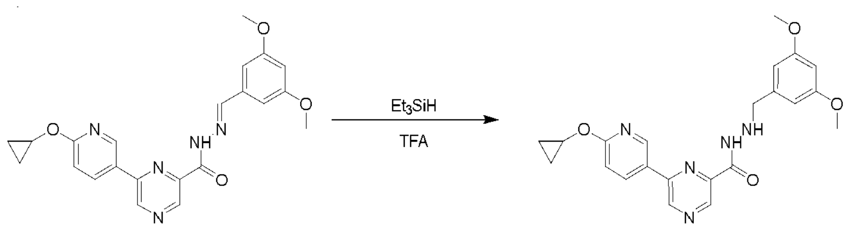


In a round-bottomed flask containing (E)-6-(6-cyclopropoxypyridin-3-yl)-*N*'-(3,5-dimethoxybenzylidene)pyrazine-2-carbohydrazide (953.7 μmol) in TFA (4.0 mL), $Et_3SiH$ (1.91 mmol) was added. The reaction mixture was kept at room temperature for 16 hours. After total consumption of the starting reagent (monitored by CCD), the mixture was concentrated under reduced pressure, the residue was diluted in $H_2O$ (10 mL), the pH was adjusted to 8 by adding an aqueous solution of $Na_2CO_3$, and the mixture was extracted with AcOEt (5.0 mL x 3). The organic phase was concentrated under reduced pressure. The residue obtained was purified by prep-TLC (petroleum ether/AcOEt: 20/1 → 2/1). 260 mg (64.6% yield) of the product of interest were obtained in the form of a yellow solid.

Compounds 1-5, 7-10, 14-16, 22, 24-33, 39-44, 49 and 51-53, as shown in Table 11, are obtained by the procedure described for Example 1, changing the corresponding intermediates II and III.

### EXAMPLE 2. 6- (4-ETHOXYPHENYL)-*N*'-((5-METHOXY-2-METHYLPYRIDINE-3-YL)METHYL)PYRAZINE-2-CARBOHYDRAZIDE.

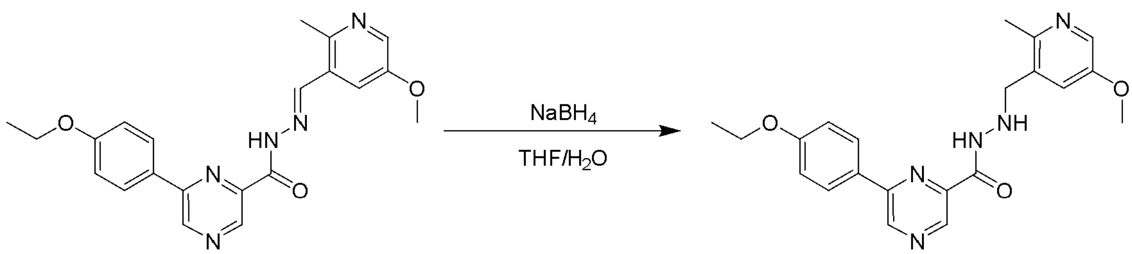


In a round-bottomed flask containing (*E*)-6-(4-ethoxyphenyl)-*N*'-((5-methoxy-2-methylpyridin-3-yl)methylene) pyrazine-2-carbohydrazide (1.15 mmol) in THF (50.0 mL) and $H_2O$ (10.0 mL), $NaBH_4$ (6.34 mmol) was added. The

reaction mixture was kept at room temperature for 16 hours. After total consumption of the starting reagent (monitored by CCD), the mixture was cooled to 0 °C and the pH was adjusted to 8 by adding HCL solution (2N). The formed precipitate was filtered and crushed with DCM/methanol (10/1). 200 mg (42.7% yield) of the product of interest were obtained in the form of a yellow solid.

Compounds 18, 34, 48, 50, as shown in Table 11, are obtained by the procedure described for Example 1, changing the corresponding intermediates II and III.

**EXAMPLE 3. 6-(4-ETHOXYPHENYL)-N'-(2-FLUOROBENZYL)PYRIDAZINE-4-CARBOHYDRAZIDE.**

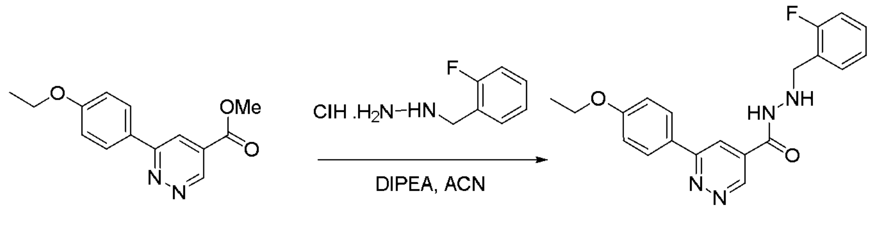


In a round-bottomed flask containing methyl 6-(4-ethoxyphenyl)pyridazine-4-carboxylate (3.87 mmol) and (2-fluorobenzyl)hydrazine hydrochloride (4.64 mmol) in ACN (10.0 mL) and $H_2O$ (10.0 mL), DIPEA (5.0 mL) was added. The reaction mixture remained at 100 °C for 16 hours. After total consumption of the starting reagent (monitored by CCD), the mixture was diluted in water (25 mL) and extracted with DCM (25 mL x 3). The organic phase was concentrated under reduced pressure and the residue was purified by prep-HPLC. 160 mg (11.2% yield) of the product of interest were obtained in the form of a white solid.

Compounds 46 and 47, as shown in Table 11, are obtained by the procedure described for Example 1, changing the corresponding intermediates II and III.

**EXAMPLE 4. N-(6-CHLORO-3,4-DIHYDROISOQUINOLINE-2(1H)-IL)-6-(4-CHLOROPHENYL)PYRAZINE-2-CARBOXAMIDE**

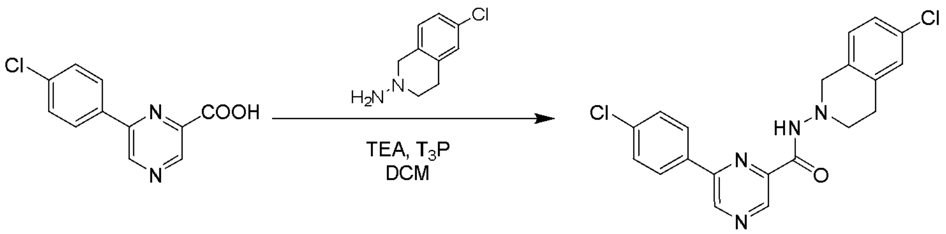


In a round-bottomed flask containing 6-(4-chlorophenyl)pyrazine-2-carboxylic acid (2.56 mmol) in DCM (6.0 mL), $T_3P$ (3.84 mmol), 6-chloro-3,4-dihydroisoquinoline-2(1*H*)-amine (2.81 mmol) and TEA (5.11 mmol) were added at 0 °C. The reaction mixture was kept at room temperature for 12 hours. After total consumption of the starting reagent (monitored by CCD), the mixture was diluted in water (10 mL) and extracted with DCM (10 mL x 2). The organic phase was concentrated under reduced pressure and the residue was purified by prep-HPLC ([$H_2O$ ($NH_4HCO_3$)-ACN] ; B%: 35%-70%). 260 mg (25.4% yield) of the product of interest were obtained in the form of a white solid.

Compounds 6, 11-13, 45 and 47, as shown in Table 11, are obtained by the procedure described for Example 1, changing the corresponding intermediates II and III.

**EXAMPLE 5. N'-(3,5-DIMETHOXYBENZYL) -6- (4-ETHOXYPHENYL)-N'-METHYLPYRAZINE-2-CARBOHYDRAZIDE**

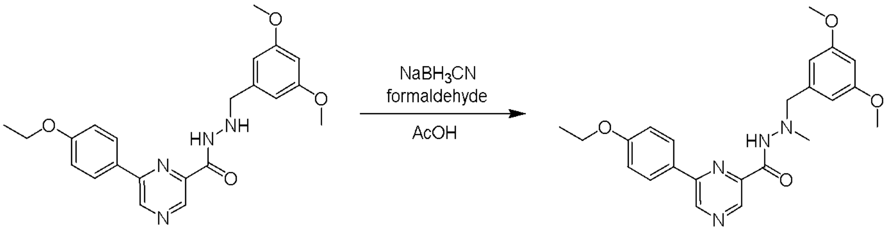


In a round-bottomed flask containing *N'*-(3,5-dimethoxybenzyl)-6-(4-ethoxyphenyl)pyrazine-2-carbohydrazide (1.05 mmol) in DCM (15.0 mL), $NaBH(OAc)_3$ (3.16 mmol) and formaldehyde (10.5 mmol) were added. The reaction mixture was kept at room temperature for 12 hours. After total consumption of the starting reagent (monitored by CCD), the mixture was diluted in water (20 mL) and extracted with DCM (5 mL x 3). The organic phase was concentrated under reduced pressure and the residue was purified by chromatography column ([$H_2O(NH_4HCO_3)$-ACN] ; B%: 37%-67%) . 278 mg (62.5% yield) of the product of interest were obtained in the form of a white solid.

## TABLE 11. COMPOUNDS OBTAINED ACCORDING TO SCHEDULE 1

| Compound Nº | Structure | $^1$H-NMR / MS (m/z) [M+H]$^+$ |
|---|---|---|
| 1 | 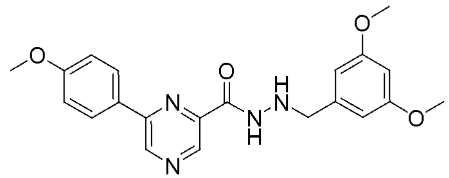 *N'*-(3,5-dimethoxybenzyl)-6-(4-methoxyphenyl)pyrazine-2-carbohydrazide | $^1$H-NMR, 400 MHz, DMSO-d$_6$ $\delta$ = 10.43 (br s, 1H), 9.39 (s, 1H), 8.97 (s, 1H), 8.38 - 8.32 (m, 2H), 7.11 - 7.06 (m, 2H), 6.61 (d, *J* = 2.2 Hz, 2H), 6.38 (t, *J* = 2.3 Hz, 1H), 5.58 (br s, 1H), 4.01 (br s, 2H), 3.85 (s, 3H), 3.72 (s, 6H). |
| 2 | 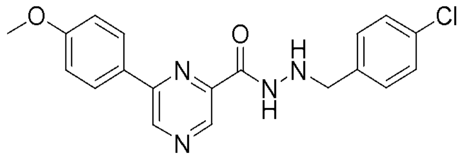 *N'*-(4-chlorobenzyl)-6-(4-methoxyphenyl)pyrazine-2-carbohydrazide | $^1$H-NMR, 400 MHz, DMSO-d$_6$ $\delta$ = 3.87 (s, 3 H) 4.08 (s, 2 H) 7.08 - 7.15 (m, 2 h) 7.37 - 7.43 (m, 2 h) 7.44 - 7.50 (m, 2 h) 8.30 - 8.40 (m, 2 h) 8.97 (s, 1 h) 9.40 (s, 1 h) 10.46 (s, 1 h). [M+H]$^+$: 369.0. |
| 3 | 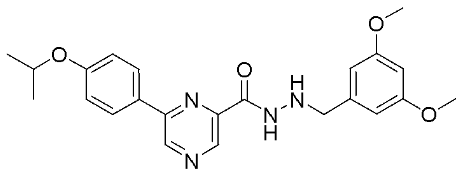 *N'*-(3,5-dimethoxybenzyl)-6-(4-isopropoxyphenyl)pyrazine-2-carbohydrazide | $^1$H-NMR, 400 MHz, DMSO-d$_6$ $\delta$ = 10.46 - 10.36 (m, 1H), 9.42 - 9.31 (m, 1H), 8.97 (s, 1H), 8.39 - 8.24 (m, 2H), 7.13 - 7.01 (m, 2H), 6.61 (d, *J* = 2.3 Hz, 2H), 6.42 - 6.32 (m, 1H), 5.57 (q, *J* = 6.1 Hz, 1H), 4.84 - 4.68 (m, 1H), 4.01 (d, *J* = 5.9 Hz, 2H), 3.72 (s, 6H), 1.31 (d, *J* = 6.0 Hz, 6H) . [M+H]$^+$: 423.4. |
| 4 | 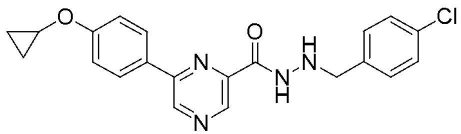 *N'*-(4-chlorobenzyl)-6-(4-cyclopropoxyphenyl)pyrazine-2-carbohydrazide | $^1$H-NMR, 400 MHz, DMSO-d$_6$ $\delta$ = 10,42 (d, *J* = 6.4 Hz, 1H), 9.39 (s, 1H), 8.97 (s, 1H), 8.39 - 8.31 (m, 2H), 7.48 - 7.44 (m, 2H), 7.42 - 7.36 (m, 2H), 7.25 - 7.18 (m, 2H), 5.65 (q, *J* = 6.0 Hz, 1H), 4.07 (d, *J* = 6.0 Hz, 2H), 3.97 (tt, *J* = 2.9, 6.0 Hz, 1H), 0.90 - 0.80 (m, 2H), 0.74 - 0.68 (m, 2H). [M+H]$^+$: 395.0. |
| 5 | 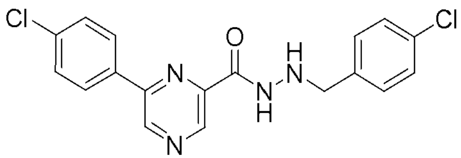 *N'*-(4-chlorobenzyl)-6-(4-chlorophenyl)pyrazine-2-carbohydrazide | $^1$H-NMR, 400 MHz, DMSO-d$_6$ $\delta$ = 10.54 (d, *J* = 6.5 Hz, 1H), 9.48 (s, 1H), 9.07 (s, 1H), 8.45 (d, *J* = 8.7 Hz, 2H), 7.63 (d, *J* = 8.6 Hz, 2H), 7.48 - 7.42 (m, 2H), 7.41 - 7.37 (m, 2H), 5.67 (d, *J* = 6.2 Hz, 1H), 4.07 (d, *J* = 6.0 Hz, 2H). [M+H]$^+$: 373.0. |

(continued)

| Compound № | Structure | $^1$H-NMR / MS (m/z) [M+H]$^+$ |
|---|---|---|
| 6 | 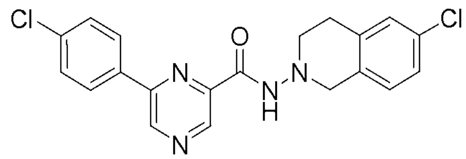<br>*N*-(6-chloro-3,4-dihydroisoquinoline-2(1H)-yl)-6-(4-chlorophenyl) pyrazine-2-carboxamide | $^1$H-NMR, 400 MHz, DMSO-$d_6$<br>$\delta$ = 10.24 (s, 1H), 9.51 (s, 1H), 9.11 (s, 1H), 8.45 (d, *J* = 8.6 Hz, 2H), 7.65 (d, *J* = 8.6 Hz, 2H), 7.33 - 7.11 (m, 3H), 4.15 (s, 2H), 3.26 (br t, *J* = 6.0 Hz, 3H), 2.99 (br t, *J* = 5.7 Hz, 2H).<br>[M+H]$^+$: 399.0. |
| 7 | 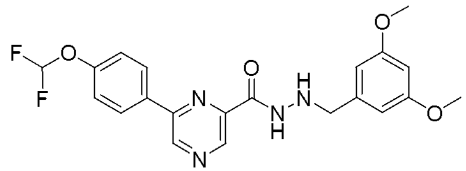<br>6-(4-(difluoromethoxy)phenyl)-*N'*-(3,5-dimethoxybenzyl)pyrazine-2-carbohydrazide | $^1$H-NMR, 400 MHz, DMSO-$d_6$<br>$\delta$ = 10.50 (s, 1H), 9.46 (s, 1H), 9.06 (s, 1H), 8.47 (d, *J* = 8.9 Hz, 2H), 7.64 - 7.16 (m, 3H), 6.60 (d, *J* = 2.0 Hz, 2H), 6.38 (t, *J* = 2.1 Hz, 1H), 5.77 - 5.44 (m, 1H), 4.01 (s, 2H), 3.71 (s, 6H).<br>[M+H]$^+$: 431.4. |
| 8 | 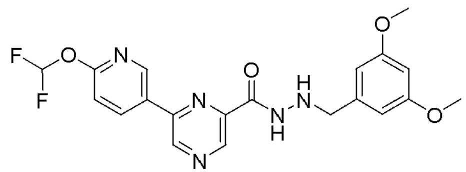<br>6-(6-(difluoromethoxy)pyridine-3-yl)-*N'*-(3,5-dimethoxybenzyl)pyrazine-2-carbohydrazide | $^1$H-NMR, 400 MHz, DMSO-$d_6$<br>$\delta$ = 10.60 (d, *J* = 6.5 Hz, 1H), 9.54 (s, 1H), 9.30 (d, *J* = 2.4 Hz, 1H), 9.11 (s, 1H), 8.95 (dd, *J* = 2.3, 8.7 Hz, 1H), 7.83 (t, *J* = 72.5 Hz, 1H), 7.30 (d, *J* = 8.8 Hz, 1H), 6.68 - 6.31 (m, 3H), 5.61 (q, *J*= 6.0 Hz, 1H), 4.02 (d, *J* = 5.9 Hz, 2H), 3.72 (s, 6H).<br>[M+H]$^+$: 431.1. |
| 9 | 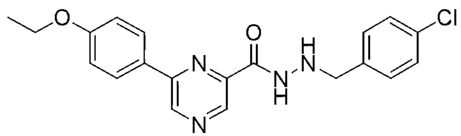<br>*N'*-(4-chlorobenzyl)-6-(4-ethoxyphenyl)pyrazine-2-carbohydrazide | $^1$H-NMR, 400 MHz, DMSO-$d_6$<br>$\delta$ = 10.43 (s, 1H), 9.39 (s, 1H), 8.96 (s, 1H), 8.42 - 8.28 (m, 2H), 7.49 - 7.43 (m, 2H), 7.42 - 7.36 (m, 2H), 7.12 - 7.04 (m, 2H), 4.14 (q, *J* = 7.0 Hz, 2H), 4.07 (s, 2H), 2.54 - 2.53 (m, 1H), 1.38 (t, *J* = 7.0 Hz, 3H).<br>[M+H]$^+$: 383.0. |

(continued)

| Compound Nº | Structure | $^1$H-NMR / MS (m/z) [M+H]$^+$ |
|---|---|---|
| 10 | 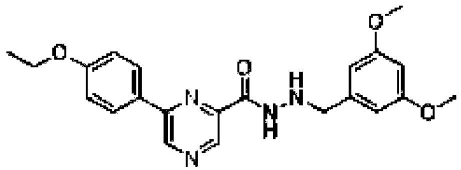 *N'*-(3,5-dimethoxybenzyl)-6-(4-ethoxyphenyl)pyrazine-2-carbohydrazide | $^1$H-NMR, 400 MHz, DMSO-$d_6$ $\delta$ = 10.49 (s, 1H), 9.39 (s, 1H), 8.97 (s, 1H), 8.33 (d, *J* = 8.8 Hz, 2H), 7.07 (d, *J* = 8.8 Hz, 2H), 6.61 (d, *J* = 2.2 Hz, 2H), 6.39 (t, *J* = 2.2 Hz, 1H), 4.13 (q, *J* = 7.1 Hz, 2H), 4.02 (s, 2H), 3.72 (s, 6H), 1.37 (t, *J* = 7.1 Hz, 3H). [M+H]$^+$: 409.0. |
| 11 | 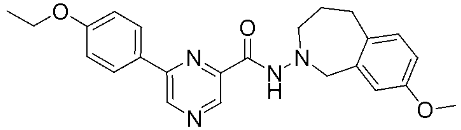 6-(4-ethoxyphenyl)-*N*-(8-methoxy-1,3,4,5-tetrahydro-2H-benzo[c]azepine-2-yl)pyrazine-2-carboxamide | $^1$H-NMR, 400 MHz, $CDCl_3$ $\delta$ = 9.19 (s, 1H), 9.08 (s, 1H), 8.77 (s, 1H), 7.64 - 7,. 57 (m, 2H), 7.21 (d, *J* = 8.3 Hz, 1H), 7.00 - 6.96 (m, 2H), 6.91 (dd, *J* = 2.7, 8.2 Hz, 1H), 6.81 (d, *J* = 2.7 Hz, 1H), 4.35 (br s, 2H), 4.14 (q, *J* = 7.0 Hz, 2H), 3.72 (s, 3H), 3.58 - 3.50 (m, 2H), 3.01 - 2.93 (m, 2H), 1.83 - 1.77 (m, 2H), 1.49 (t, *J* = 7.0 Hz, 3H). [M+H]$^+$: 419.2. |
| 12 | 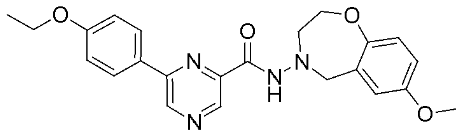 6-(4-ethoxyphenyl)-*N*-(7-methoxy-2,3-dihydrobenzo[f][1,4]oxazepine-4 (5H)-yl)pyrazine-2-carboxamide | $^1$H-NMR, 400 MHz, $CDCl_3$ $\delta$ = 9.12 (s, 1H), 9.03 (s, 1H), 8.91 (s, 1H), 7.63 - 7.56 (m, 2H), 7.02 (d, *J* = 8.7 Hz, 1H), 6.93 - 6.82 (m, 3H), 6.74 (d, *J* = 3.1 Hz, 1H), 4.28 (s, 2H), 4.10 - 4.01 (m, 4H), 3.68 (s, 3H), 3.53 - 3.45 (m, 2H), 1.41 (t, *J* = 7.0 Hz, 3H). [M+H]$^+$: 421.2. |
| 13 | 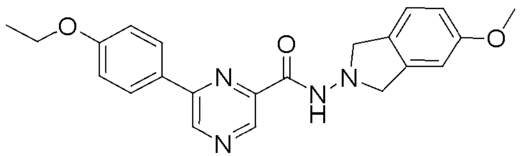 6-(4-ethoxyphenyl)-*N*-(5-methoxyisoindoline-2-yl)pyrazine-2-carboxamide | $^1$H-NMR, 400 MHz, DMSO-$d_6$ $\delta$ = 1.37 (t, *J* = 6.8 Hz, 3 H) 3.76 (s, 3 H) 4.14 (q, *J* = 6.8 Hz, 2 H) 4.37 (s, 2 H), 4.20 (s, 2 H) 6.83 - 6.89 (m, 2 H) 7.08 - 7.20 (m, 3 H) 4.37 (s, 2 h) 4.20 (s, 2 h) 8.37 - 8.40 (m, 2 h) 9.01 (s, 1 H) 9.42 (s, 1 H) 10.35 (s, 1 H). [M+H]$^+$: 391.2. |
| 14 | 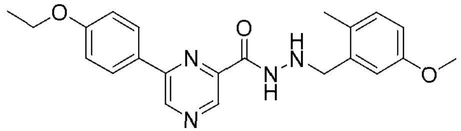 6-(4-ethoxyphenyl)-*N'*-(5-methoxy-2-methylbenzyl)pyrazine-2-carbohydrazide | $^1$H-NMR, 400 MHz, DMSO-$d_6$ $\delta$ = 1.37 (t, *J* = 6.94 Hz, 3 H) 2.30 (s, 3 H) 3.71 (s, 3 H) 3.91 - 4.20 (m, 4 H) 5.46 (s, 1 H) 6.74 (dd, *J* = 8.38, 2.75 Hz, 1 H) 6.86 - 7.20 (m, 4 H) 8.36 (d, *J* = 8.88 Hz, 2 H) 8.99 (s, 1 H) 9.39 (s, 1 H) 10.50 (d, *J* = 6.38 Hz, 1 H). [M+H]$^+$: 393.4. |

(continued)

| Compound Nº | Structure | $^1$H-NMR / MS (m/z) [M+H]$^+$ |
|---|---|---|
| 15 | 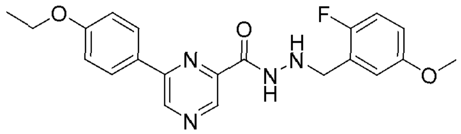 6-(4-ethoxyphenyl)-*N'*-(2-fluoro-5-methoxybenzyl)pyrazine-2-carbohydrazide | $^1$H-NMR, 400 MHz, DMSO-$d_6$ $\delta$ = 2.18 - 2.33 (m, 3 H) 3.53 (s, 3 H) 7.05 - 7.73 (m, 7 H) 8.08 - 8.17 (m, 1 H) 8.98 (s, 1 H) 11.10 - 11.19 (m, 1 H). [M+H]$^+$: 397.4. |
| 16 | 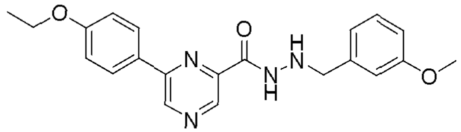 6-(4-ethoxyphenyl)-*N'*-(3-methoxybenzyl)pyrazine-2-carbohydrazide | $^1$H-NMR, 400 MHz, DMSO-$d_6$ $\delta$ = 1.37 (t, *J* = 6.94 Hz, 3 H) 3.74 (s, 3 H) 3.96 - 4.21 (m, 4 H) 5.55 (q, *J* = 6.21 Hz, 1 H) 7.35 (d, *J* = 12.13 Hz, 6 H) 8.33 (d, J = 8.88 Hz, 2 H) 8.84 (d, *J* = 4.50 Hz, 1 H) 9.38 (s, 1 H) 10.34 - 10.61 (m, 1 H). [M+H]$^+$: 379.4. |
| 17 | 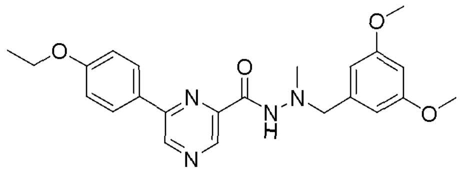 *N'*-(3,5-dimethoxybenzyl)-6-(4-ethoxyphenyl)-*N'*-methylpyrazine-2-carbohydrazide | $^1$H-NMR, 400 MHz, $CDCl_3$ $\delta$ = 9.23 (s, 1H), 9.10 (s, 1H), 8.68 (s, 1H), 7.98 - 7.83 (m, 2H), 7.04 (d, *J* = 8.8 Hz, 2H), 6.58 (d, *J* = 2.3 Hz, 2H), 6.42 (t, *J* = 2.2 Hz, 1H), 4.22 - 4.02 (m, 4H), 3.76 (s, 6H), 2.82 (s, 3H), 1.48 (t, *J* = 7.0 Hz, 3H). [M+H]$^+$: 423.5. |
| 18 | 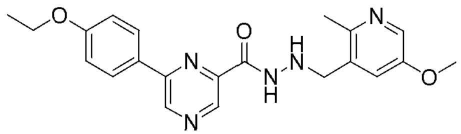 6-(4-ethoxyphenyl)-*N'*-((5-methoxy-2-methylpyridine-3-yl)methyl) pyrazine-2-carbohydrazide | $^1$H-NMR, 400 MHz, DMSO-$d_6$ $\delta$ = 1.30 (t, *J* = 6.94 Hz, 3 H) 2.50 (s, 3 H) 3.79 (s, 3 H) 4.04 (q, *J* = 6.84 Hz, 2 H) 4.20 (d, *J* =1.38 Hz, 2 H) 6.94 (d, *J* = 8.88 Hz, 2 H) 7.22 - 7.32 (m, 1 H) 7.47 (br d, *J* = 5.38 Hz, 1 H) 7.59 (d, *J* = 3.13 Hz, 1 H) 7.92 (d, *J* = 8.88 Hz, 2 H) 8.12 (d, *J* = 2.88 Hz, 1 H) 8.68 (s, 1 H) 10.85 (s, 1 H). [M+H]$^+$: 394.1. |
| 19 | 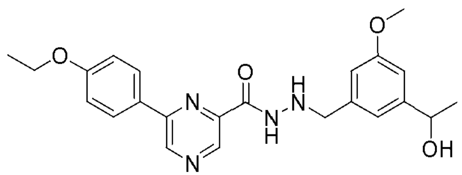 6-(4-ethoxyphenyl)-*N'*-(3-(1-hydroxyethyl)-5-methoxybenzyl)pyrazine-2-carbohydrazide | |

(continued)

| Compound № | Structure | $^1$H-NMR / MS (m/z) $[M+H]^+$ |
|---|---|---|
| 20 | 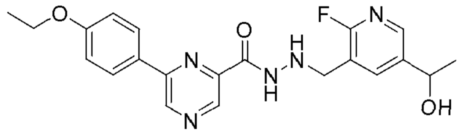 6-(4-ethoxyphenyl)-*N'*-((2-fluoro-5-(1-hydroxyethyl)pyridine-3-yl)methyl)pyrazine-2-carbohydrazide | |
| 21 | 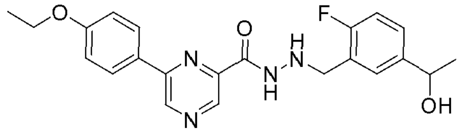 6-(4-ethoxyphenyl)-*N'*-(2-fluoro-5-(1-hydroxyethyl)benzyl)pyrazine-2-carbohydrazide | |
| 22 | 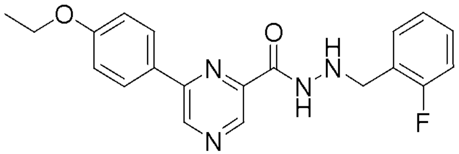 6-(4-ethoxyphenyl)-*N'*-(2-fluorobenzyl)pyrazine-2-carbohydrazide | $^1$H-NMR, 400 MHz, DMSO-$d_6$ $\delta$ = 1.37 (t, *J* = 6.94 Hz, 3 H) 4.05 - 4.17 (m, 4 H) 5.49 - 5.66 (m, 1 H) 7.02 - 7.21 (m, 4 H) 7.27 - 7.38 (m, 1 H) 7.47 - 7.64 (m, 1 H) 8.35 (d, *J* = 8.75 Hz, 2 H) 8.96 (s, 1 H) 9.38 (s, 1 H) 10.51 (d, *J* = 6.50 Hz, 1 H). $[M+H]^+$: 367.4. |
| 23 | 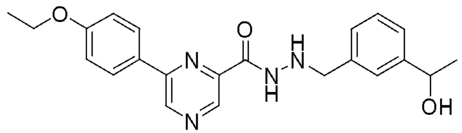 6-(4-ethoxyphenyl)-*N'*-(3-(1-hydroxyethyl)benzyl)pyrazine-2-carbohydrazide. | |
| 24 | 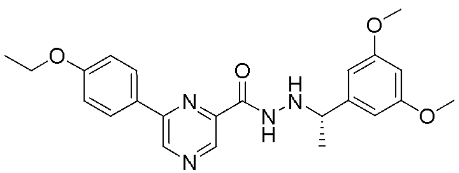 (*S**)-*N'*-(1-(3,5-dimethoxyphenyl)ethyl)-6-(4-ethoxyphenyl)pyrazine-2-carbohydrazide[1] | $^1$H-NMR, 400 MHz, $CDCl_3$ $\delta$ = 9.19 (s, 1H), 9.11 (s, 1H), 9.01 (s, 1H), 7.94 - 7.78 (m, 2H), 7.08 - 6.94 (m, 2H), 6.67 - 6.58 (m, 2H), 6.42 (t, *J* = 2.3 Hz, 1H), 5.33 - 4.77 (m, 1H), 4.23 (q, *J* = 6.6 Hz, 1H), 4.12 (q, *J* = 7.0 Hz, 2H), 3.80 (s, 6H), 1.52 - 1.43 (m, 6H). $[M+H]^+$: 423.5. |

(continued)

| Compound No | Structure | $^1$H-NMR / MS (m/z) $[M+H]^+$ |
|---|---|---|
| 25 | 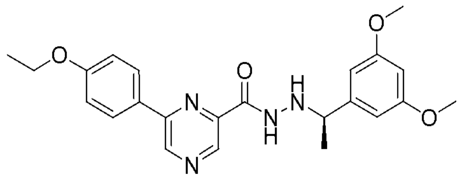 (*R**)-*N*'-(1-(3,5-dimethoxyphenyl)ethyl)-6-(4-ethoxyphenyl)pyrazine-2-carbohydrazide[1] | $^1$H-NMR, 400 MHz, $CDCl_3$ $\delta$ = 9.21 - 9.07 (m, 2H), 7.91 - 7.79 (m, 2H), 7.01 (d, $J$ = 8.9 Hz, 2H), 6.62 (d, $J$ = 2.3 Hz, 2H), 6.42 (t, $J$ = 2.3 Hz, 1H), 4.24 (q, $J$ = 6.5 Hz, 1H), 4.12 (q, $J$ = 6.9 Hz, 2H), 3.86 - 3.68 (m, 6H), 1.52 - 1.42 (m, 6H). $[M+H]^+$: 423.5. |
| 26 | 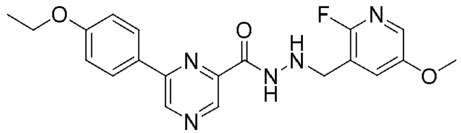 6-(4-ethoxyphenyl)-*N'*-((2-fluoro-5-methoxypyridine-3-yl)methyl)pyrazine-2-carbohydrazide | $^1$H-NMR, 400 MHz, DMSO-$d_6$ $\delta$ = 1.37 (t, $J$ = 6.94 Hz, 3 H) 3.80 (s, 3 H) 4.06 - 4.17 (m, 4 H) 5.83 (q, $J$ = 5.63 Hz, 1 H) 7.07 (d, $J$ = 8.76 Hz, 2 H) 7.74 - 7.78 (m, 2 H) 8.34 (d, $J$ = 8.76 Hz, 2 H) 8.95 (s, 1 H) 9.38 (s, 1 H) 10.48 (d, $J$ = 6.00 Hz, 1 H). $[M+H]^+$: 398.1. |
| 27 | 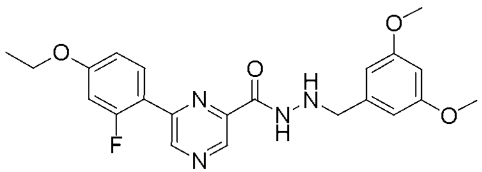 *N'*-(3,5-dimethoxybenzyl)-6-(4-ethoxy-2-fluorophenyl)pyrazine-2-carbohydrazide | $^1$H-NMR, 400 MHz, DMSO-$d_6$ $\delta$ = 10.38 (d, $J$ = 6,4 Hz, 1H), 9.15 (d, $J$ = 2.0 Hz, 1H), 9.02 (s, 1H), 8.42 - 8.22 (m, 1H), 7.09 - 6.90 (m, 2H), 6.60 (d, $J$ = 2.1 Hz, 2H), 6.38 (s, 1H), 5.58 (q, $J$ = 6.0 Hz, 1H), 4.15 (q, $J$ = 6.9 Hz, 2H), 4.03 - 3.97 (m, 2H), 3.71 (s, 6H), 1.37 (t, $J$ = 6.9 Hz, 3H). $[M+H]^+$: 427.4. |
| 28 | 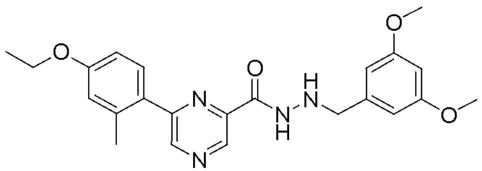 *N'*-(3,5-dimethoxybenzyl)-6-(4-ethoxy-2-methylphenyl)pyrazine-2-carbohydrazide | $^1$H-NMR, 400 MHz, DMSO-$d_6$ $\delta$ = 10,07 (d, $J$ = 6.4 Hz, 1H), 9.08 - 8.89 (m, 2H), 7.54 (d, $J$ = 8.4 Hz, 1H), 6.99 - 6.83 (m, 2H), 6.57 (d, $J$ = 2.1 Hz, 2H), 6.36 (t, $J$ = 2.1 Hz, 1H), 5.60 (q, $J$ = 5.8 Hz, 1H), 4.09 (q, $J$ = 6.9 Hz, 2H), 3.96 (d, $J$ = 5.6 Hz, 2H), 3.70 (s, 6H), 2.34 (s, 3H), 1.35 (t, $J$ = 6.9 Hz, 3H). $[M+H]^+$: 423.4. |

(continued)

| Compound No | Structure | $^1$H-NMR / MS (m/z) [M+H]$^+$ |
|---|---|---|
| 29 | 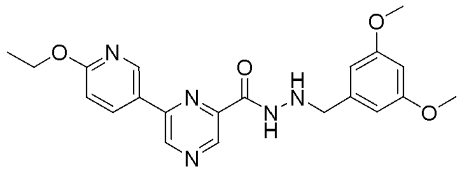 *N'*-(3,5-dimethoxybenzyl)-6-(6-ethoxypyridine-3-yl)pyrazine-2-carbohydrazide | $^1$H-NMR, 400 MHz, DMSO-$d_6$ $\delta$ = 10.52 (d, *J* = 6.4 Hz, 1H), 9.45 (s, 1H), 9.19 (d, *J* = 2.1 Hz, 1H), 9.03 (s, 1H), 8.72 (dd, *J* = 2.5, 8.8 Hz, 1H), 6.96 (d, *J* = 8.8 Hz, 1H), 6.60 (d, *J* = 2.3 Hz, 2H), 6.37 (t, *J* = 2.3 Hz, 1H), 5.58 (q, *J* = 6.1 Hz, 1H), 4.40 (q, *J* = 7.0 Hz, 2H), 4.10 - 3.94 (m, 2H), 3.71 (s, 6H), 1.36 (t, *J* = 7.1 Hz, 3H). [M+H]$^+$: 409.1. |
| 30 | 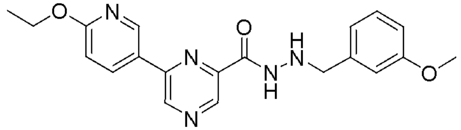 6-(6-ethoxypyridine-3-yl)-*N'*-(3-methoxybenzyl)pyrazine-2-carbohydrazide | $^1$H-NMR, 400 MHz, DMSO-$d_6$ $\delta$ = 1,36 (t, *J* = 7.00 Hz, 3 H) 3.73 (s, 3 H) 4.04 (br d, *J* = 5.75 Hz, 2 H) 4.40 (q, *J* = 7.05 Hz, 2 H) 5.57 (q, *J* = 6.00 Hz, 1 H) 6.82 (dd, *J* = 8.19, 2.19 Hz, 1 H) 6.93 - 7.04 (m, 3 H) 7.21 - 7.26 (m, 1 H) 8.72 (dd, *J* = 8.76, 2.50 Hz, 1 H) 9.03 (s, 1 H) 9.20 (d, *J* = 2.25 Hz, 1 H) 9.45 (s, 1 H) 10.55 (br d, *J* = 6.50 Hz, 1 H). [M+H]$^+$: 380.4. |
| 31 | 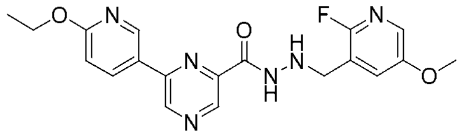 6-(6-ethoxypyridine-3-yl)-*N'*-((2-fluoro-5-methoxypyridine-3-yl)methyl)pyrazine-2-carbohydrazide | $^1$H-NMR, 400 MHz, DMSO-$d_6$ $\delta$ = 1.33 - 1.38 (m, 3 H) 3,3 80 (d, *J* = 1.50 Hz, 3 H) 4.09 (br d, *J* = 4.75 Hz, 2 H) 4.37 - 4.43 (m, 2 H) 5.79 - 5.85 (m, 1 H) 6.93 - 6.99 (m, 1 H) 7.71 - 7.79 (m, 2 H) 8.68 - 8.74 (m, 1 H) 9.01 (d, *J* = 1.25 Hz, 1 H) 9.15 - 9.22 (m, 1 H) 9.42 - 9.48 (m, 1 H) 10.53 (br d, *J* = 6.38 Hz, 1 H) . [M+H]$^+$: 399.4. |
| 32 | 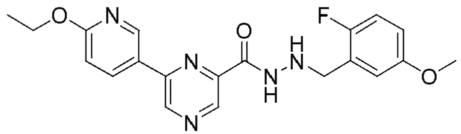 6-(6-ethoxypyridine-3-yl)-*N'*-(2-fluoro-5-methoxybenzyl)pyrazine-2-carbohydrazide | $^1$H-NMR, 400 MHz, DMSO-$d_6$ $\delta$ = 1.33 - 1.40 (m, 3 H) 3.71 (br s, 3 h) 4.09 (br s, 2 h) 4.37 - 4.44 (m, 2 h) 5.65 (br d, *J* = 1.38 Hz, 1 h) 6.79 - 6.86 (m, 1 h) 6.93 - 6.99 (m, 1 h) 7.04 - 7.16 (m, 2 h) 8.68 - 8.75 (m, 1 h) 9.00 - 9.05 (m, 1 H) 9.19 (br s, 1 H) 9.42 - 9.48 (m, 1 H) 10.52 - 10.59 (m, 1 H). [M+H]$^+$: 398.4. |
| 33 | 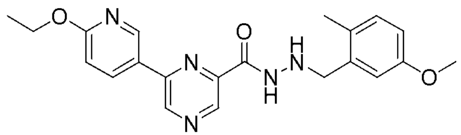 6-(6-ethoxypyridine-3-yl)-*N'*-(5-methoxy-2-methylbenzyl)pyrazine-2-carbohydrazide | $^1$H-NMR, 400 MHz, DMSO-$d_6$ $\delta$ = 1.36 (t, *J* = 7.07 Hz, 3 H) 2.29 (s, 3 H) 3.71 (s, 3 H) 4.01 (d, *J* = 6.25 Hz, 2 H) 4.41 (q, *J* = 7.09 Hz, 2 H) 5.41 (q, *J* = 6.38 Hz, 1 H) 6.74 (dd, *J* = 8.25, 2.75 Hz, 1 H) 6.94 - 7.03 (m, 2 H) 7.07 (d, *J* = 8.38 Hz, 1 H) 8.73 (dd, *J* = 8.76, 2.50 Hz, 1 H) 9.05 (s, 1 H) 9.21 (d, *J* = 2.13 Hz, 1 H) 9.46 (s, 1 H) 10.58 (d, *J* = 6.50 Hz, 1 H). [M+H]$^+$: 394.4. |

(continued)

| Compound № | Structure | $^1$H-NMR / MS (m/z) [M+H]$^+$ |
|---|---|---|
| 34 | 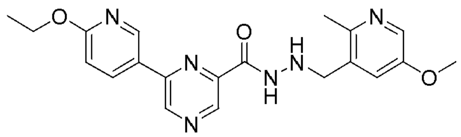 6-(6-ethoxypyridine-3-yl)-*N'*-((5-methoxy-2-methylpyridine-3-yl) methyl)pyrazine-2-carbohydrazide | $^1$H-NMR, 400 MHz, DMSO-$d_6$ $\delta$ = 1.31 - 1.37 (m, 3 H) 2.56 (s, 3 H) 3.85 (s, 3 H) 4.30 (d, *J* = 1.50 Hz, 2 H) 4.37 (q, *J* = 7.05 Hz, 2 H) 6.89 (d, *J* = 8.75 Hz, 1 H) 7.34 (d, *J* = 6.13 Hz, 1 H) 7.58 - 7.70 (m, 2 H) 8.18 (d, *J* = 3.00 Hz, 1 H) 8.49 (dd, *J* = 8.76, 2.50 Hz, 1 H) 8.66 - 8.75 (m, 2 H) 10.93 (s, 1 H). [M+H]$^+$: 395.4. |
| 35 | 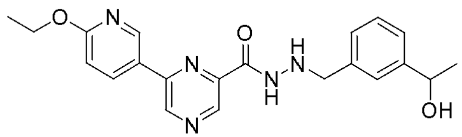 6-(6-ethoxypyridine-3-yl)-*N'*-(3-(1-hydroxyethyl)benzyl)pyrazine-2-carbohydrazide | |
| 36 | 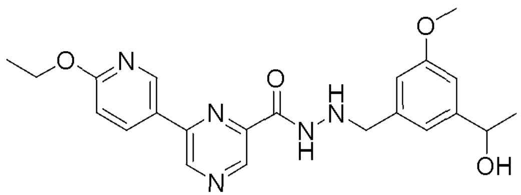 6-(6-ethoxypyridine-3-yl)-*N'*-(3-(1-hydroxyethyl)-5-methoxybenzyl)pyrazine-2-carbohydrazide | |
| 37 | 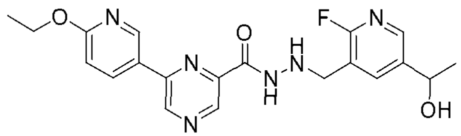 6-(6-ethoxypyridine-3-yl)-*N'*-((2-fluoro-5-(1-hydroxyethyl)pyridine-3-yl)methyl)pyrazine-2-carbohydrazide | |
| 38 | 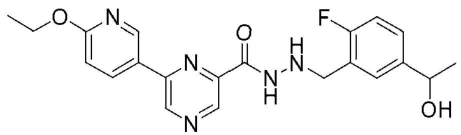 6-(6-ethoxypyridine-3-yl)-*N'*-(2-fluoro-5-(1-hydroxyethyl)benzyl)pyrazine-2-carbohydrazide | |

(continued)

| Compound № | Structure | $^1$H-NMR / MS (m/z) [M+H]$^+$ |
|---|---|---|
| 39 | 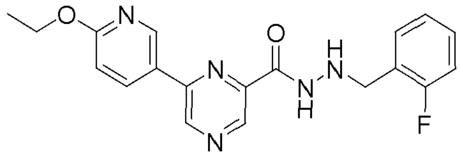 6-(6-ethoxypyridine-3-yl)-*N'*-(2-fluorobenzyl)pyrazine-2-carbohydrazide | $^1$H-NMR, 400 MHz, DMSO-$d_6$ $\delta$ = 1.37 (q, *J* = 7.09 Hz, 3 H) 4.12 (br s, 2 h) 4.37 - 4.46 (m, 2 h) 5.57 - 5.67 (m, 1 h) 6.93 - 7.01 (m, 1 h) 7.12 - 7.22 (m, 2 h) 7.27 - 7.37 (m, 1 h) 7.52 - 7.60 (m, 1 h) 8.69 - 8.77 (m, 1 h) 8.99 - 9.05 (m, 1 h) 9.21 (br d, *J* = 4.88 Hz, 1 H) 9.46 (br d, *J* = 7.88 Hz, 1 H) 10.59 (br t, *J* = 6.82 Hz, 1 H). [M+H]$^+$: 368.3. |
| 40 | 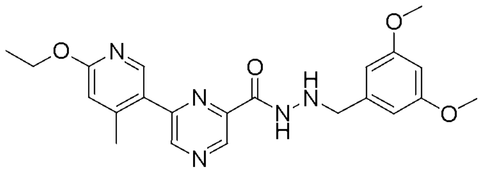 *N'*-(3,5-dimethoxybenzyl)-6-(6-ethoxy-4-methylpyridine-3-yl)pyrazine-2-carbohydrazide | $^1$H-NMR, 400 MHz, DMSO-$d_6$ $\delta$ = 9.33 (s, 1H), 8.89 (s, 1H), 8.22 (s, 1H), 6.71 (s, 1H), 6.57 (d, *J* = 1.8 Hz, 2H), 6.44 - 6.37 (m, 1H), 4.44 (q, *J* = 7.0 Hz, 2H), 4.08 (s, 2H), 3.79 (s, 6H), 2.32 (s, 3H), 1.44 (t, *J* = 7.1 Hz, 3H). [M+H]$^+$: 423.4. |
| 41 | 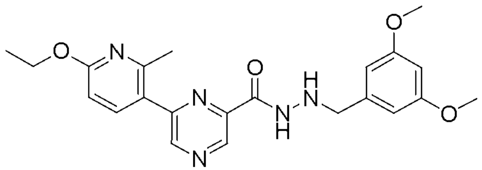 *N'*-(3,5-dimethoxybenzyl)-6-(6-ethoxy-2-methylpyridine-3-yl)pyrazine-2-carbohydrazide | $^1$H-NMR, 400 MHz, DMSO-$d_6$ $\delta$ = 10.15 (d, *J* = 6.5 Hz, 1H), 9.04 (d, *J* = 9.5 Hz, 2H), 8.00 (d, *J* = 8.5 Hz, 1H), 6.86 - 6.74 (m, 1H), 6.57 (d, *J* = 2.3 Hz, 2H), 6.36 (t, *J* = 2.3 Hz, 1H), 5.60 (q, *J* = 5.9 Hz, 1H), 4.37 (q, *J* = 7.0 Hz, 2H), 3.97 (d, *J* = 5.8 Hz, 2H), 3.70 (s, 6H), 2.49 (br s, 3H), 1.34 (t, *J* = 7.1 Hz, 3H). [M+H]$^+$: 423.4. |
| 42 | 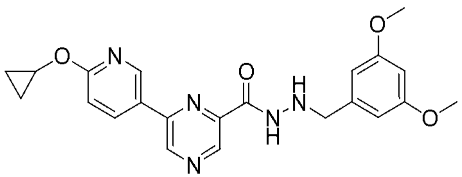 6-(6-cyclopropoxypyridine-3-yl)-*N'*-(3,5-dimethoxybenzyl)pyrazine-2-carbohydrazide | $^1$H-NMR, 400 MHz, DMSO-$d_6$ $\delta$ = 10.52 (br d, *J* = 6.1 Hz, 1H), 9.46 (s, 1H), 9.23 (s, 1H), 9.04 (s, 1H), 8.73 (br dd, *J* = 1.7, 8.6 Hz, 1H), 7.02 (br d, *J* = 8.6 Hz, 1H), 6.60 (s, 2H), 6.37 (br s, 1H), 5.66 - 5.49 (m, 1H), 4.31 (br d, *J* = 2.9 Hz, 1H), 4.01 (br d, *J* = 5.5 Hz, 2H), 3.71 (s, 6H), 0.89 - 0.62 (m, 4H). [M+H]$^+$: 421.4. |

(continued)

| Compound № | Structure | $^1$H-NMR / MS (m/z) [M+H]$^+$ |
|---|---|---|
| 43 | 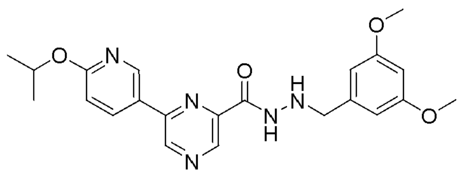 *N'*-(3,5-dimethoxybenzyl)-6-(6-isopropoxypyridine-3-yl)pyrazine-2-carbohydrazide | $^1$H-NMR, 400 MHz, DMSO-$d_6$ $\delta$ = 10.52 (d, *J* = 6.5 Hz, 1H), 9.44 (s, 1H), 9.19 (d, *J* = 2.4 Hz, 1H), 9.02 (s, 1H), 8.70 (dd, *J* = 2.4, 8.8 Hz, 1H), 6.90 (d, *J* = 8.6 Hz, 1H), 6.60 (d, *J* = 2.1 Hz, 2H), 6.37 (t, *J* = 2.1 Hz, 1H), 5.58 (q, *J* = 6.0 Hz, 1H), 5.35 (q, *J* = 6.2 Hz, 1H), 4.01 (d, *J* = 6.0 Hz, 2H), 3.71 (s, 6H), 1.34 (d, *J* = 6.3 Hz, 6H). [M+H]$^+$: 423.4. |
| 44 | 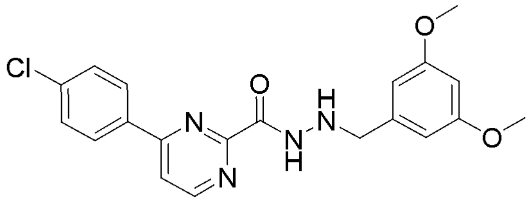 4-(4-chlorophenyl)-*N'*-(3,5-dimethoxybenzyl)pyrimidine-2-carbohydrazide | $^1$H-NMR, 400 MHz, DMSO-$d_6$ $\delta$ 10.44 (s, 1H), 8.99 (d, *J* = 5.4 Hz, 1H), 8.43 - 8.36 (m, 2H), 8.24 (d, *J* = 5.4 Hz, 1H), 7.69 - 7.62 (m, 2H), 6.61 (d, *J* = 2.3 Hz, 2H), 6.38 (t, *J* = 2.3 Hz, 1H), 4.01 (s, 2H), 3.72 (s, 6H). |
| 45 | 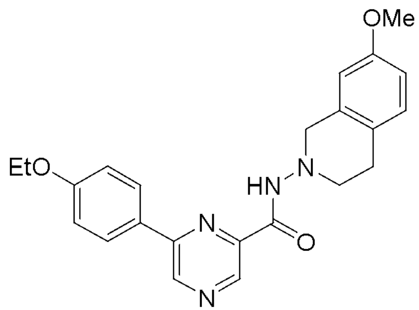 6-(4-ethoxyphenyl)-*N*-(7-methoxy-3,4-dihydroisoquinoline-2(1*H*)-yl) pyrazine-2-carboxamide | $^1$H-NMR, 400 MHz, DMSO-$d_6$ $\delta$ = 1.36 (t, *J* = 6.8 Hz, 3 H) 2.87 - 2.90 (m, 2 H), 3.21 - 3.24 (m, 2 H) 3.70 (s, 3 H) 4.09 - 4.15 (m, 4 H), 6.67 - 6.76 (m, 2 H), 7.01 - 7.09 (m, 3 H) 8.32 - 8.34 (m, 2 H), 8.99 (s, 1 h) 9.39 (s, 1 h) 10.06 (s, 1 H). [M+H]$^+$: 405.2. |
| 46 | 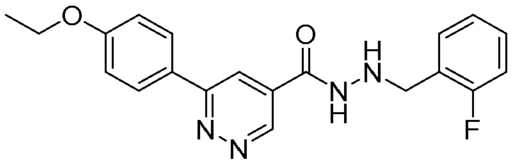 6-(4-ethoxyphenyl)-*N'*-(2-fluorobenzyl)pyridazine-4-carbohydrazide | $^1$H NMR, 400 MHz, DMSO-$d_6$ δ: 10,55 (br s, 1H), 9,35 (d, *J* = 2,0 Hz, 1H), 8,36 (d, *J* = 2,0 Hz, 1H), 8,16-8,12 (m, 2H), 7,53-7,49 (m,1H), 7,36-7,31 (m, 1H), 7,20-7,17 (m, 4H), 5,76 (br s, 1H), 4,14 (q, *J* = 6,8 Hz, 2H), 4,11-4,08 (m, 2H), 1,38 (t, *J* = 6,8 Hz, 3H). [M+H]$^+$: 367,21. |

(continued)

| Compound № | Structure | $^1$H-NMR / MS (m/z) $[M+H]^+$ |
|---|---|---|
| 47 | 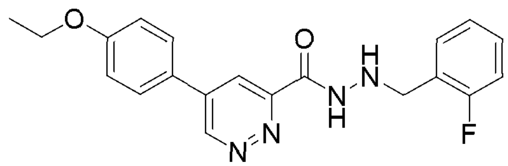 5-(4-ethoxyphenyl)-*N'*-(2-fluorobenzyl)pyridazine-3-carbohydrazide | $^1$H NMR, 400 MHz, DMSO-$d_6$ δ: 11,71(s, 1H), 10,70 (d, *J* = 6,0 Hz, 1H), 9,76 (d, *J* = 2,4 Hz, 1H), 8,27 (d, *J* = 2,4 Hz, 1H), 7,99-7,95 (m,2H), 7,59-7,,55 (m, 1H), 7,32-7,28 (m, 1H), 7,18-7,09 (m, 4H), 5,69 (dd, *J* = 6,0 Hz, 12,0 Hz, 1H), 4,15 (m, 4H), 1,36 (t, *J* = 6,8 Hz, 3H). $[M+H]^+$: 367,24. |
| 48 | 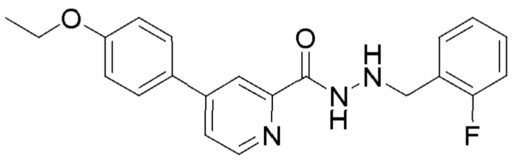 4-(4-ethoxyphenyl)-*N'*-(2-fluorobenzyl)picolinohydrazide | $^1$H NMR, 400 MHz, $CDCl_3$ δ 8.51 (d, *J* = 5.14 Hz, 1H), 8.40 (d, *J* = 1.35 Hz, 1H), 7.68 (d, *J* = 8.80 Hz, 2H), 7.63 (dd, *J* = 5.07, 1.77 Hz, 1H), 7.50 - 7.44 (m, 1H), 7.34 - 7.28 (m, 1H), 7.18 - 7.12 (m, 1H), 7.08 (t, *J* = 9.17 Hz, 1H), 7.02 (d, *J* = 8.80 Hz, 2H), 4.22 (s, 2H), 4.15 - 4.07 (m, 2H), 1.46 (t, *J* = 6.97 Hz, 3H). $[M+H]^+$: 366.1. |
| 49 | 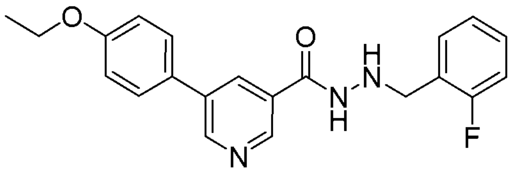 5-(4-ethoxyphenyl)-*N'*-(2-fluorobenzyl)nicotinohydrazide | $^1$H NMR, 400 MHz, DMSO-$d_6$ δ 10.29 (br d, *J* = 6.13 Hz, 1H), 8.97 (d, *J* = 2.13 Hz, 1H), 8.84 (d, *J* = 1.88 Hz, 1H), 8.32 (t, *J* = 2.06 Hz, 1H), 7.75 - 7.69 (m, 2H), 7.50 (td, *J* = 7.60, 1.81 Hz, 1H), 7.36 - 7.29 (m, 1H), 7.21 - 7.12 (m, 2H), 7.07 (d, *J* = 8.76 Hz, 2H), 5.64 (br d, *J* = 5.63 Hz, 1H), 4.13 - 4.03 (m, 4H), 1.35 (t, *J* = 6.94 Hz, 3H). $[M+H]^+$: 366.1. |
| 50 | 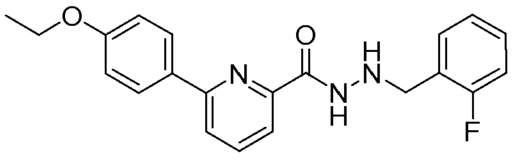 6-(4-ethoxyphenyl)-*N'*-(2-fluorobenzyl)picolinohydrazide | $^1$H NMR, 400 MHz, DMSO-$d_6$ δ 10.24 (d, *J* = 6.75 Hz, 1H), 8.24 (d, *J* = 8.76 Hz, 2H), 8.10 - 8.04 (m, 1H), 7.98 (t, *J* = 7.82 Hz, 1H), 7.84 (d, *J* = 7.38 Hz, 1H), 7.55 (td, *J* = 7.63, 1.63 Hz, 1H), 7.37 - 7.29 (m, 1H), 7.21 - 7.13 (m, 2H), 7.02 (d, *J* = 8.88 Hz, 2H), 5.54 (q, *J* = 6.21 Hz, 1H), 4.15 - 4.07 (m, 4H), 1.36 (t, *J* = 6.94 Hz, 3H). $[M+H]^+$: 366.1. |
| 51 | 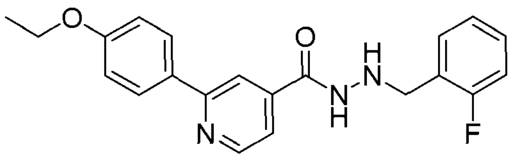 2-(4-ethoxyphenyl)-*N'*-(2-fluorobenzyl)isonicotinohydrazide | $^1$H NMR, 400 MHz, DMSO-$d_6$ δ 10.40 (d, *J* = 6.25 Hz, 1H), 8.72 (dd, *J* = 5.00, 0.63 Hz, 1H), 8.16 (s, 1H), 8.10 - 8.03 (m, 2H), 7.58 (dd, *J* = 5.00, 1.50 Hz, 1H), 7.50 (td, *J* = 7.60, 1.81 Hz, 1H), 7.37 - 7.29 (m, 1H), 7.22 - 7.14 (m, 2H), 7.09 - 7.03 (m, 2H), 5.67 (q, *J* = 5.50 Hz, 1H), 4.15 - 4.05 (m, 4H), 1.36 (t, *J* = 7.00 Hz, 3H). $[M+H]^+$: 366.1. |

(continued)

| Compound № | Structure | $^1$H-NMR / MS (m/z) [M+H]$^+$ |
|---|---|---|
| 52 | 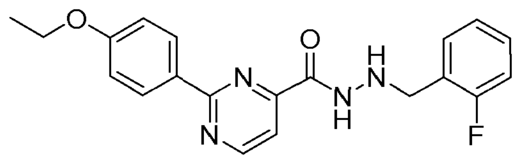 2-(4-ethoxyphenyl)-*N'*-(2-fluorobenzyl)pyrimidine-4-carbohydrazide | $^1$H NMR, 400 MHz, DMSO-$d_6$ δ 10.65 (br d, *J* = 6,. 38 Hz, 1H), 9.03 (d, *J* = 4.88 Hz, 1H), 8.59 - 8.53 (m, 2H), 7.75 (d, *J* = 4.88 Hz, 1H), 7.54 (td, *J* = 7.66, 1.81 Hz, 1H), 7.32 (tdd, *J* = 7.69, 7.69, 5.63, 1.75 Hz, 1H), 7.21 - 7.13 (m, 2H), 7.08 - 7.02 (m, 2H), 5.63 (q, *J* = 6.25 Hz, 1H), 4.17 - 4.09 (m, 4H), 1.37 (t, *J* = 7.00 Hz, 3H). [M+H]$^+$: 367.1. |
| 53 | 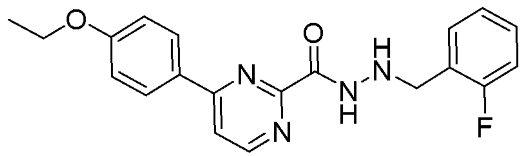 4-(4-ethoxyphenyl)-*N'*-(2-fluorobenzyl)pyrimidine-2-carbohydrazide | $^1$H NMR, 400 MHz, DMSO-$d_6$ δ 10.39 (d, *J* = 6,75 Hz, 1H), 8.86 (d, *J* = 5.38 Hz, 1H), 8.32 (d, *J* = 8.88 Hz, 2H), 8.11 (d, *J* =5.50 Hz, 1H), 7.55 (td, *J* = 7.63, 1.75 Hz, 1H), 7.32 (tdd, *J* = 7.71, 7.71, 5.60, 1.75 Hz, 1H), 7.21 - 7.13 (m, 2H), 7.09 (d, *J* = 9.01 Hz, 2H), 5.61 (q, *J* = 6.09 Hz, 1H), 4.18 - 4.07 (m, 4H), 1.37 (s, 3H). [M+H]$^+$: 367.1. |
| [1] Relative Stereochemistry | | |

**EXAMPLE 3. TESTS *IN VITRO:***

Biological assays were performed on Chinese hamster ovary (CHO) cells that express human sodium channels Nav 1.8 or Nav 1.7 in a stable manner.

The experimental protocol *voltage-clamp* using the whole cell configuration was established on the automated ScreenPatch® 384P platform (SP384PE, Nanion Technologies, Livingston, NJ) and the results were recorded with a Nanion 384-well Patch Clamp chip (NPC) (Nanion Technologies, Livingston, NJ).

Formula I compounds were diluted in eight concentrations in the extracellular solution composed of saline saline solution, buffered with HEPES (mM): NaCl, 137; KCl, 4; $CaCl_2$, 3,8; $MgCl_2$, 1; HEPES, 10; Glucose, 10; pH 7.4. The extracellular solution is composed of (mM) CsCl, 50; CsF, 90; $MgCl_2$, 5; EGTA, 5; HEPES, 10; pH 7.2. The duration of exposure of each compound with cells expressing Nav 1.7 or Nav 1.8 was at least five minutes, and the assays were performed at room temperature.

The measurements of the sodium currents of Nav 1.8 and Nav 1.7 were obtained using the voltage protocol described below.

A holding voltage of -100 mV was established followed by an inactivation voltage step at -40 mV for 8 seconds, followed by a step of -100 mV for 20 ms, followed by a 20 ms step for 10 mV for Nav 1.8 or 0 mV for Nav 1.7 (TP1A) before returning to the holding voltage of -100 mV.

The protocol was repeated at a frequency of 0.05 Hz and the amplitude of the current was quantified throughout the recording of the TP1A phase. The variation in the amplitude of the peak current was evaluated according to the formula described below, after exposing the cells expressing the channels to each concentration of the different compounds:

$$\% \text{ Block} = (1 - (I_{TP1A,molecule} / I_{TP1A,basal}) \times 100\%,$$

wherein $I_{TP1A,basal}$ and $I_{TP1A,molecule}$ represent the peaks of sodium current input into TP1A before exposure to the compound and in the presence of the compound, respectively.

The decrease in the amplitude of the peak current, after the exposure of the cells to the compounds, was used to calculate the percentage of relative blockage of the channels in relation to the positive control according to the formula below:

$$\% \text{ Block'} = 100\% - ((\% \text{ Block} - \% \text{ CP}) * (100\% / (\%V - \% \text{ CP})),$$

wherein %V and %CP represent the means of the values of current inhibition with a vehicle (DMSO) and positive controls, respectively. The sodium currents of the positive control were considered as 100%.

$$\% \text{ Block'} = (100\% / [1 + ([\text{Test}] / CI_{50})^N],$$

wherein [Test] represents the concentration of the molecule evaluated, $CI_{50}$ is the concentration of the compound that generates half of the maximum inhibition, N is the Hill coefficient, and % Block' is the percentage of sodium channel current (Nav 1.8 and Nav 1.7) blocked at each concentration of the molecule evaluated. Data were obtained by nonlinear regression (*nonlinear least squares*) with XLfit for Excel (Microsoft, Redmond, WA).

The compounds were tested in at least one assay to obtain the value of $CI_{50}$. For compounds that were tested in two or more assays, the results are described as the means of the $CI_{50}$ values.

Table 5 shows the efficacy *in vitro* of selected compounds against Nav1.8 and Nav1.7. $CI_{50}$ values less than 500 nM are represented with the legend (+++); $CI_{50}$ values between 500 nM and 1000 nM are represented with the legend (++), $CI_{50}$ values greater than 1000 nM are demonstrated with the symbology (+).

**TABLE 5. $CI_{50}$ VALUES OF THE COMPOUNDS OF THIS INVENTION IN NAV 1.8 AND NAV 1.7 CHANNELS.**

| Compound № | Nav1.8 $IC_{50}$ (nM) | Nav1.7 $IC_{50}$ (nM) |
|---|---|---|
| 1 | ++ | + |
| 2 | + | + |
| 3 | +++ | + |
| 4 | + | + |
| 5 | + | + |
| 6 | + | + |

(continued)

| Compound № | Nav1.8 $IC_{50}$ (nM) | Nav1.7 $IC_{50}$ (nM) |
|---|---|---|
| 7 | ++ | + |
| 8 | +++ | + |
| 9 | + | + |
| 10 | +++ | ++ |
| 11 | + | + |
| 12 | + | + |
| 13 | +++ | + |
| 14 | +++ | + |
| 15 | +++ | + |
| 16 | +++ | + |
| 17 | + | + |
| 18 | +++ | + |
| 22 | +++ | + |
| 24 | + | + |
| 25 | ++ | + |
| 26 | ++ | + |
| 27 | +++ | + |
| 28 | +++ | + |
| 29 | ++ | + |
| 30 | +++ | + |
| 31 | + | + |
| 32 | +++ | + |
| 33 | +++ | + |
| 34 | +++ | + |
| 39 | +++ | + |
| 40 | + | + |
| 41 | ++ | + |
| 42 | ++ | + |
| 43 | +++ | ++ |
| 44 | + | + |
| 45 | +++ | + |
| 47 | + | + |
| 48 | + | + |
| 49 | +++ | + |
| 50 | + | + |

From these results, the blocking activity of Nav 1.7 and/or 1.8 is proven, whose application is readily performed by those skilled in the art in pharmaceutical compositions, which may comprise one or more of the aforementioned Formula I compounds, kits, in addition to uses in the treatment of pain-related pathologies.

In particular, these results indicate the possibility of using Formula (I) compounds in the preparation of drugs for the treatment of conditions such as peripheral neuropathic pain, chemotherapy-induced neuropathy, complex regional

pain, neuropathy related to viral infection, neuropathy secondary to tumor infiltration, diabetic neuropathy, phantom limb pain, postherpetic neuralgia, trigeminal neuralgia, and postsurgical neuralgia.

It should be understood that the embodiments described above are merely illustrative and that several modifications can be made by a person skilled in the art without departing from the scope of this invention. Consequently, the present invention should not be regarded as being limited to the illustrative specifications described in this application.

## Claims

1. COMPOUND, **characterized by** being of Formula (I):

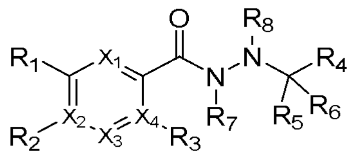

Formula (I)

or a pharmaceutically acceptable salt, hydrate, solvate, ester and isomer thereof, wherein:

- $X_1$, $X_2$, $X_3$, and $X_4$ are independently selected from the group consisting of carbon, CH, or nitrogen, wherein:
- when at least one of the substituents $X_2$, $X_4$ is N or CH, the corresponding R ($R_2$, $R_3$) is null;
- $R_1$ is:

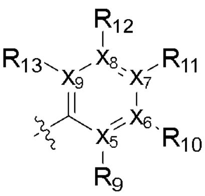

,

wherein:

- $X_5$, $X_6$, $X_7$, $X_8$, and $X_9$ are independently selected from the group consisting of carbon, CH, or nitrogen, wherein:

  - when at least one of the substituents $X_5$-$X_9$ is N or CH, the corresponding R ($R_9$-$R_{13}$) is null;
  - $R_9$, $R_{10}$, $R_{11}$, $R_{12}$, and $R_{13}$ are independently selected from the group consisting of hydrogen, halogen, linear or branched $C_1$-$C_6$ alkyl, linear or branched $C_1$-$C_6$ alkoxy, $C_3$-$C_7$ cycloalkoxy, difluoromethyl, trifluoromethyl, linear or branched $C_1$-$C_6$ haloalkoxy;
  - $R_2$ and $R_3$ are independently selected from the group consisting of hydrogen, halogen, amine, hydroxy, and alkyl $C_1$-$C_6$ linear or branched, alkoxy $C_1$-$C_6$ linear or branched;
  - $R_4$ is:

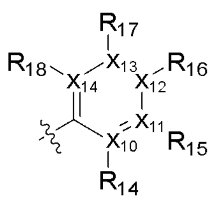

,

wherein:

- $X_{10}$, $X_{11}$, $X_{12}$, $X_{13}$, and $X_{14}$ are independently selected from the group consisting of carbon, CH, or nitrogen, wherein:

  - when at least one of the substituents $X_{10}$-$X_{14}$ is N or CH, the corresponding R ($R_{14}$-$R_{18}$) is null,

- $R_{14}$, $R_{13}$, $R_{16}$, $R_{17}$, and $R_{18}$ are independently selected from the group consisting of hydrogen, halogen, hydroxy(alkyl $C_2$-$C_4$ linear or branched), alkyl $C_1$-$C_6$ linear or branched, alkoxy $C_1$-$C_6$ linear or branched;
- $R_4$ and $R_8$ are optionally substituted to form a piperidine, homopiperidine, homomorpholine, or pyrrolidine;
- $R_5$ and $R_6$ are independently selected from the group consisting of hydrogen, linear or branched $C_1$-$C_6$ alkyl; and
- $R_7$ and $R_8$ are independently selected from the group consisting of hydrogen, linear or branched $C_1$-$C_6$ alkyl.

2. COMPOUND, in accordance with claim 1, **characterized by** being selected from the group consisting of:

- *N'*-(3,5-dimethoxybenzyl)-6-(4-methoxyphenyl)pyrazine-2-carbohydrazide (Compound 1);
- *N'*-(4-chlorobenzyl)-6-(4-methoxyphenyl)pyrazine-2-carbohydrazide (Compound 2);
- *N'*-(3,5-dimethoxybenzyl)-6-(4-isopropoxyphenyl)pyrazine-2-carbohydrazide (Compound 3);
- *N'*-(4-chlorobenzyl)-6-(4-cyclopropoxyphenyl)pyrazine-2-carbohydrazide (Compound 4);
- *N'*-(4-chlorobenzyl)-6-(4-chlorophenyl)pyrazine-2-carbohydrazide (Compound 5);
- *N*-(6-chloro-3,4-dihydroisoquinoline-2(1H)-yl)-6-(4-chlorophenyl)pyrazine-2-carboxamide (Compound 6);
- 6-(4-(difluoromethoxy)phenyl)-*N'*-(3,5-dimethoxybenzyl)pyrazine-2-carbohydrazide (Compound 7);
- 6-(6-(difluoromethoxy)pyridin-3-yl)-*N'*-(3,5-dimethoxybenzyl)pyrazine-2-carbohydrazide (Compound 8);
- *N'*-(4-chlorobenzyl)-6-(4-ethoxyphenyl)pyrazine-2-carbohydrazide (Compound 9);
- *N'*-(3,5-dimethoxybenzyl)-6-(4-ethoxyphenyl)pyrazine-2-carbohydrazide (Compound 10);
- 6-(4-ethoxyphenyl)-*N*-(8-methoxy-1,3,4,5-tetrahydro-2H-benzo[c]azepine-2-yl)pyrazine-2-carboxamide (Compound 11);
- 6-(4-ethoxyphenyl)-*N*-(7-methoxy-2,3-dihydrobenzo[f][1,4]oxazepine-4(5H)-yl)pyrazine-2-carboxamide (Compound 12);
- 6-(4-ethoxyphenyl)-*N*-(5-methoxyisoindoline-2-yl)pyrazine-2-carboxamide (Compound 13);
- 6-(4-ethoxyphenyl)-*N'*-(5-methoxy-2-methylbenzyl)pyrazine-2-carbohydrazide (Compound 14);
- 6-(4-ethoxyphenyl)-*N'*-(2-fluoro-5-methoxybenzyl)pyrazine-2-carbohydrazide (Compound 15);
- 6-(4-ethoxyphenyl)-*N'*-(3-methoxybenzyl)pyrazine-2-carbohydrazide (Compound 16);
- *N'*-(3,5-dimethoxybenzyl)-6-(4-ethoxyphenyl)-*N'*-methylpyrazine-2-carbohydrazide (Compound 17);
- 6-(4-ethoxyphenyl)-*N'*-((5-methoxy-2-methylpyridin-3-yl)methyl)pyrazine-2-carbohydrazide (Compound 18);
- 6-(4-ethoxyphenyl)-*N'*-(3-(1-hydroxyethyl)-5-methoxybenzyl)pyrazine-2-carbohydrazide (Compound 19);
- 6-(4-ethoxyphenyl)-*N'*-((2-fluoro-5-(1-hydroxyethyl)pyridin-3-yl)methyl)pyrazine-2-carbohydrazide (Compound 20);
- 6-(4-ethoxyphenyl)-*N'*-(2-fluoro-5-(1-hydroxyethyl)benzyl)pyrazine-2-carbohydrazide (Compound 21);
- 6-(4-ethoxyphenyl)-*N'*-(2-fluorobenzyl)pyrazine-2-carbohydrazide (Compound 22);
- 6-(4-ethoxyphenyl)-*N'*-(3-(1-hydroxyethyl)benzyl)pyrazine-2-carbohydrazide (Compound 23);
- (*S*)-*N'*-(1-(3,5-dimethoxyphenyl)ethyl)-6-(4-ethoxyphenyl)pyrazine-2-carbohydrazide (Compound 24);
- (*R*)-*N'*-(1-(3,5-dimethoxyphenyl)ethyl)-6-(4-ethoxyphenyl)pyrazine-2-carbohydrazide (Compound 25);
- 6-(4-ethoxyphenyl)-*N'*-((2-fluoro-5-methoxypyridin-3-yl)methyl)pyrazine-2-carbohydrazide (Compound 26);
- *N'*-(3,5-dimethoxybenzyl)-6-(4-ethoxy-2-fluorophenyl)pyrazine-2-carbohydrazide (Compound 27);
- *N'*-(3,5-dimethoxybenzyl)-6-(4-ethoxy-2-methylphenyl)pyrazine-2-carbohydrazide (Compound 28);
- *N'*-(3,5-dimethoxybenzyl)-6-(6-ethoxypyridin-3-yl)pyrazine-2-carbohydrazide (Compound 29);
- 6-(6-ethoxypyridin-3-yl)-*N'*-(3-methoxybenzyl)pyrazine-2-carbohydrazide (Compound 30);
- 6-(6-ethoxypyridin-3-yl)-*N'*-((2-fluoro-5-methoxypyridin-3-yl)methyl)pyrazine-2-carbohydrazide (Compound 31);
- 6-(6-ethoxypyridin-3-yl)-*N'*-(2-fluoro-5-methoxybenzyl)pyrazine-2-carbohydrazide (Compound 32);
- 6-(6-ethoxypyridin-3-yl)-*N'*-(5-methoxy-2-methylbenzyl)pyrazine-2-carbohydrazide (Compound 33);
- 6-(6-ethoxypyridin-3-yl)-*N'*-((5-methoxy-2-methylpyridin-3-yl)methyl)pyrazine-2-carbohydrazide (Compound 34);
- 6-(6-ethoxypyridin-3-yl)-*N'*-(3-(1-hydroxyethyl)benzyl)pyrazine-2-carbohydrazide (Compound 35);
- 6-(6-ethoxypyridin-3-yl)-*N'*-(3-(1-hydroxyethyl)-5-methoxybenzyl)pyrazine-2-carbohydrazide (Compound 36);
- 6-(6-ethoxypyridin-3-yl)-*N'*-((2-fluoro-5-(1-hydroxyethyl)pyridin-3-yl)methyl)pyrazine-2-carbohydrazide (Compound 37);
- 6-(6-ethoxypyridin-3-yl)-*N'*-(2-fluoro-5-(1-hydroxyethyl)benzyl)pyrazine-2-carbohydrazide (Compound 38);
- 6-(6-ethoxypyridin-3-yl)-*N'*-(2-fluorobenzyl)pyrazine-2-carbohydrazide (Compound 39);
- *N'*-(3,5-dimethoxybenzyl)-6-(6-ethoxy-4-methylpyridin-3-yl)pyrazine-2-carbohydrazide (Compound 40);

- *N'*-(3,5-dimethoxybenzyl)-6-(6-ethoxy-2-methylpyridin-3-yl)pyrazine-2-carbohydrazide (Compound 41);
- 6-(6-cyclopropoxypyridin-3-yl)-*N'*-(3,5-dimethoxybenzyl)pyrazine-2-carbohydrazide (Compound 42);
- *N'*-(3,5-dimethoxybenzyl)-6-(6-isopropoxypyridin-3-yl)pyrazine-2-carbohydrazide (Compound 43);
- 4-(4-chlorophenyl)-*N'*-(3,5-dimethoxybenzyl)pyrimidine-2-carbohydrazide (Compound 44);
- 6-(4-ethoxyphenyl)-*N*-(7-methoxy-3,4-dihydroisoquinoline-2(1*H*)-yl)pyrazine-2-carboxamide (Compound 45);
- 6-(4-ethoxyphenyl)-*N'*-(2-fluorobenzyl)pyridazine-4-carbohydrazide (Compound 46);
- 5-(4-ethoxyphenyl)-*N'*-(2-fluorobenzyl)pyridazine-3-carbohydrazide (Compound 47);
- 4-(4-ethoxyphenyl)-*N'*-(2-fluorobenzyl)picolinohydrazide (Compound 48);
- 5-(4-ethoxyphenyl)-*N'*-(2-fluorobenzyl)nicotinohydrazide (Compound 49);
- 6-(4-ethoxyphenyl)-*N'*-(2-fluorobenzyl)picolinohydrazide (Compound 50);
- 2-(4-ethoxyphenyl)-*N'*-(2-fluorobenzyl)isonicotinohydrazide (Compound 51);
- 2-(4-ethoxyphenyl)-*N'*-(2-fluorobenzyl)pyrimidine-4-carbohydrazide (Compound 52) and
- 4-(4-ethoxyphenyl)-*N'*-(2-fluorobenzyl)pyrimidine-2-carbohydrazide (Compound 53).

**3.** COMPOUND, according to any of claims 1 or 2, **characterized by** being a blocker of voltage-gated sodium channels Nav 1.7 and/or Nav 1.8.

**4.** PHARMACEUTICAL COMPOSITION, **characterized by** comprising a therapeutically effective amount of one or more compounds of Formula (I) or of a pharmaceutically acceptable salt, hydrate, solvate, and isomer thereof, as defined in any of claims 1 to 3, and one or more pharmaceutically acceptable excipients.

**5.** PHARMACEUTICAL COMPOSITION, according to claim 4, **characterized by** being formulated as an oral, sublingual, nasal, parenteral, injectable, submuscular, topical, transdermal, ocular, or rectal composition.

**6.** USE OF FORMULA (I) COMPOUND(S), as defined in any of claims 1 to 3, **characterized by** being to prepare a drug to treat pathologies related to neuropathic pain.

**7.** USE, according to claim 6, **characterized by** said pathologies being selected from the group consisting of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia, and postsurgical neuralgia.

**8.** METHOD OF TREATMENT, PREVENTION, RELIEF, SUPPRESSION AND/OR CONTROL of pathologies related to neuropathic pain, **characterized by the** administration of an effective amount of at least one compound of Formula (I) or a pharmaceutically acceptable salt, hydrate, solvate and isomer thereof, as defined in any of claims 1 to 3.

**9.** METHOD, according to claim 8, **characterized by** being for the treatment or prophylaxis of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia, and postsurgical neuralgia.

**10.** METHOD, according to any of claims 8 or 9, **characterized by** the administration of at least one compound of Formula (I) being selected from the group comprising oral, sublingual, nasal, parenteral, injectable, submuscular, topical, transdermal, ocular, and rectal routes.

**11.** PROCESS OF OBTAINING GENERAL FORMULA COMPOUND (I), as defined in any of the claims 1 to 3, **characterized by** comprising the steps:

(a) formation of the Formula IV or Formula VI intermediate:

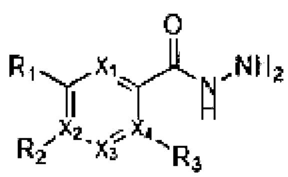

Formula IV

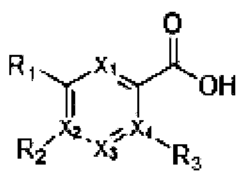

Formula VI

from the hydrazinolysis reaction or hydrolysis of a Formula V intermediate:

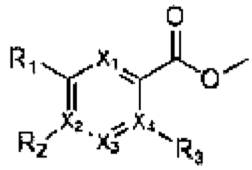

**Formula V**

(b) formation of the Formula II intermediate:

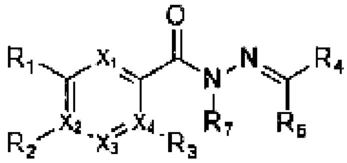

**Formula II**

from condensation between Formula III intermediates:

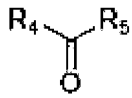

**Formula III**

and Formula IV intermediates with or without the presence of a catalyst and a suitable solvent;
(c) Formula I compound is obtained:

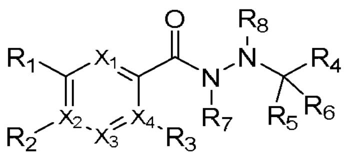 Formula (I)

from the reduction of Formula II intermediates or from reactions and amide coupling between intermediates VI and VII with or without the presence of a catalyst and a suitable solvent:

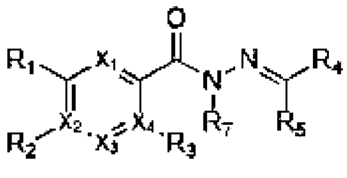

**Formula II**

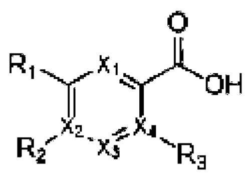

**Formula VI**

$RNH_2$ **Formula VII**

wherein:

- $X_1$, $X_2$, $X_3$, and $X_4$ are independently selected from the group consisting of carbon, CH, or nitrogen, wherein:
- when at least one of the substituents $X_2$, $X_4$ is N or CH, the corresponding R ($R_2$, $R_3$) is null;
- $R_1$ is:

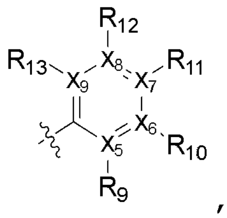

wherein:

- $X_5$, $X_6$, $X_7$, $X_8$, and $X_9$ are independently selected from the group consisting of carbon, CH, or nitrogen, wherein:

  - when at least one of the substituents $X_5$-$X_9$ is N or CH, the corresponding R ($R_9$-$R_{13}$) is null;
  - $R_9$, $R_{10}$, $R_{11}$, $R_{12}$, and $R_{13}$ are independently selected from the group consisting of hydrogen, halogen, linear or branched $C_1$-$C_6$ alkyl, linear or branched $C_1$-$C_6$ alkoxy, $C_3$-$C_7$ cycloalkoxy, difluoromethyl, trifluoromethyl, linear or branched $C_1$-$C_6$ haloalkoxy;
  - $R_2$ and $R_3$ are independently selected from the group consisting of hydrogen, halogen, amine, hydroxy, and alkyl $C_1$-$C_6$ linear or branched, alkoxy $C_1$-$C_6$ linear or branched;
  - $R_4$ is:

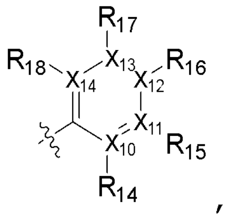

  where:

  - $X_{10}$, $X_{11}$, $X_{12}$, $X_{13}$, and $X_{14}$ are independently selected from the group consisting of carbon, CH, or nitrogen, wherein:
  - when at least one of the substituents $X_{10}$-$X_{14}$ is N or CH, the corresponding R ($R_{14}$-$R_{18}$) is null,
  - $R_{14}$, $R_{13}$, $R_{16}$, $R_{17}$, and $R_{18}$ are independently selected from the group consisting of hydrogen, halogen, hydroxy(alkyl $C_2$-$C_4$ linear or branched), alkyl $C_1$-$C_6$ linear or branched, alkoxy $C_1$-$C_6$ linear or branched;
  - $R_4$ and $R_8$ are optionally substituted to form a piperidine, homopiperidine, homomorpholine, or pyrrolidine;
  - $R_5$ and $R_6$ are independently selected from the group consisting of hydrogen, linear or branched $C_1$-$C_6$ alkyl; and
  - $R_7$ and $R_8$ are independently selected from the group consisting of hydrogen, linear or branched $C_1$-$C_6$ alkyl.

**12.** PROCESS, according to claim 11, **characterized by** additionally comprising a step (d) of compound formation(s) of Formula (I) in which $R_7$ is methyl from the N-alkylation reaction of a compound obtained in step (c) with formaldehyde.

**13.** PROCESS, according to any of claims 11 or 12, **characterized by** the compound of Formula III of step (b) being an acetophenone derivative or a benzaldehyde derivative.

**14.** PROCESS, according to any of claims 12 or 13, **characterized by** in step (c) the step-reducing agent being selected between triethylsilane or sodium borohydride, and the acid being TFA.

**15.** KIT, **characterized by** comprising a pharmaceutical composition as defined in claim 4 and an application device.

**INTERNATIONAL SEARCH REPORT**

International application No. **PCT/BR2024/050024**

**A. CLASSIFICATION OF SUBJECT MATTER**

***C07C 233/64***(2006.01)i; ***C07C 243/38***(2006.01)i; ***C07C 243/14***(2006.01)i; **C07C 15/00**(2006.01)i; **C07D 213/00**(2006.01)i; **C07D 401/00**(2006.01)i; **A61K 31/166**(2006.01)i; **A61K 31/4965**(2006.01)i; **A61K 31/505**(2006.01)i; **A61K 31/44**(2006.01)i; **A61K 31/4353**(2006.01)i; ***A61P 25/02***(2006.01)i; ***A61P 25/04***(2006.01)i; **A61P 29/00**(2006.01)i

CPC: C07C 233/64, C07C 243/38, C07C 243/14, C07C 15/00, C07D 213/00; C07D 401/00, A61K 31/166, A61K 31/4965, A61K 31/505, A61K 31/44; A61K 31/4353, A61P 25/02, A61P 25/04, A61P 29/00

According to International Patent Classification (IPC) or to both national classification and IPC

**B. FIELDS SEARCHED**

Minimum documentation searched (classification system followed by classification symbols)

C07C 233/64; C07C 243/38; C07C 243/14; C07C 15/00; C07D 213/00; C07D 401/00; A61K 31/166; A61K 31/4965; A61K 31/505; A61K 31/44; A61K 31/4353; A61P 25/02; A61P 25/04; A61P 29/00

CPC: C07C 233/64, C07C 243/38, C07C 243/14, C07C 15/00, C07D 213/00, C07D 401/00, A61K 31/166, A61K 31/4965, A61K 31/505, A61K 31/44, A61K 31/4353, A61P 25/02, A61P 25/04, A61P 29/00

Documentation searched other than minimum documentation to the extent that such documents are included in the fields searched

Banco de dados INPI-BR; Periódicos Capes; Google

Electronic data base consulted during the international search (name of data base and, where practicable, search terms used)

REGISTRY, CAPLUS, MARPAT (STN®); DERWENT INNOVATION

**C. DOCUMENTS CONSIDERED TO BE RELEVANT**

| Category* | Citation of document, with indication, where appropriate, of the relevant passages | Relevant to claim No. |
|---|---|---|
| X<br>A | WO 2006006569 A1 (NIHON NOHYAKU CO LTD [JP]) 19 January 2006 (2006-01-19)<br>See abstract and claims | 1-5, 11-15<br>6, 7 |
| A | Da Silva Y.K. et al. Synthesis and pharmacological evaluation of pyrazine N-acylhydrazone derivatives designed as novel analgesic and anti-inflammatory drug candidates. Bioorg Med Chem. 2010 July 15:18(14):5007-15. doi: 10.1016/j.bmc.2010.06.002. Epub 2010 Jun 8. PMID: 20598893. | 1-7, 11-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 March 2024** | **03 May 2024** |

| Name and mailing address of the ISA/BR | Authorized officer |
|---|---|
| **National Institute of Industrial Property (Brazil)**<br>**Rua Mayrink Veiga, 9, 6º andar, CEP 20.090-910 Rio de Janeiro – RJ**<br>**Brazil** | **Rodinelli OLIVEIRA** |
| Telephone No. **(55 21) 3037-3742, 3037-3984** | Telephone No. **+55 21 3037 4528 - 3037 3319** |

Form PCT/ISA/210 (second sheet) (July 2022)

**INTERNATIONAL SEARCH REPORT**

International application No. **PCT/BR2024/050024**

**C. DOCUMENTS CONSIDERED TO BE RELEVANT**

| Category* | Citation of document, with indication, where appropriate, of the relevant passages | Relevant to claim No. |
|---|---|---|
| A | Lopes A.B. et al. Characterization of amide bond conformers for a novel heterocyclic template of N-acylhydrazone derivatives. Molecules. 2013 Sep 25;18(10):11683-704. doi: 10.3390/molecules181011683. PMID: 24071978; PMCID: PMC6270085. | 1-7, 11-15 |
| A | US 2008300240 A1 (OMEROS CORP [US]) 04 December 2008 (2008-12-04)<br>The whole document | 1-7, 11-15 |
| A | WO 2008021388 A1 (KEMIA INC [US]) 21 February 2008 (2008-02-21)<br>The whole document | 1-7, 11-15 |
| A | WO 2020210428 A1 (BAYLOR COLLEGE MEDICINE [US]) 15 October 2020 (2020-10-15)<br>The whole document | 1-7, 11-15 |
| A | WO 2020138271 A1 (RAQUALIA PHARMA INC [JP]) 02 July 2020 (2020-07-02)<br>The whole document | 1-7, 11-15 |
| A | WO 2018235851 A1 (RAQUALIA PHARMA INC [JP]) 27 December 2018 (2018-12-27)<br>The whole document | 1-7, 11-15 |
| A | WO 2006051311 A1 (BIOFOCUS DISCOVERY LTD [GB]) 18 May 2006 (2006-05-18)<br>The whole document | 1-7, 11-15 |
| A | WO 2017184999 A1 (DANA FARBER CANCER INST INC [US]) 26 October 2017 (2017-10-26)<br>The whole document | 1-7, 11-15 |
| A | WO 2008121877 A2 (INST ONEWORLD HEALTH [US]) 09 October 2008 (2008-10-09)<br>The whole document | 1-7, 11-15 |
| A | EP 1514544 A1 (INST MED MOLECULAR DESIGN INC [JP]) 16 March 2005 (2005-03-16)<br>The whole document | 1-7, 11-15 |
| A | WO 2004111034 A1 (ASTRAZENECA AB [SE]) 23 December 2004 (2004-12-23)<br>The whole document | 1-7, 11-15 |
| A | WO 2004069277 A1 (ASTRAZENECA AB [SE]) 19 August 2004 (2004-08-19)<br>The whole document | 1-7, 11-15 |
| A | WO 03051851 A1 (ASTRAZENECA AB [SE]) 26 June 2003 (2003-06-26)<br>The whole document | 1-7, 11-15 |
| A | WO 2011069039 A1 (US HEALTH [US]) 09 June 2011 (2011-06-09)<br>The whole document | 1-7, 11-15 |
| A | WO 2022067114 A1 (NEUROPORE THERAPIES INC [US]) 31 March 2022 (2022-03-31)<br>The whole document | 1-7, 11-15 |
| A | CN 105218426 A (LANZHOU CHEM PHYS INST) 06 January 2016 (2016-01-06)<br>The whole document | 1-7, 11-15 |
| A | Rodrigues F.A. et al. Biological evaluation of isoniazid derivatives as an anticancer class. Sci Pharm. 2013 Sep 22;82(1):21-8. doi: 10.3797/scipharm.1307-25. PMID: 24634839; PMCID: PMC3951230. | 1-7, 11-15 |
| A | Sharma M. et al. Discovery of novel 1,2,4-triazol-5-ones as tumor necrosis factor-alpha inhibitors for the treatment of neuropathic pain. Chem Biol Drug Des. 2012 Dec;80(6):961-70. doi: 10.1111/cbdd.12049. Epub 2012 Oct 9. PMID: 22958416. | 1-7, 11-15 |

Form PCT/ISA/210 (second sheet) (July 2022)

**INTERNATIONAL SEARCH REPORT**

International application No.
**PCT/BR2024/050024**

**C. DOCUMENTS CONSIDERED TO BE RELEVANT**

| Category* | Citation of document, with indication, where appropriate, of the relevant passages | Relevant to claim No. |
|---|---|---|
| A | WO 2004054977 A1 (CYTOPIA PTY LTD [AU]) 01 July 2004 (2004-07-01)<br>The whole document | 1-7, 11-15 |

Form PCT/ISA/210 (second sheet) (July 2022)

## INTERNATIONAL SEARCH REPORT

International application No.
**PCT/BR2024/050024**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **8-10**
because they relate to subject matter not required to be searched by this Authority, namely:

Claims 8-10 contain material that falls under the provisions of PCT Rule 39.1(iv) concerning methods for treatment of the human or animal body by surgery or therapy.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/BR2024/050024**

| Patent document cited in search report | | | Publication date (day/month/year) | Patent family member(s) | | | Publication date (day/month/year) |
|---|---|---|---|---|---|---|---|
| WO | 2006006569 | A1 | 19 January 2006 | NONE | | | |
| US | 2008300240 | A1 | 04 December 2008 | US | 7786139 | B2 | 31 August 2010 |
| | | | | US | 2011021509 | A1 | 27 January 2011 |
| | | | | US | 8278327 | B2 | 02 October 2012 |
| WO | 2008021388 | A1 | 21 February 2008 | AR | 062427 | A1 | 05 November 2008 |
| | | | | CL | 2007002375 | A1 | 04 April 2008 |
| | | | | PE | 20080906 | A1 | 05 July 2008 |
| | | | | TW | 200815422 | A | 01 April 2008 |
| WO | 2020210428 | A1 | 15 October 2020 | NONE | | | |
| WO | 2020138271 | A1 | 02 July 2020 | BR | 112021008995 | A2 | 10 August 2021 |
| | | | | CA | 3120268 | A1 | 02 July 2020 |
| | | | | CN | 113164461 | A | 23 July 2021 |
| | | | | EP | 3902544 | A1 | 03 November 2021 |
| | | | | JP | 2022515630 | A | 21 February 2022 |
| | | | | KR | 20210107698 | A | 01 September 2021 |
| | | | | MX | 2021007425 | A | 05 August 2021 |
| | | | | TW | 202038950 | A | 01 November 2020 |
| | | | | US | 2022048892 | A1 | 17 February 2022 |
| WO | 2018235851 | A1 | 27 December 2018 | AU | 2018289731 | A1 | 14 November 2019 |
| | | | | AU | 2018289731 | B2 | 18 November 2021 |
| | | | | BR | 112019021736 | A2 | 05 May 2020 |
| | | | | CA | 3059687 | A1 | 27 December 2018 |
| | | | | CN | 110612285 | A | 24 December 2019 |
| | | | | CN | 110612285 | B | 04 April 2023 |
| | | | | DK | 3487839 | T3 | 18 January 2021 |
| | | | | EP | 3487839 | A1 | 29 May 2019 |
| | | | | EP | 3487839 | A4 | 18 March 2020 |
| | | | | EP | 3487839 | B1 | 23 December 2020 |
| | | | | ES | 2851004 | T3 | 02 September 2021 |
| | | | | HU | E053460 | T2 | 28 June 2021 |
| | | | | JP | 6592702 | B1 | 23 October 2019 |
| | | | | JP | 2019532911 | A | 14 November 2019 |
| | | | | KR | 20200015888 | A | 13 February 2020 |
| | | | | KR | 102577137 | B1 | 11 September 2023 |
| | | | | MX | 2019012635 | A | 11 December 2019 |
| | | | | PH | 12019502850 | A1 | 28 September 2020 |
| | | | | PL | 3487839 | T3 | 14 June 2021 |
| | | | | PT | 3487839 | T | 11 February 2021 |
| | | | | RU | 2019133530 | A | 20 July 2021 |
| | | | | RU | 2019133530 | A3 | 04 August 2021 |
| | | | | RU | 2768149 | C2 | 23 March 2022 |
| | | | | SG | 11201909793Y | A | 28 November 2019 |
| | | | | TW | 201904944 | A | 01 February 2019 |
| | | | | TW | I769266 | B | 01 July 2022 |
| | | | | US | 2020016135 | A1 | 16 January 2020 |
| | | | | US | 11154544 | B2 | 26 October 2021 |
| | | | | ZA | 201906888 | B | 24 February 2021 |
| WO | 2006051311 | A1 | 18 May 2006 | CA | 2585490 | A1 | 18 May 2006 |
| | | | | EP | 1814856 | A1 | 08 August 2007 |

Form PCT/ISA/210 (patent family annex) (July 2022)

**INTERNATIONAL SEARCH REPORT**
**Information on patent family members**

International application No.
**PCT/BR2024/050024**

| Patent document cited in search report | Publication date (day/month/year) | Patent family member(s) | Publication date (day/month/year) |
|---|---|---|---|
| | | JP 2008519814 A | 12 June 2008 |
| WO 2017184999 A1 | 26 October 2017 | AU 2017252460 A1 | 11 October 2018 |
| | | AU 2017252460 B2 | 18 November 2021 |
| | | CA 3018270 A1 | 26 October 2017 |
| | | CN 109069508 A | 21 December 2018 |
| | | EP 3445365 A1 | 27 February 2019 |
| | | EP 3445365 A4 | 16 October 2019 |
| | | JP 2019514884 A | 06 June 2019 |
| | | US 2019135796 A1 | 09 May 2019 |
| | | US 2020039968 A9 | 06 February 2020 |
| | | US 10633371 B2 | 28 April 2020 |
| WO 2008121877 A2 | 09 October 2008 | WO 2008121877 A3 | 31 December 2008 |
| | | BR PI0809498 A2 | 23 September 2014 |
| | | CN 101668732 A | 10 March 2010 |
| | | EP 2142498 A2 | 13 January 2010 |
| | | JP 2010523579 A | 15 July 2010 |
| | | KR 20100016073 A | 12 February 2010 |
| | | TW 200845957 A | 01 December 2008 |
| | | TW I351949 B | 11 November 2011 |
| | | US 2008269206 A1 | 30 October 2008 |
| | | US 8283351 B2 | 09 October 2012 |
| EP 1514544 A1 | 16 March 2005 | EP 1514544 A4 | 07 January 2009 |
| | | AU 2003242103 A1 | 22 December 2003 |
| | | CA 2488367 A1 | 18 December 2003 |
| | | CN 1658872 A | 24 August 2005 |
| | | CN 1658872 B | 22 September 2010 |
| | | EA 200401612 A1 | 25 August 2005 |
| | | EA 009701 B1 | 28 February 2008 |
| | | JP 2010215644 A | 30 September 2010 |
| | | JP WO2003103665 A1 | 06 October 2005 |
| | | JP 4660674 B2 | 30 March 2011 |
| | | KR 20050023287 A | 09 March 2005 |
| | | KR 101054562 B1 | 04 August 2011 |
| | | TW 200402291 A | 16 February 2004 |
| | | US 2006122243 A1 | 08 June 2006 |
| | | US 2007185059 A1 | 09 August 2007 |
| | | US 2007185110 A1 | 09 August 2007 |
| | | US 7700655 B2 | 20 April 2010 |
| | | US 2008090779 A1 | 17 April 2008 |
| | | WO 03103665 A1 | 18 December 2003 |
| WO 2004111034 A1 | 23 December 2004 | AR 044825 A1 | 05 October 2005 |
| | | AT E406361 T1 | 15 September 2008 |
| | | AU 2004247616 A1 | 23 December 2004 |
| | | BR PI0411508 A | 25 July 2006 |
| | | CA 2527035 A1 | 23 December 2004 |
| | | CN 1809554 A | 26 July 2006 |
| | | DE 602004016158 D1 | 09 October 2008 |
| | | EP 1638953 A1 | 29 March 2006 |
| | | EP 1638953 B1 | 27 August 2008 |
| | | ES 2311159 T3 | 01 February 2009 |

Form PCT/ISA/210 (patent family annex) (July 2022)

**INTERNATIONAL SEARCH REPORT**
**Information on patent family members**

International application No.
**PCT/BR2024/050024**

| Patent document cited in search report | Publication date (day/month/year) | Patent family member(s) | Publication date (day/month/year) |
|---|---|---|---|
| | | GB 0314057 D0 | 23 July 2003 |
| | | HK 1088003 A1 | 27 October 2006 |
| | | IS 8226 A | 11 January 2006 |
| | | JP 2006527771 A | 07 December 2006 |
| | | KR 20060023152 A | 13 March 2006 |
| | | MX PA05013711 A | 08 March 2006 |
| | | NO 20055919 L | 16 February 2006 |
| | | RU 2005138365 A | 27 July 2006 |
| | | TW 200524605 A | 01 August 2005 |
| | | US 2007093484 A1 | 26 April 2007 |
| | | UY 28376 A1 | 31 January 2005 |
| | | ZA 200510101 B | 27 December 2006 |
| WO 2004069277 A1 | 19 August 2004 | AT E399566 T1 | 15 July 2008 |
| | | DE 602004014725 D1 | 14 August 2008 |
| | | EP 1592451 A1 | 09 November 2005 |
| | | EP 1592451 B1 | 02 July 2008 |
| | | ES 2308144 T3 | 01 December 2008 |
| | | GB 0302673 D0 | 12 March 2003 |
| | | JP 2006516986 A | 13 July 2006 |
| | | US 2006134146 A1 | 22 June 2006 |
| | | US 7473693 B2 | 06 January 2009 |
| WO 03051851 A1 | 26 June 2003 | AR 038044 A1 | 22 December 2004 |
| | | AT E354570 T1 | 15 March 2007 |
| | | AU 2002352425 A1 | 30 June 2003 |
| | | BR 0214989 A | 14 December 2004 |
| | | CA 2469786 A1 | 26 June 2003 |
| | | CN 1620438 A | 25 May 2005 |
| | | CO 5590917 A2 | 30 December 2005 |
| | | DE 60218340 D1 | 05 April 2007 |
| | | DE 60218340 T2 | 29 November 2007 |
| | | EP 1458690 A1 | 22 September 2004 |
| | | EP 1458690 B1 | 21 February 2007 |
| | | ES 2280599 T3 | 16 September 2007 |
| | | HU P0402026 A2 | 28 February 2005 |
| | | HU P0402026 A3 | 29 August 2005 |
| | | IS 7314 A | 16 June 2004 |
| | | JP 2005517655 A | 16 June 2005 |
| | | KR 20040068286 A | 30 July 2004 |
| | | MX PA04005990 A | 27 September 2004 |
| | | NO 20043022 L | 15 July 2004 |
| | | NZ 533275 A | 24 February 2006 |
| | | PL 369731 A1 | 02 May 2005 |
| | | RU 2004116916 A | 10 November 2005 |
| | | SE 0104330 D0 | 19 December 2001 |
| | | TW 200410694 A | 01 July 2004 |
| | | US 2005032808 A1 | 10 February 2005 |
| | | US 7342019 B2 | 11 March 2008 |
| | | ZA 200404805 B | 15 August 2005 |
| WO 2011069039 A1 | 09 June 2011 | US 2012316198 A1 | 13 December 2012 |
| | | US 8518968 B2 | 27 August 2013 |

Form PCT/ISA/210 (patent family annex) (July 2022)

**INTERNATIONAL SEARCH REPORT**
**Information on patent family members**

International application No.
**PCT/BR2024/050024**

| Patent document cited in search report | Publication date (day/month/year) | Patent family member(s) | Publication date (day/month/year) |
|---|---|---|---|
| WO 2022067114 A1 | 31 March 2022 | AR 123604 A1 | 21 December 2022 |
| | | AU 2021347364 A1 | 23 March 2023 |
| | | CA 3193577 A1 | 31 March 2022 |
| | | CL 2023000853 A1 | 01 September 2023 |
| | | CN 116507607 A | 28 July 2023 |
| | | CO 2023005057 A2 | 19 May 2023 |
| | | CR 20230177 A | 30 June 2023 |
| | | EP 4217342 A1 | 02 August 2023 |
| | | IL 301574 A | 01 May 2023 |
| | | JP 2023547770 A | 14 November 2023 |
| | | KR 20230074744 A | 31 May 2023 |
| | | MX 2023003348 A | 29 March 2023 |
| | | PE 20231209 A1 | 17 August 2023 |
| | | TW 202229249 A | 01 August 2022 |
| | | US 2024043395 A1 | 08 February 2024 |
| CN 105218426 A | 06 January 2016 | NONE | |
| WO 2004054977 A1 | 01 July 2004 | CA 2508171 A1 | 01 July 2004 |
| | | CA 2508171 C | 28 August 2012 |
| | | EP 1569907 A1 | 07 September 2005 |
| | | EP 1569907 A4 | 31 October 2007 |
| | | EP 1569907 B1 | 09 March 2016 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 10550080 B
- US 9765029 B
- US 10000475 B
- WO 2020261114 A
- WO 2020092667 A
- US 9163042 B
- WO 2014120808 A
- WO 2014120815 A
- WO 2018213426 A
- WO 2019014352 A
- WO 2015006280 A
- US 7928107 B
- WO 2018235851 A
- US 8629149 B
- JP 2017001991 B


### Non-patent literature cited in the description


- **SCHAIBLE.** *Langenbecks Arch. Surg*, 2004, vol. 389, 237
- **SMITH**. *Pain*, 2020, vol. 161 (1), S127
- *Cavalli. Int. J. Immunopathol. Pharmacol.*, 2019, vol. 33, 2058738419838383
- *Bouhassira. Rev Neurol (Paris).*, 2019, vol. 175 (1-2), 16
- **SCHOLZ.** *Nature Neurosci.*, 2002, vol. 5, 1062
- **COSTIGAN**. *Annu. Rev. Neurosci.*, 2009, vol. 32, 1
- **DWORKIN**. *Clin. J. Pain*, 2002, vol. 18 (6), 343
- **DUCREUX.** *Brain*, 2006, vol. 129, 963
- **PAK.** *Curr. Pain Headache Rep*, 2018, vol. 22 (2), 9
- **KUSHNAREV.** *Expert Opin. Investig. Drugs*, 2020, vol. 29 (3), 259
- **EMERY.** *Expert Opin. Ther. Targets*, 2016, vol. 20 (8), 975
- **CATTERALL.** *Nat. Chem. Biol.*, 2020, vol. 16, 1314
- **WISEDCHAISRI.** *Cell*, 2019, vol. 178 (4), 993
- **CLAIRFEUILLE**. *Science*, 2019, vol. 363, 1302
- **LERA-RUIZ.** *J. Med. Chem.*, 2015, vol. 58 (18), 7093
- **BAGAL.** *J. Med. Chem.*, 2013, vol. 56 (3), 593
- **BAGAL.** *Channels*, 2015, vol. 9 (6), 360
- **LAW.** *Drug Discovery Today*, 2019, vol. 24 (7), 1389
- **BAGAL.** *Channels (Austin)*, 2015, vol. 9 (6), 360
- **VETTER.** *Pharmacology & Therapeutics*, 2017, vol. 172, 73
- **AHUJA.** *Science*, 2015, vol. 350 (6267), 1491
- **KINGWELL.** *Nat. Rev. Drug Discov.*, 2019, vol. 18, 321
- **SAFINA.** *J. Med. Chem.*, 2021, vol. 64, 2953
- **LUO.** *J. Med. Chem.*, 2019, vol. 62, 831
- **BANKAR**. *Cell Reports*, 2018, vol. 24, 3133
- **BROWN.** *Bioorg. Med. Chem*, 2019, vol. 27 (1), 230
- **PAYNE. BR.** *J. Pharmacol.*, 2015, vol. 172 (10), 2654
- **BAGAL.** *Med. Chem. Lett.*, 2015, vol. 6 (6), 650
- **KORT.** *J. Med. Chem.*, 2008, vol. 51, 407
- **ZHANG.** *Neuropharmacology*, 2010, vol. 59, 201-207
- **KORNECOOK.** *J. Pharmacol. Exp. Ther.*, 2017, vol. 362, 146
- **KINGWELL.** *Nat. Rev. Drug Discov*, 2019, vol. 18, 321
- **DEUIS.** *Neuropharmacology*, 2017, vol. 127, 87-108
- **MCKERRALL.** *Bioorganic & Medicinal Chemistry Lett.*, 2018, vol. 28, 3141
- **BAGAL.** *Bioorganic & Medicinal Chemistry Lett*, 2014, vol. 24, 3690